# EUROPEAN PATENT APPLICATION

(11) **EP 4 745 153 A1**
(43) Date of publication of application: **20.05.2026**
(21) Application number: 24842296.6
(22) Date of filing: 12.07.2024
(51) Int. Cl.: C07K 14/605, C07K 14/00, A61K 38/16, A61P 3/10, A61P 3/04, A61P 1/16, A61P 3/06

(54) **GLP-1, GIP AND GCG RECEPTOR TRIAGONIST AND USE THEREOF**

(30) Priority: 14.07.2023 CN 202310869978; 28.11.2023 CN 202311605389; 10.01.2024 CN 202410038906
(71) Applicant: Jiangsu Hengrui Pharmaceuticals Co., Ltd., Lianyungang, Jiangsu 222047 (CN)
(72) Inventor: WU, Fangzhou, Beijing 102206 (CN); JIANG, Peng, Beijing 102206 (CN); HUANG, Xuchao, Beijing 102206 (CN); WANG, Jingyi, Beijing 102206 (CN); TANG, Jingjing, Beijing 102206 (CN); JING, Lutao, Beijing 102206 (CN); REN, Lejiao, Beijing 102206 (CN); ZHANG, Linshuang, Beijing 102206 (CN); QU, Liang, Beijing 102206 (CN); HE, Xiang, Beijing 102206 (CN); WANG, Lei, Beijing 102206 (CN)
(74) Representative: J A Kemp LLP
(86) International application number: PCT/CN2024/105136
(87) International publication number: WO 2025/016303

(57) **Abstract**

A GLP-1, GIP and GCG receptor triagonist and a use thereof. The corresponding compound has an agonist effect on a human glucagon-like peptide-1 (GLP-1) receptor, a human glucose-dependent insulinotropic polypeptide (GIP) receptor and a human glucagon (GCG) receptor, and can be used for preventing and/or treating diseases or conditions related to metabolic disorders.

## Description

### FIELD OF THE INVENTION

The present disclosure belongs to the field of biomedicine, and specifically relates to a compound that has an agonist effect on a human glucagon-like peptide-1 (GLP-1) receptor, a human glucose-dependent insulinotropic polypeptide (GIP) receptor and a human glucagon (GCG) receptor, and can be used for preventing and/or treating diseases or conditions related to metabolic disorders.

### BACKGROUND OF THE INVENTION

Glucagon-like peptide-1 (GLP-1) is a gastrointestinal regulatory polypeptide containing 30 or 31 amino acid residues. The secretion of GLP-1 is mainly regulated by L-cells in the small intestine according to the absorption of nutrients and fluctuating blood sugar levels in the body. After food intake, L-cells in the small intestine secrete large amounts of GLP-1 to enhance the endocrine function of the pancreas. GLP-1 polypeptide performs its physiological functions of controlling blood sugar and reducing appetite in vivo mainly by activating GLP-1 receptors distributed on the surface of cell membranes. The mechanism by which GLP-1 controls blood sugar levels in the body is mainly to activate the GLP-1 receptors distributed in the pancreatic β cells to promote the biosynthesis and secretion of insulin. Meanwhile, GLP-1 polypeptide can inhibit the secretion of glucagon, gastric emptying and food intake in the presence of high blood sugar levels in the body, and enhance the degradation of glucose in the body through specific neurological actions. Notably, the physiological function of GLP-1 polypeptide in promoting insulin secretion is controlled by plasma glucose concentration. Therefore, compared with other diabetes treatment drugs, GLP-1 polypeptide does not cause severe and persistent hypoglycemia. In addition, it has been reported in the literature that GLP-1 polypeptide and its analogs have direct promoting effects on the growth, differentiation and proliferation of β cells in experimental animals, indicating that GLP-1 polypeptide and its analogs have the physiological functions of protecting pancreatic islets, delaying the progression of diabetes and inhibiting the apoptosis of β cells. GLP-1 polypeptide also has a potential effect on inhibiting gastrin and food-stimulated gastric acid secretion. These characteristics mean that GLP-1 polypeptide also has a physiological effect of preventing peptic ulcers. GLP-1 polypeptide can also activate its GLP-1 receptor distributed in the central nervous system of the brain to enhance satiety, reduce food intake and achieve the physiological effect of maintaining or reducing body weight. Therefore, the extensive mechanisms of action and physiological functions of GLP-1 polypeptide and its analogs mean that GLP-1 polypeptide is an ideal drug for the treatment of non-insulin-dependent diabetes mellitus and obese diabetes mellitus.

Glucose-dependent insulinotropic polypeptide (GIP) and GLP-1 polypeptide are both incretins, which play a key physiological role in the metabolism of blood glucose in the body. GIP is mainly composed of 42 amino acid residues in the body and is secreted by K cells in the duodenum and adjacent jejunum according to the level of glucose in plasma. GIP polypeptide exerts its physiological effects by binding to its GIP receptors distributed in pancreatic β cells, adipose tissue and the central nervous system. Similar to GLP-1 polypeptide, GIP polypeptide can stimulate pancreatic β cells to secrete insulin, thereby lowering the concentration of blood glucose in plasma and can protect pancreatic β cells, thereby controlling the metabolism of glucose in the body. In addition, the physiological functions of GIP polypeptide also include activating its GIP receptor in adipose tissue to promote the metabolism of fat. Interestingly, intracerebroventricular injection of GIP polypeptide in mice can reduce the food intake and body weight of the test animals, which seems to suggest that GIP polypeptide also has some specific physiological functions in reducing body weight. Studies have shown that the incretin function of GIP polypeptide is greatly reduced in patients with non-insulin-dependent diabetes mellitus, resulting in the lack or loss of incretin effect in patients. Studies have shown that the inhibitory properties of GIP polypeptide produced by these diabetic patients are greatly diminished when blood sugar levels return to normal.

Glucagon (GCG) is secreted by pancreatic α cells and mainly acts on glucagon receptors in the liver to raise blood glucose levels. In addition to raising blood sugar levels, recent studies have shown that glucagon can promote fat and amino acid metabolism, increase energy expenditure, and suppress appetite. Therefore, activation of glucagon receptors also has the potential to treat metabolic diseases such as obesity and non-alcoholic steatohepatitis. When activation of glucagon receptors is combined with the activation of GLP-1 and GIP receptors, it can have unparalleled therapeutic effects on metabolic diseases. Currently, no triagonist products targeting GLP-1, GIP, and GCG receptors have been approved for marketing.

The present disclosure provides compounds that have an agonist effect on human GLP-1 receptor, human GIP receptor, and human GCG receptor. In addition, some of the compounds of the present disclosure have stronger efficacy in reducing body weight than other triagonist products in the art. Finally, some of the compounds of the present disclosure have extremely high plasma stability and good druggability.

### SUMMARY OF THE INVENTION

The purpose of the present disclosure is to provide compounds of the structure shown in formula (I) or pharmaceutically acceptable salts thereof, GLP-1/GIP/GCG receptor triagonists, pharmaceutical compositions, medical uses, and methods of treating diseases.

In one aspect, the present disclosure provides a compound comprising a polypeptide sequence.

In another aspect, the present disclosure provides a compound comprising a polypeptide sequence and a substituent, wherein the substituent is linked to an amino acid residue in the polypeptide sequence, for example, via a functional group on an amino acid side chain.

### Compounds represented by formula (1)

In some embodiments, a compound represented by formula (I) or a pharmaceutically acceptable salt thereof is provided:

R₁-X₁-X₂-X₃-Gly-Thr-Phe-Thr-Ser-Asp-X₁₀-Ser-X₁₂-X₁₃-X₁₄-Glu-X₁₆-X₁₇-X₁₈-X₁₉-X₂₀-X₂₁-Phe-Val-Glu-Trp-Leu-X₂₇-X₂₈-X₂₉-Z-X₄₀-R₂ (SEQ ID NO: 145) (I);

wherein R₁ is hydrogen, alkyl, acetyl, formyl, benzoyl, trifluoroacetyl, or pGlu, and R₂ is -NH₂ or -OH;
X₁, X₂, X₃, X₁₀, X₁₂, X₁₃, X₁₄, X₁₆, X₁₇, X₁₈, X₁₉, X₂₀, X₂₁, X₂₇, X₂₈, X₂₉, Z, and X₄₀ are each independently selected from any natural amino acids, unnatural amino acids, or peptide segments composed of natural amino acids and/or unnatural amino acids.

In some embodiments, the compound represented by formula (I) or the pharmaceutically acceptable salt thereof, wherein:
R₁ is hydrogen, alkyl, acetyl, formyl, benzoyl, trifluoroacetyl, or pGlu, and R₂ is -NH₂ or -OH;
X₁ is selected from an amino acid residue of Tyr or His;
X₂ is an amino acid residue of Aib;
X₃ is selected from an amino acid residue of Gln or His;
X₁₀ is selected from an amino acid residue of Leu or Tyr;
X₁₂ is selected from an amino acid residue of Lys, alpha-methyl-Lys, or Ile;
X₁₃ is selected from an amino acid residue of Aib, Ile, Leu, Tyr, alpha-methyl-Tyr or alpha-methyl-Leu;
X₁₄ is selected from an amino acid residue of Leu or alpha-methyl-Leu;
X₁₆ is selected from an amino acid residue of Glu or Lys, or Y₁;
X₁₇ is selected from an amino acid residue of Glu, Arg, homoArg, Ile, Lys, Gln or alpha-methyl-Lys, or Y₁;
X₁₈ is selected from an amino acid residue of Ala or Aib;
X₁₉ is selected from an amino acid residue of Ala or Gln;
X₂₀ is selected from an amino acid residue of Gln, Aib, isovaline, alpha-methyl-Ser, 1-aminocyclopropane-1-carboxylic acid or 1-aminocyclobutane-1-carboxylic acid;
X₂₁ is selected from an amino acid residue of Glu, or Y₁;
X₂₇ is selected from an amino acid residue of Ile or Leu;
X₂₈ is selected from an amino acid residue of Ala or Glu;
X₂₉ is selected from an amino acid residue of Gly or D-Glu;
X₄₀ is selected from an amino acid residue of Lys, or Y₁, or is absent;
Z is selected from Gly-Pro-Ser-Ser-Gly-Ala-Pro-Pro-Pro-Ser (SEQ ID NO: 146), Gly-Pro-Ser-Ser-Gly-Ala-Pro-Pro-Ser (SEQ ID NO: 147), Pro-Ser-Ser-Gly-Ala-Pro-Pro-Pro-Ser (SEQ ID NO: 148), or Pro-Ser-Ser-Gly-Ala-Pro-Pro-Ser (SEQ ID NO: 149);
Y₁ is a Lys, Orn, Dap, Dab, or Cys residue with the side chain linked to a substituent.

In some embodiments, the amino acid at at least one of X₁₆, X₁₇ and X₂₁ is Y₁.

In some embodiments, X₁₆ is Y₁. In some embodiments, X₁₇ is Y₁.

In some embodiments, the present disclosure provides a compound represented by formula (Ia) or a pharmaceutically acceptable salt thereof:

R₁-X₁-X₂-X₃-Gly-Thr-Phe-Thr-Ser-Asp-X₁₀-Ser-X₁₂-X₁₃-X₁₄-Glu-Y₁-X₁₇-X₁₈-X₁₉-X₂₀-Glu-Phe-Val-Glu-Trp-Leu-X₂₇-X₂₈-X₂₉-Z-X₄₀-R₂ (SEQ ID NO: 151) (Ia);

wherein R₁ is hydrogen, alkyl, acetyl, formyl, benzoyl, trifluoroacetyl, or pGlu, and R₂ is -NH₂ or -OH;
X₁ is selected from an amino acid residue of Tyr or His;
X₂ is an amino acid residue of Aib;
X₃ is selected from an amino acid residue of Gln or His;
X₁₀ is selected from an amino acid residue of Leu or Tyr;
X₁₂ is selected from an amino acid residue of Lys, alpha-methyl-Lys, or Ile;
X₁₃ is selected from an amino acid residue of Aib, Ile, Leu, Tyr, alpha-methyl-Tyr or alpha-methyl-Leu;
X₁₄ is selected from an amino acid residue of Leu or alpha-methyl-Leu;
X₁₇ is selected from an amino acid residue of Glu, Arg, homoArg, Ile, Lys, Gln, or alpha-methyl-Lys;
X₁₈ is selected from an amino acid residue of Ala or Aib;
X₁₉ is selected from an amino acid residue of Ala or Gln;
X₂₀ is selected from an amino acid residue of Gln, Aib, isovaline, alpha-methyl-Ser, 1-aminocyclopropane-1-carboxylic acid or 1-aminocyclobutane-1-carboxylic acid;
X₂₇ is selected from an amino acid residue of Ile or Leu;
X₂₈ is selected from an amino acid residue of Ala or Glu;
X₂₉ is selected from an amino acid residue of Gly or D-Glu;
X₄₀ is selected from an amino acid residue of Lys, or is absent;
Z is selected from Gly-Pro-Ser-Ser-Gly-Ala-Pro-Pro-Pro-Ser (SEQ ID NO: 146), Gly-Pro-Ser-Ser-Gly-Ala-Pro-Pro-Ser (SEQ ID NO: 147), Pro-Ser-Ser-Gly-Ala-Pro-Pro-Pro-Ser (SEQ ID NO: 148), or Pro-Ser-Ser-Gly-Ala-Pro-Pro-Ser (SEQ ID NO: 149).
Y₁ is a Lys, Orn, Dap, Dab, or Cys residue with the side chain linked to a substituent.

In some embodiments, the compound represented by formula (Ia), R₁ is hydrogen.

In some embodiments, the compound represented by formula (Ia), R₂ is -NH₂ or -OH.

In some embodiments, the compound represented by formula (Ia), R₂ is -NH₂.

In some embodiments, the compound represented by formula (Ia), Z is Gly-Pro-Ser-Ser-Gly-Ala-Pro-Pro-Pro-Ser (SEQ ID NO: 146).

In some embodiments, the compound represented by formula (Ia), X₄₀ is absent.

In some embodiments, the compound represented by formula (Ia), Y₁ is a Lys residue with the side chain linked to a substituent.

In some embodiments, the present disclosure provides a compound represented by formula (Ib) or a pharmaceutically acceptable salt thereof:

R₁-X₁-X₂-X₃-Gly-Thr-Phe-Thr-Ser-Asp-X₁₀-Ser-X₁₂-X₁₃-X₁₄-Glu-Y₁-X₁₇-X₁₈-X₁₉-X₂₀-Glu-Phe-Val-Glu-Trp-Leu-X₂₇-X₂₈-X₂₉-Gly-Pro-Ser-Ser-Gly-Ala-Pro-Pro-Pro-Ser-R₂ (SEQ ID NO: 150) (Ib);

wherein R₁ is hydrogen, alkyl, acetyl, formyl, benzoyl, trifluoroacetyl, or pGlu, and R₂ is -NH₂ or -OH;
X₁ is selected from an amino acid residue of Tyr or His;
X₂ is an amino acid residue of Aib;
X₃ is selected from an amino acid residue of Gln or His;
X₁₀ is selected from an amino acid residue of Leu or Tyr;
X₁₂ is selected from an amino acid residue of Lys, alpha-methyl-Lys, or Ile;
X₁₃ is selected from an amino acid residue of Aib, Ile, Leu, Tyr, alpha-methyl-Tyr or alpha-methyl-Leu;
X₁₄ is selected from an amino acid residue of Leu or alpha-methyl-Leu;
X₁₇ is selected from an amino acid residue of Ile, Lys, Gln, or alpha-methyl-Lys;
X₁₈ is selected from an amino acid residue of Ala or Aib;
X₁₉ is selected from an amino acid residue of Ala or Gln;
X₂₀ is selected from an amino acid residue of Gln, Aib, isovaline, alpha-methyl-Ser, 1-aminocyclopropane-1-carboxylic acid or 1-aminocyclobutane-1-carboxylic acid;
X₂₇ is selected from an amino acid residue of Ile or Leu;
X₂₈ is selected from an amino acid residue of Ala or Glu;
X₂₉ is selected from an amino acid residue of Gly or D-Glu;
Y₁ is a Lys, Orn, Dap, Dab, or Cys residue with the side chain linked to a substituent.

In some embodiments, the compound represented by formula (Ib), R₁ is hydrogen.

In some embodiments, the compound represented by formula (Ib), R₂ is -NH₂ or -OH.

In some embodiments, the compound represented by formula (Ib), R₂ is -NH₂.

In some embodiments, the compound represented by formula (Ib), Y₁ is a Lys residue with the side chain linked to a substituent.

In some embodiments, the compound of the structure shown in any of the foregoing embodiments, X₁, X₂ and X₃ are selected from any of the following:
X₁ is His, X₂ is Aib, and X₃ is His;
X₁ is Tyr, X₂ is Aib, and X₃ is Gln; or
X₁ is His, X₂ is Aib, and X₃ is Gln.

In some embodiments, the compound of the structure shown in any of the foregoing embodiments,
X₁ is His, X₂ is Aib, and X₃ is His; or
X₁ is His, X₂ is Aib, and X₃ is Gln.

In some embodiments, the compound of the structure shown in any of the foregoing embodiments, X₁, X₂ and X₃ are His, Aib and His, respectively.

In some embodiments, the compound of the structure shown in any of the foregoing embodiments, wherein X₁₇ is a Lys residue.

In some embodiments, X₁₇, X₁₈, X₁₉ and X₂₀ are selected from any of the following:
X₁₇ is Lys, X₁₈ is Ala, X₁₉ is Ala, and X₂₀ is Gln;
X₁₇ is Lys, X₁₈ is Ala, X₁₉ is Ala, and X₂₀ is alpha-methyl-Ser;
X₁₇ is Lys, X₁₈ is Ala, X₁₉ is Ala, and X₂₀ is isovaleine;
X₁₇ is Lys, X₁₈ is Ala, X₁₉ is Ala, and X₂₀ is 1-aminocyclopropane-1-carboxylic acid;
X₁₇ is Lys, X₁₈ is Ala, X₁₉ is Ala, and X₂₀ is 1-aminocyclobutane-1-carboxylic acid;
X₁₇ is Lys, X₁₈ is Ala, X₁₉ is Ala, and X₂₀ is Aib;
X₁₇ is Lys, X₁₈ is Aib, X₁₉ is Ala, and X₂₀ is Gln; and
X₁₇ is Lys, X₁₈ is Ala, X₁₉ is Gln, and X₂₀ is Gln.

In some embodiments, the compound of the structure shown in any of the foregoing embodiments, wherein X₁₇ is a Gln residue. In some embodiments, X₁₇, X₁₈, X₁₉ and X₂₀ are selected from any of the following:
X₁₇ is Gln, X₁₈ is Ala, X₁₉ is Ala, and X₂₀ is Gln;
X₁₇ is Gln, X₁₈ is Ala, X₁₉ is Ala, and X₂₀ is isovaleine;
X₁₇ is Gln, X₁₈ is Ala, X₁₉ is Ala, and X₂₀ is 1-aminocyclopropane-1-carboxylic acid; and
X₁₇ is Gln, X₁₈ is Ala, X₁₉ is Ala, and X₂₀ is 1-aminocyclobutane-1-carboxylic acid.

In some embodiments, the compound of the structure shown in any of the foregoing embodiments, wherein X₁₇ is alpha-methyl-Lys.

In some embodiments, X₁₇, X₁₈, X₁₉ and X₂₀ are alpha-methyl-Lys, Ala, Ala and Gln, respectively. In some embodiments, the compound of the structure shown in any of the foregoing embodiments, wherein X₁₇ is Ile. In some embodiments, X₁₇, X₁₈, X₁₉ and X₂₀ are Ile, Ala, Ala and Gln, respectively.

In some embodiments, the compound of the structure shown in any of the foregoing embodiments, X₁₂ is an Ile residue.

In some embodiments, X₁₂, X₁₃ and X₁₄ are selected from any of the following:
X₁₂ is Ile, X₁₃ is Leu, and X₁₄ is Leu; or
X₁₂ is Ile, X₁₃ is alpha-methyl-Leu, and X₁₄ is Leu.

In some embodiments, the compound of the structure shown in any of the foregoing embodiments, wherein X₁₂ is a Lys residue.

In some embodiments, X₁₂, X₁₃ and X₁₄ are selected from any of the following:
X₁₂ is Lys, X₁₃ is alpha-methyl-Tyr, and X₁₄ is Leu;
X₁₂ is Lys, X₁₃ is Aib, and X₁₄ is Leu;
X₁₂ is Lys, X₁₃ is Tyr, and X₁₄ is alpha-methyl-Leu; and
X₁₂ is Lys, X₁₃ is Tyr, and X₁₄ is Leu.

In some embodiments, the compound of the structure shown in any of the foregoing embodiments, wherein X₁₂ is an alpha-methyl-Lys residue.

In some embodiments, X₁₂, X₁₃ and X₁₄ are selected from any of the following:
X₁₂ is alpha-methyl-Lys, X₁₃ is Tyr, and X₁₄ is Leu;
X₁₂ is alpha-methyl-Lys, X₁₃ is Leu, and X₁₄ is Leu; and
X₁₂ is alpha-methyl-Lys, X₁₃ is Ile, and X₁₄ is Leu.

In some embodiments, the compound of the structure shown in any of the foregoing embodiments, X₁ to X₂₉ are selected from any of the following groups:
(i) X₁, X₂ and X₃ are selected from any of the following:
   X₁ is His, X₂ is Aib, and X₃ is His;
   X₁ is His, X₂ is Aib, and X₃ is Gln; or
   X₁ is Tyr, X₂ is Aib, and X₃ is Gln;
   X₁₀ is selected from an amino acid residue of Leu or Tyr,
   X₁₂ is selected from an amino acid residue of Lys, alpha-methyl-Lys, or Ile,
   X₁₃ is selected from an amino acid residue of Aib, Ile, Leu, Tyr, alpha-methyl-Tyr or alpha-methyl-Leu,
   X₁₄ is selected from an amino acid residue of Leu or alpha-methyl-Leu,
   X₁₆ is Y₁,
   X₁₇ is selected from an amino acid residue of Lys,
   X₁₈ is selected from an amino acid residue of Ala or Aib,
   X₁₉ is selected from an amino acid residue of Ala or Gln,
   X₂₀ is selected from an amino acid residue of Gln, Aib, isovaline, alpha-methyl-Ser, 1-aminocyclopropane-1-carboxylic acid or 1-aminocyclobutane-1-carboxylic acid,
   X₂₇ is selected from an amino acid residue of Ile or Leu,
   X₂₈ is selected from an amino acid residue of Ala or Glu, and X₂₉ is selected from an amino acid residue of Gly or D-Glu;

In some embodiments,
X₁ is His, X₂ is Aib, and X₃ is His; or
X₁ is His, X₂ is Aib, and X₃ is Gln;
(ii) X₁, X₂ and X₃ are selected from any of the following:
   X₁ is His, X₂ is Aib, and X₃ is His;
   X₁ is His, X₂ is Aib, and X₃ is Gln; or
   X₁ is Tyr, X₂ is Aib, and X₃ is Gln;
   X₁₀ is selected from an amino acid residue of Leu or Tyr,
   X₁₂ is selected from an amino acid residue of Lys, alpha-methyl-Lys, or Ile,
   X₁₃ is selected from an amino acid residue of Aib, Ile, Leu, Tyr, alpha-methyl-Tyr or alpha-methyl-Leu,
   X₁₄ is selected from an amino acid residue of Leu or alpha-methyl-Leu,
   X₁₆ is Y₁, X₁₇ is selected from an amino acid residue of Lys,
   X₁₈ is selected from an amino acid residue of Ala or Aib,
   X₁₉ is selected from an amino acid residue of Ala or Gln,
   X₂₀ is selected from an amino acid residue of Gln,
   X₂₇ is selected from an amino acid residue of Ile or Leu,
   X₂₈ is selected from an amino acid residue of Ala or Glu,
   X₂₉ is selected from an amino acid residue of Gly or D-Glu;
(iii) X₁, X₂ and X₃ are selected from any of the following:
   X₁ is His, X₂ is Aib, and X₃ is His;
   X₁ is His, X₂ is Aib, and X₃ is Gln; or
   X₁ is Tyr, X₂ is Aib, and X₃ is Gln;
   X₁₀ is selected from an amino acid residue of Leu or Tyr,
   X₁₂ is selected from an amino acid residue of alpha-methyl-Lys or Ile,
   X₁₃ is selected from an amino acid residue of Ile, Leu or alpha-methyl-Leu,
   X₁₄ is selected from an amino acid residue of Leu or alpha-methyl-Leu,
   X₁₆ is Y₁, X₁₇ is selected from an amino acid residue of Lys,
   X₁₈ is selected from an amino acid residue of Ala,
   X₁₉ is selected from an amino acid residue of Ala,
   X₂₀ is selected from an amino acid residue of Aib, isovaline, alpha-methyl-Ser, 1-aminocyclopropane-1-carboxylic acid or 1-aminocyclobutane-1-carboxylic acid,
   X₂₇ is selected from an amino acid residue of Ile or Leu,
   X₂₈ is selected from an amino acid residue of Ala or Glu,
   X₂₉ is selected from an amino acid residue of Gly or D-Glu;

In some embodiments,
X₁ is His, X₂ is Aib, and X₃ is His; or
X₁ is His, X₂ is Aib, and X₃ is Gln;
(iv) X₁ is His;
X₂ is Aib;
X₃ is His;
X₁₀ is selected from an amino acid residue of Tyr,
X₁₂ is selected from an amino acid residue of Lys or alpha-methyl-Lys,
X₁₃ is selected from an amino acid residue of Tyr,
X₁₄ is selected from an amino acid residue of Leu or alpha-methyl-Leu,
X₁₆ is Y₁, X₁₇ is selected from an amino acid residue of Lys,
X₁₈ is selected from an amino acid residue of Ala or Aib,
X₁₉ is selected from an amino acid residue of Ala or Gln,
X₂₀ is selected from an amino acid residue of Gln,
X₂₇ is selected from an amino acid residue of Ile or Leu,
X₂₈ is selected from an amino acid residue of Ala or Glu,
X₂₉ is selected from an amino acid residue of Gly;
(v) X₁ is His;
X₂ is Aib;
X₃ is His or Gln;
X₁₀ is selected from an amino acid residue of Leu or Tyr,
X₁₂ is selected from an amino acid residue of Ile,
X₁₃ is selected from an amino acid residue of alpha-methyl-Leu,
X₁₄ is selected from an amino acid residue of Leu,
X₁₆ is Y₁, X₁₇ is selected from an amino acid residue of Lys,
X₁₈ is selected from an amino acid residue of Ala,
X₁₉ is selected from an amino acid residue of Ala,
X₂₀ is selected from an amino acid residue of Gln, Aib, 1-aminocyclobutane-1-carboxylic acid, or isovaleine,
X₂₇ is selected from an amino acid residue of Leu,
X₂₈ is selected from an amino acid residue of Ala,
X₂₉ is selected from an amino acid residue of Gly or D-Glu;
(vi) X₁, X₂ and X₃ are selected from any of the following:
   X₁ is His, X₂ is Aib, and X₃ is His;
   X₁ is His, X₂ is Aib, and X₃ is Gln; or
   X₁ is Tyr, X₂ is Aib, and X₃ is Gln;
   X₁₀ is selected from an amino acid residue of Leu or Tyr,
   X₁₂ is selected from an amino acid residue of alpha-methyl-Lys or Ile,
   X₁₃ is selected from an amino acid residue of Leu;
   X₁₄ is selected from an amino acid residue of Leu,
   X₁₆ is Y₁, X₁₇ is selected from an amino acid residue of Ile, Lys or Gln,
   X₁₈ is selected from an amino acid residue of Ala,
   X₁₉ is selected from an amino acid residue of Ala,
   X₂₀ is selected from an amino acid residue of Gln, Aib, isovaline, alpha-methyl-Ser, 1-aminocyclopropane-1-carboxylic acid or 1-aminocyclobutane-1-carboxylic acid,
   X₂₇ is selected from an amino acid residue of Ile or Leu,
   X₂₈ is selected from an amino acid residue of Ala or Glu,
   X₂₉ is selected from an amino acid residue of Gly or D-Glu;

In some embodiments,
X₁ is His, X₂ is Aib, and X₃ is His; or
X₁ is His, X₂ is Aib, and X₃ is Gln;
(vii) X₁ is His;
X₂ is Aib;
X₃ is His;
X₁₀ is selected from an amino acid residue of Tyr,
X₁₂ is selected from an amino acid residue of Lys,
X₁₃ is selected from an amino acid residue of alpha-methyl-Tyr or Aib;
X₁₄ is selected from an amino acid residue of Leu,
X₁₆ is Y₁,
X₁₇ is selected from an amino acid residue of Lys,
X₁₈ is selected from an amino acid residue of Ala,
X₁₉ is selected from an amino acid residue of Ala,
X₂₀ is selected from an amino acid residue of Gln,
X₂₇ is selected from an amino acid residue of Leu,
X₂₈ is selected from an amino acid residue of Ala,
X₂₉ is selected from an amino acid residue of Gly;
(viii) X₁, X₂ and X₃ are selected from any of the following:
   X₁ is His, X₂ is Aib, and X₃ is His; or
   X₁ is Tyr, X₂ is Aib, and X₃ is Gln;
   X₁₀ is selected from an amino acid residue of Leu or Tyr,
   X₁₂ is selected from an amino acid residue of alpha-methyl-Lys,
   X₁₃ is selected from an amino acid residue of Ile;
   X₁₄ is selected from an amino acid residue of Leu,
   X₁₆ is Y₁,
   X₁₇ is selected from an amino acid residue of Lys,
   X₁₈ is selected from an amino acid residue of Ala,
   X₁₉ is selected from an amino acid residue of Ala,
   X₂₀ is selected from an amino acid residue of Gln or isovaleine,
   X₂₇ is selected from an amino acid residue of Leu or Ile,
   X₂₈ is selected from an amino acid residue of Ala or Glu,
   X₂₉ is selected from an amino acid residue of Gly;
   In some embodiments, X₁ is His, X₂ is Aib, and X₃ is His;
   (ix) X₁ is His;
   X₂ is Aib;
   X₃ is His;
   X₁₀ is selected from an amino acid residue of Leu or Tyr,
   X₁₂ is selected from an amino acid residue of Lys, alpha-methyl-Lys, or Ile,
   X₁₃ is selected from an amino acid residue of Ile, Leu or Tyr,
   X₁₄ is selected from an amino acid residue of Leu,
   X₁₆ is Y₁, X₁₇ is selected from an amino acid residue of Ile, Gln or alpha-methyl-Lys,
   X₁₈ is selected from an amino acid residue of Ala,
   X₁₉ is selected from an amino acid residue of Ala,
   X₂₀ is selected from an amino acid residue of Gln, isovaline, 1-aminocyclopropane-1-carboxylic acid or 1-aminocyclobutane-1-carboxylic acid,
   X₂₇ is selected from an amino acid residue of Leu,
   X₂₈ is selected from an amino acid residue of Ala,
   X₂₉ is selected from an amino acid residue of Gly or D-Glu;
   (x) X₁ is His;
   X₂ is Aib;
   X₃ is His;
   X₁₀ is selected from an amino acid residue of Leu or Tyr,
   X₁₂ is selected from an amino acid residue of alpha-methyl-Lys or Ile,
   X₁₃ is selected from an amino acid residue of Ile or Leu,
   X₁₄ is selected from an amino acid residue of Leu,
   X₁₆ is Y₁,
   X₁₇ is selected from an amino acid residue of Gln,
   X₁₈ is selected from an amino acid residue of Ala,
   X₁₉ is selected from an amino acid residue of Ala,
   X₂₀ is selected from an amino acid residue of Gln, isovaline, 1-aminocyclopropane-1-carboxylic acid or 1-aminocyclobutane-1-carboxylic acid,
   X₂₇ is selected from an amino acid residue of Leu,
   X₂₈ is selected from an amino acid residue of Ala,
   X₂₉ is selected from an amino acid residue of Gly or D-Glu;
   (xi) X₁ is His;
   X₂ is Aib;
   X₃ is His;
   X₁₀ is selected from an amino acid residue of Tyr,
   X₁₂ is selected from an amino acid residue of Lys or alpha-methyl-Lys,
   X₁₃ is selected from an amino acid residue of Leu or Tyr,
   X₁₄ is selected from an amino acid residue of Leu,
   X₁₆ is Y₁,
   X₁₇ is selected from an amino acid residue of Ile or alpha-methyl-Lys,
   X₁₈ is selected from an amino acid residue of Ala,
   X₁₉ is selected from an amino acid residue of Ala,
   X₂₀ is selected from an amino acid residue of Gln,
   X₂₇ is selected from an amino acid residue of Leu,
   X₂₈ is selected from an amino acid residue of Ala,
   X₂₉ is selected from an amino acid residue of Gly.

In some embodiments, the amino acid combination as shown in any of (i) to (xi) above, wherein Z is selected from Gly-Pro-Ser-Ser-Gly-Ala-Pro-Pro-Pro-Ser (SEQ ID NO: 146), Gly-Pro-Ser-Ser-Gly-Ala-Pro-Pro-Ser (SEQ ID NO: 147), Pro-Ser-Ser-Gly-Ala-Pro-Pro-Pro-Ser (SEQ ID NO: 148) or Pro-Ser-Ser-Gly-Ala-Pro-Pro-Ser (SEQ ID NO: 149). In some embodiments, Z is Gly-Pro-Ser-Ser-Gly-Ala-Pro-Pro-Pro-Ser (SEQ ID NO: 146).

In some embodiments, the amino acid combination as shown in any of (i) to (xi) above, wherein X₄₀ is selected from an amino acid residue of Lys or is absent. In some embodiments, X₄₀ is absent.

In some embodiments, the amino acid combinations as shown in any of (i) to (xi) above, wherein Y₁ is a Lys, Orn, Dap, Dab, or Cys residue with the side chain linked to a substituent. In some embodiments, Y₁ is a Lys residue with the side chain linked to a substituent.

In some embodiments, the amino acid combination as shown in any of (i) to (xi) above, wherein R₁ is hydrogen, alkyl, acetyl, formyl, benzoyl, trifluoroacetyl, or pGlu, and R₂ is -NH₂ or -OH. In some embodiments, R₁ is hydrogen, and R₂ is -NH₂ or -OH. In some embodiments, R₁ is hydrogen, and R₂ is -NH₂.

In some embodiments, the compound represented by formula (I), formula (Ia), or formula (Ib), comprising (or being) any of the polypeptide sequences shown below:

**Table 1**

| Polypeptide Sequence | SEQ ID NO: |
|---|---|
| H-Aib-HGTFTSDYS-αK-YLEEKAAQEFVEWLLAGGPSSGAPPPS-NH₂ | 1 |
| H-Aib-HGTFTSDYS-αK-YLEKKAAQEFVEWLLAGGPSSGAPPPS-NH₂ | 2 |
| H-Aib-HGTFTSDYSK-αY-LEKKAAQEFVEWLLAGGPSSGAPPPS-NH₂ | 3 |
| H-Aib-HGTFTSDYSK-Aib-LEKKAAQEFVEWLLAGGPSSGAPPPS-NH₂ | 4 |
| H-Aib-HGTFTSDYSKY-αL-EKKAAQEFVEWLLAGGPSSGAPPPS-NH₂ | 5 |
| H-Aib-HGTFTSDYSKYLEK-αK-AAQEFVEWLLAGGPSSGAPPPS-NH₂ | 6 |
| H-Aib-HGTFTSDYSKYLEKK-Aib-AQEFVEWLLAGGPSSGAPPPS-NH₂ | 7 |
| H-Aib-HGTFTSDYS-αK-YLEKKAAQEFVEWLIAGGPSSGAPPPS-NH₂ | 8 |
| H-Aib-HGTFTSDYS-αK-LLEKKAAQEFVEWLLAGGPSSGAPPPS-NH₂ | 9 |
| H-Aib-HGTFTSDYS-αK-YLEKKAQQEFVEWLLAGGPSSGAPPPS-NH₂ | 10 |
| Y-Aib-QGTFTSDYS-αK-LLEKKAAQEFVEWLLAGGPSSGAPPPS-NH₂ | 11 |
| H-Aib-HGTFTSDYS-αK-YLEKKAAQEFVEWLIEGGPSSGAPPPS-NH₂ | 12 |
| H-Aib-HGTFTSDYS-αK-LLEKQAAQEFVEWLLAGGPSSGAPPPS-NH₂ | 13 |
| H-Aib-HGTFTSDYS-αK-LLEKIAAQEFVEWLLAGGPSSGAPPPS-NH₂ | 14 |
| H-Aib-HGTFTSDYS-αK-ILEKKAAQEFVEWLLAGGPSSGAPPPS-NH₂ | 15 |
| H-Aib-HGTFTSDYSILLEKKAAQEFVEWLLAGGPSSGAPPPS-NH₂ | 16 |
| H-Aib-HGTFTSDYSI-αL-LEKKAAQEFVEWLLAGGPSSGAPPPS-NH₂ | 17 |
| H-Aib-HGTFTSDYS-αK-LLEKKAA-αS-EFVEWLIEGGPSSGAPPPS-NH₂ | 18 |
| Y-Aib-QGTFTSDYS-αK-LLEKKAA-Iva-EFVEWLLAGGPSSGAPPPS-NH₂ | 19 |
| H-Aib-HGTFTSDYS-αK-LLEKQAA-Iva-EFVEWLLAGGPSSGAPPPS-NH₂ | 20 |
| Y-Aib-QGTFTSDYS-αK-ILEKKAA-Iva-EFVEWLLAGGPSSGAPPPS-NH₂ | 21 |
| H-Aib-HGTFTSDYS-αK-ILEKQAA-Iva-EFVEWLLAGGPSSGAPPPS-NH₂ | 22 |
| H-Aib-HGTFTSDYS-αK-LLEKKAA-Iva-EFVEWLIEGGPSSGAPPPS-NH₂ | 23 |
| H-Aib-HGTFTSDYS-αK-ILEKKAA-Iva-EFVEWLIEGGPSSGAPPPS-NH₂ | 24 |
| H-Aib-HGTFTSDLS-αK-LLEKKAA-Iva-EFVEWLIEGGPSSGAPPPS-NH₂ | 25 |
| H-Aib-HGTFTSDLS-αK-ILEKKAA-Iva-EFVEWLIEGGPSSGAPPPS-NH₂ | 26 |
| H-Aib-HGTFTSDYSILLEKKAA-Iva-EFVEWLLAGGPSSGAPPPS-NH₂ | 27 |
| H-Aib-HGTFTSDLSILLEKKAA-Iva-EFVEWLLAGGPSSGAPPPS-NH₂ | 28 |
| H-Aib-HGTFTSDYSI-αL-LEKKAA-Iva-EFVEWLLAGGPSSGAPPPS-NH₂ | 29 |
| H-Aib-HGTFTSDLSI-αL-LEKKAA-Iva-EFVEWLLAGGPSSGAPPPS-NH₂ | 30 |
| H-Aib-HGTFTSDYSI-αL-LEKKAAQEFVEWLLAeGPSSGAPPPS-NH₂ | 31 |
| H-Aib-HGTFTSDLSI-αL-LEKKAA-Ac4c-EFVEWLLAGGPSSGAPPPS-NH₂ | 32 |
| H-Aib-HGTFTSDLSI-αL-LEKKAAQEFVEWLLAeGPSSGAPPPS-NH₂ | 33 |
| H-Aib-HGTFTSDLSI-αL-LEKKAAQEFVEWLLAGGPSSGAPPPS-NH₂ | 34 |
| H-Aib-HGTFTSDLSILLEKKAAQEFVEWLLAeGPSSGAPPPS-NH₂ | 35 |
| H-Aib-HGTFTSDLSILLEKQAA-Ac4c-EFVEWLLAeGPSSGAPPPS-NH₂ | 36 |
| H-Aib-HGTFTSDLSILLEKKAA-Ac3c-EFVEWLLAeGPSSGAPPPS-NH₂ | 37 |
| H-Aib-HGTFTSDLSILLEKKAA-Ac4c-EFVEWLLAeGPSSGAPPPS-NH₂ | 38 |
| H-Aib-HGTFTSDLSILLEKQAA-Ac3c-EFVEWLLAeGPSSGAPPPS-NH₂ | 39 |
| H-Aib-HGTFTSDYSILLEKQAA-Ac3c-EFVEWLLAeGPSSGAPPPS-NH₂ | 40 |
| H-Aib-HGTFTSDYSILLEKQAA-Ac4c-EFVEWLLAeGPSSGAPPPS-NH₂ | 41 |
| H-Aib-HGTFTSDYSILLEKKAAQEFVEWLLAeGPSSGAPPPS-NH₂ | 42 |
| H-Aib-HGTFTSDYSILLEKQAAQEFVEWLLAGGPSSGAPPPS-NH₂ | 43 |
| H-Aib-HGTFTSDYSILLEKQAAQEFVEWLLAeGPSSGAPPPS-NH₂ | 44 |
| H-Aib-QGTFTSDYSILLEKKAAQEFVEWLLAGGPSSGAPPPS-NH₂ | 45 |
| H-Aib-QGTFTSDYSI-αL-LEKKAAQEFVEWLLAGGPSSGAPPPS-NH₂ | 46 |
| H-Aib-QGTFTSDYSILLEKKAA-Aib-EFVEWLLAGGPSSGAPPPS-NH₂ | 47 |
| H-Aib-QGTFTSDYSI-αL-LEKKAA-Aib-EFVEWLLAGGPSSGAPPPS-NH₂ | 48 |

In Table 1, αK is alpha-methyl-Lys, αY is alpha-methyl-Tyr, αL is alpha-methyl-Leu, Iva is isovaleine, e is D-Glu, Aib is α-aminoisobutyric acid, Ac3c is 1-aminocyclopropane-1-carboxylic acid, and Ac4c is 1-aminocyclobutane-1-carboxylic acid.

In some embodiments, the compound of the structure shown in any of the foregoing embodiments, further comprising a substituent. In some embodiments, the substituent is linked to an amino acid residue at any position. In some embodiments, the substituent is covalently linked to the side chain of the amino acid residue. For example, the substituent is covalently linked to the ε-amino group of the Lys residue.

In some embodiments, the substituent comprises the structure shown below: -Z₁-Z₂.

In some embodiments, Z₂ comprises (or is) a C₁₂-C₃₂ fatty acid. By way of example, examples of the C₁₂-C₃₂ fatty acid include, but are not limited to, palmitic acid (hexadecanoic acid) (C₁₆ monoacid), hexadecanedioic acid (C₁₆ diacid), margaric acid (heptadecanoic acid) (C₁₇ monoacid), heptadecanedioic acid (C₁₇ diacid), stearic acid (C₁₈ monoacid), octadecanedioic acid (C₁₈ diacid), nonadecylic acid (nonadecanoic acid) (C₁₉ monoacid), nonadecanedioic acid (C₁₉ diacid), arachidic acid (eicosanoic acid) (C₂₀ monoacid), eicosanedioic acid (C₂₀ diacid), heneicosylic acid (heneicosanoic acid) (C₂₁ monoacid), heneicosanedioic acid (C₂₁ diacid), behenic acid (docosanoic acid) (C₂₂ monoacid), and docosanedioic acid (C₂₂ diacid), including branched and substituted derivatives thereof.

In some embodiments, Z₁ may contain amino acids, amino polyethylene glycol, or blends thereof.

In some embodiments, Z₁ comprises (or is) 0-10 amino acid residues (e.g., 1, 2, 3, 4, 5, 6, 7, 8, 9 or 10) independently selected from Gly, Glu, γGlu, and OEG.

By way of example, when the linker comprises at least one amino acid, the amino acid may be 1 to 4 Glu or γGlu amino acid residues. In some embodiments, the linker may comprise one or two Glu or γGlu amino acid residues, including D forms thereof. For example, the linker may comprise one or two γGlu amino acid residues. Alternatively, the linker may comprise 1 to 4 amino acid residues (e.g., Glu or γGlu amino acids) used in combination with OEGs.

By way of example, the linker may be a combination of 1 to 4 Glu or γGlu amino acids and 1 to 4 OEGs. In other cases, the linker can be a combination of 1 or 2 γGlu amino acids and 1 or 2 OEGs.

As described in the present disclosure, the amino acid residue of "OEG" is [2-(2-amino-ethoxy)-ethoxy]-acetyl, and specifically, its structure is shown below:

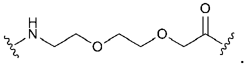

As described in the present disclosure, the structure of the amino acid residue of "γGlu" is shown below:

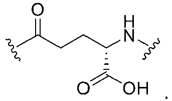

As described in the present disclosure, "pGlu" is polyglutamic acid with the structure Glu-(Glu)d-Glu. By way of example, pGlu has a molecular weight of 700,000 to 2,000,000 units (e.g., 100 units).

In some embodiments, Z₁ comprises the structure shown below: -(OEG)ₐ-(γGlu)_{b}-; wherein a is any integer between 0 and 4 (e.g., 0, 1, 2, 3, or 4), and b is any integer between 1 and 2 (e.g., 0, 1, or 2). In some specific embodiments, a is 1, 2 or 3, and b is 1 or 2. In some specific embodiments, a is 2 or 3, and b is 1 or 2. In some specific embodiments, a is 2, and b is 1 or 2. In some specific embodiments, a is 2, and b is 1.

In some embodiments, Z₂ comprises the structure shown below: -CO-(CH₂)_{c}-COOH, wherein c is any integer between 10 and 30 (e.g., 10, 11, 12, 13, 14, 15, 16, 17, 18, 19, 20, 21, 22, 23, 24, 25, 26, 27, 28, 29, or 30). In some specific embodiments, c is any integer between 16 and 20 (e.g., 16, 17, 18, 19, 20). In some specific embodiments, c is 16, 18, or 20. In some specific embodiments, c is 16 or 18.

In some embodiments, the substituent has the structure shown below: {[2-(2-amino-ethoxy)-ethoxy]-acetyl}ₐ-(γGlu)_{b}-CO-(CH₂)_{c}-COOH;
wherein a is any integer between 0 and 4 (e.g., 0, 1, 2, 3, or 4), b is any integer between 1 and 2 (e.g., 1 or 2), and c is any integer between 10 and 30 (e.g., 10, 11, 12, 13, 14, 15, 16, 17, 18, 19, 20, 21, 22, 23, 24, 25, 26, 27, 28, 29, or 30).

In some specific embodiments, a is 1, 2 or 3, b is 1 or 2, and c is any integer between 16 and 20 (e.g., 16, 17, 18, 19, 20).

In some specific embodiments, a is 2 or 3, b is 1 or 2, and c is 16, 18 or 20.

In some specific embodiments, a is 2, b is 1 or 2, and c is 16, 18 or 20.

In some specific embodiments, a is 2, b is 1 or 2, and c is 16 or 18.

In some specific embodiments, a is 2, b is 1, and c is 16 or 18.

In some specific embodiments, the substituent has the structure shown below: 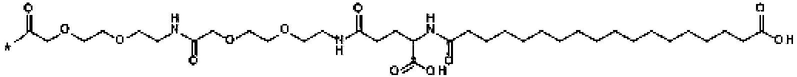 or

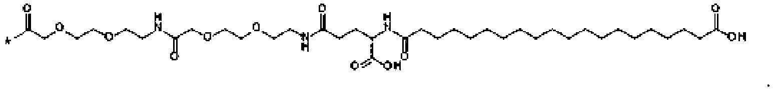

In some embodiments, the compound of the structure shown in any of the foregoing embodiments, X₁₆, X₂₁ and X₄₀ are each independently selected from Y₁.

In some embodiments, Y₁ is a Lys, Orn, Dap, Dab, or Cys residue with the side chain linked to a substituent. In some embodiments, the substituent has the structure: -Z₁-Z₂, wherein Z₁comprises 0-10 amino acid residues independently selected from Gly, Glu, γGlu, and OEG, and Z₂ is selected from C₁₂₋₃₂fatty acids.

In some embodiments, Z₁ comprises (or is) the structure shown below: -(OEG)ₐ-(γGlu)_{b}-; wherein a is any integer between 0 and 4 (e.g., 0, 1, 2, 3, or 4), and b is any integer between 1 and 2 (e.g. 1 or 2). In some embodiments, Z₂ comprises (or is) the structure shown below: -CO-(CH₂)_{c}-COOH, wherein c is any integer between 10 and 30 (e.g., 10, 11, 12, 13, 14, 15, 16, 17, 18, 19, 20, 21, 22, 23, 24, 25, 26, 27, 28, 29, or 30).

In some embodiments, the substituent comprises (or is) the structure shown below: {[2-(2-amino-ethoxy)-ethoxy]-acetyl}ₐ-(γGlu)₃-CO-(CH₂)_{c}-COOH;
wherein a is any integer between 0 and 4, b is any integer between 1 and 2, and c is any integer between 10 and 30. In some specific embodiments, a is 1, 2 or 3, b is 1 or 2, and c is any integer between 16 and 20 (e.g., 16, 17, 18, 19, 20). In some specific embodiments, a is 2 or 3, b is 1 or 2, and c is 16, 18 or 20. In some specific embodiments, a is 2, b is 1 or 2, and c is 16, 18 or 20. In some specific embodiments, a is 2, b is 1 or 2, and c is 16 or 18. In some specific embodiments, a is 2, b is 1, and c is 16 or 18.

In some embodiments, Y₁ is a Lys residue with the side chain linked to a substituent, the substituent being covalently linked to the ε-amino group of the Lys residue via an amide bond.

In some embodiments, Y₁ is K(-OEG-OEG-γGlu-C(O)-(CH₂)₁₆-COOH), K(-OEG-OEG-γGlu-C(O)-(CH₂)₁₈-COOH), or K(-OEG-OEG-γGlu-C(O)- (CH₂)₂₀-COOH).
Wherein K(-OEG-OEG-γGlu-C(O)-(CH₂)₁₆-COOH) has the structure shown below:

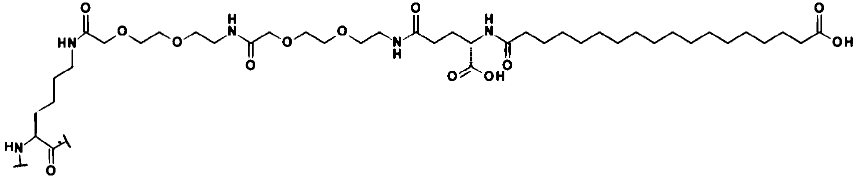

K(-OEG-OEG-γGlu-C(O)-(CH₂)₁₈-COOH) has the structure shown below:

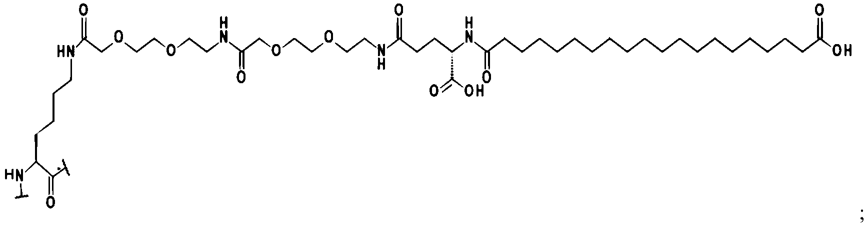

In some embodiments, Y₁ has the structure shown below:

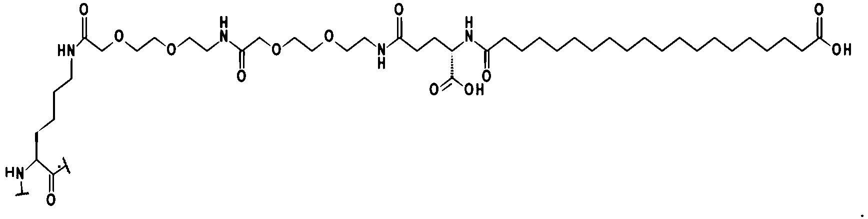

In some embodiments, the compound has the structure shown in formula (I) or a pharmaceutically acceptable salt thereof,

R₁-X₁-X₂-X₃-Gly-Thr-Phe-Thr-Ser-Asp-X₁₀-Ser-X₁₂-X₁₃-X₁₄-Glu-X₁₆-X₁₇-X₁₈-X₁₉-X₂₀-X₂₁-Phe-Val-Glu-Trp-Leu-X₂₇-X₂₈-X₂₉-Z-X₄₀-R₂ (SEQ ID NO: 145) (I);

wherein:
R₁ is hydrogen, alkyl, acetyl, formyl, benzoyl, trifluoroacetyl, or pGlu,
R₂ is -NH₂ or -OH;
X₁ is selected from an amino acid residue of Tyr or His;
X₂ is an amino acid residue of Aib;
X₃ is selected from an amino acid residue of Gln or His;
X₁₀ is selected from an amino acid residue of Leu or Tyr;
X₁₂ is selected from an amino acid residue of Lys, alpha-methyl-Lys, or Ile;
X₁₃ is selected from an amino acid residue of Aib, Ile, Leu, Tyr, alpha-methyl-Tyr or alpha-methyl-Leu;
X₁₄ is selected from an amino acid residue of Leu or alpha-methyl-Leu;
X₁₆ is selected from an amino acid residue of Glu or Lys, or Y₁;
X₁₇ is selected from an amino acid residue of Glu, Arg, homoArg, Ile, Lys, Gln or alpha-methyl-Lys, or Y₁;
X₁₈ is selected from an amino acid residue of Ala or Aib;
X₁₉ is selected from an amino acid residue of Ala or Gln;
X₂₀ is selected from an amino acid residue of Gln, Aib, isovaline, alpha-methyl-Ser, 1-aminocyclopropane-1-carboxylic acid or 1-aminocyclobutane-1-carboxylic acid;
X₂₁ is selected from an amino acid residue of Glu, or Y₁;
X₂₇ is selected from an amino acid residue of Ile or Leu;
X₂₈ is selected from an amino acid residue of Ala or Glu;
X₂₉ is selected from an amino acid residue of Gly or D-Glu;
X₄₀ is selected from an amino acid residue of Lys, or Y₁, or is absent;
Z is selected from Gly-Pro-Ser-Ser-Gly-Ala-Pro-Pro-Pro-Ser (SEQ ID NO: 146), Gly-Pro-Ser-Ser-Gly-Ala-Pro-Pro-Ser (SEQ ID NO: 147), Pro-Ser-Ser-Gly-Ala-Pro-Pro-Pro-Ser (SEQ ID NO: 148), or Pro-Ser-Ser-Gly-Ala-Pro-Pro-Ser (SEQ ID NO: 149).
Y₁ is a Lys, Orn, Dap, Dab, or Cys residue with the side chain linked to a substituent having the structure shown below: -Z₁-Z₂, wherein Z₁ comprises 0-10 amino acid residues independently selected from Gly, Glu, γGlu, and OEG, and Z₂ is selected from C₁₂₋₃₂ fatty acids. In some embodiments, Z₁ comprises (or is) the structure shown below: - (OEG)ₐ-(γGlu)_{b}-; wherein a is any integer between 0 and 4, and b is any integer between 1 and 2. In some embodiments, Z₂ comprises (or is) the structure shown below: -CO-(CH₂)_{c}-COOH, wherein c is any integer between 10 and 30.

In some embodiments, the substituent has the structure shown below:
{[2-(2-amino-ethoxy)-ethoxy]-acetyl}ₐ-(γGlu)_{b}-CO-(CH₂)_{c}-COOH;
wherein a is any integer between 0 and 4, b is any integer between 1 and 2, and c is any integer between 10 and 30. In some specific embodiments, a is 1, 2 or 3, b is 1 or 2, and c is any integer between 16 and 20 (e.g., 16, 17, 18, 19, 20). In some specific embodiments, a is 2 or 3, b is 1 or 2, and c is 16, 18 or 20. In some specific embodiments, a is 2, b is 1 or 2, and c is 16, 18 or 20. In some specific embodiments, a is 2, b is 1 or 2, and c is 16 or 18. In some specific embodiments, a is 2, b is 1, and c is 16 or 18.

In some embodiments, Y₁ is a Lys residue with the side chain linked to a substituent, the substituent being covalently linked to the ε-amino group of the Lys residue via an amide bond. In some embodiments, Y₁ is K(-OEG-OEG-γGlu-C(O)-(CH₂)₁₆-COOH), K(-OEG-OEG-γGlu-C(O)-(CH₂)₁₈-COOH), or K(-OEG-OEG-γGlu-C(O)-(CH₂)₂₀-COOH).

In some embodiments, the group has the structure shown below: 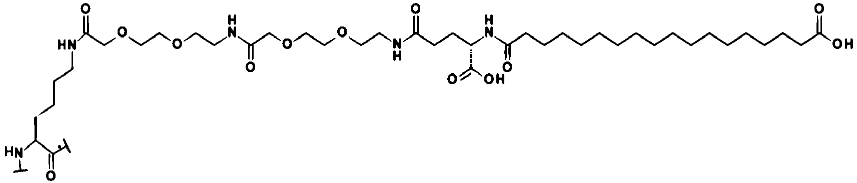 or

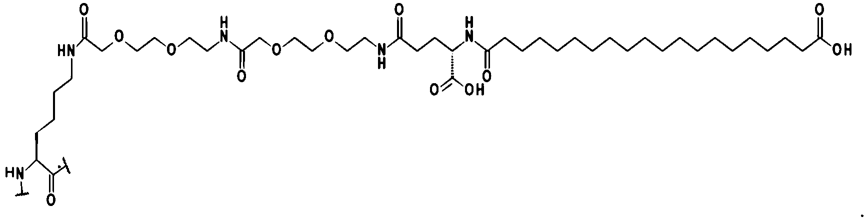

In some embodiments, the amino acid at at least one of X₁₆, X₁₇ and X₂₁ is Y₁. In some embodiments, X₁₆ is Y₁.

In some embodiments, the present disclosure provides a compound represented by formula (Ia) or a pharmaceutically acceptable salt thereof:

R₁-X₁-X₂-X₃-Gly-Thr-Phe-Thr-Ser-Asp-X₁₀-Ser-X₁₂-X₁₃-X₁₄-Glu-Y₁-X₁₇-X₁₈-X₁₉-X₂₀-Glu-Phe-Val-Glu-Trp-Leu-X₂₇-X₂₈-X₂₉-Z-X₄₀-R₂ (SEQ ID NO: 151) (Ia);

wherein R₁ is hydrogen, alkyl, acetyl, formyl, benzoyl, trifluoroacetyl, or pGlu,
R₂ is -NH₂ or -OH;
X₁ is selected from an amino acid residue of Tyr or His;
X₂ is an amino acid residue of Aib;
X₃ is selected from an amino acid residue of Gln or His;
X₁₀ is selected from an amino acid residue of Leu or Tyr;
X₁₂ is selected from an amino acid residue of Lys, alpha-methyl-Lys, or Ile;
X₁₃ is selected from an amino acid residue of Aib, Ile, Leu, Tyr, alpha-methyl-Tyr or alpha-methyl-Leu;
X₁₄ is selected from an amino acid residue of Leu or alpha-methyl-Leu;
X₁₇ is selected from an amino acid residue of Glu, Arg, homoArg, Ile, Lys, Gln, or alpha-methyl-Lys;
X₁₈ is selected from an amino acid residue of Ala or Aib;
X₁₉ is selected from an amino acid residue of Ala or Gln;
X₂₀ is selected from an amino acid residue of Gln, Aib, isovaline, alpha-methyl-Ser, 1-aminocyclopropane-1-carboxylic acid or 1-aminocyclobutane-1-carboxylic acid;
X₂₇ is selected from an amino acid residue of Ile or Leu;
X₂₈ is selected from an amino acid residue of Ala or Glu;
X₂₉ is selected from an amino acid residue of Gly or D-Glu;
X₄₀ is selected from an amino acid residue of Lys, or is absent;
Z is selected from Gly-Pro-Ser-Ser-Gly-Ala-Pro-Pro-Pro-Ser (SEQ ID NO: 146), Gly-Pro-Ser-Ser-Gly-Ala-Pro-Pro-Ser (SEQ ID NO: 147), Pro-Ser-Ser-Gly-Ala-Pro-Pro-Pro-Ser (SEQ ID NO: 148), or Pro-Ser-Ser-Gly-Ala-Pro-Pro-Ser (SEQ ID NO: 149);
Y₁ is a Lys, Orn, Dap, Dab, or Cys residue with the side chain linked to a substituent having the structure shown below: -Z₁-Z₂, wherein Z₁ comprises 0-10 amino acid residues independently selected from Gly, Glu, γGlu, and OEG ([2-(2-amino-ethoxy)-ethoxy]-acetyl), and Z₂ is selected from C₁₂₋₃₂ fatty acids. In some embodiments, Z₁ comprises (or is) the structure shown below: -(OEG)ₐ-(γGlu)_{b}-; wherein a is any integer between 0 and 4, and b is any integer between 1 and 2. In some embodiments, Z₂ comprises (or is) the structure shown below: -CO-(CH₂)_{c}-COOH, wherein c is any integer between 10 and 30.

In some embodiments, the substituent has the structure shown below:
{[2-(2-amino-ethoxy)-ethoxy]-acetyl}ₐ-(γGlu)_{b}-CO-(CH₂)_{c}-COOH;
wherein a is any integer between 0 and 4, b is any integer between 1 and 2, and c is any integer between 10 and 30. In some specific embodiments, a is 1, 2 or 3, b is 1 or 2, and c is any integer between 16 and 20 (e.g., 16, 17, 18, 19, 20). In some specific embodiments, a is 2 or 3, b is 1 or 2, and c is 16, 18 or 20. In some specific embodiments, a is 2, b is 1 or 2, and c is 16, 18 or 20. In some specific embodiments, a is 2, b is 1 or 2, and c is 16 or 18. In some specific embodiments, a is 2, b is 1, and c is 16 or 18.

In some embodiments, Y₁ is a Lys residue with the side chain linked to a substituent, the substituent being covalently linked to the ε-amino group of the Lys residue via an amide bond. In some embodiments, Y₁ is K(-OEG-OEG-γGlu-C(O)-(CH₂)₁₆-COOH), K(-OEG-OEG-γGlu-C(O)-(CH₂)₁₈-COOH), or K(-OEG-OEG-γGlu-C(O)-(CH₂)₂₀-COOH).

In some embodiments, Y₁ has the structure shown below: 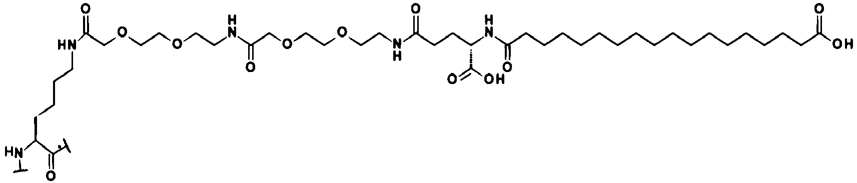 or

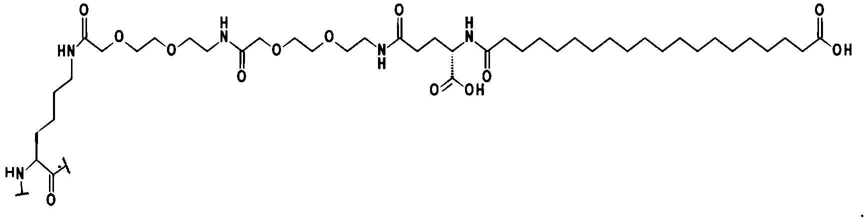

In some embodiments, R₁ is hydrogen.

In some embodiments, R₂ is -NH₂ or -OH.

In some embodiments, R₂ is -NH₂.

In some embodiments, Z is Gly-Pro-Ser-Ser-Gly-Ala-Pro-Pro-Pro-Ser (SEQ ID NO: 146).

In some embodiments, X₄₀ is absent.

In some embodiments, Y₁ is a Lys residue with the side chain linked to a substituent.

In some embodiments, the present disclosure provides a compound represented by formula (Ib) or a pharmaceutically acceptable salt thereof:

R₁-X₁-X₂-X₃-Gly-Thr-Phe-Thr-Ser-Asp-X₁₀-Ser-X₁₂-X₁₃-X₁₄-Glu-Y₁-X₁₇-X₁₈-X₁₉-X₂₀-Glu-Phe-Val-Glu-Trp-Leu-X₂₇-X₂₈-X₂₉-Gly-Pro-Ser-Ser-Gly-Ala-Pro-Pro-Pro-Ser-R₂ (SEQ ID NO: 150) (Ib);

wherein R₁ is hydrogen,
R₂ is -NH₂ or -OH;
X₁ is selected from an amino acid residue of Tyr or His;
X₂ is an amino acid residue of Aib;
X₃ is selected from an amino acid residue of Gln or His;
X₁₀ is selected from an amino acid residue of Leu or Tyr;
X₁₂ is selected from an amino acid residue of Lys, alpha-methyl-Lys, or Ile;
X₁₃ is selected from an amino acid residue of Aib, Ile, Leu, Tyr, alpha-methyl-Tyr or alpha-methyl-Leu;
X₁₄ is selected from an amino acid residue of Leu or alpha-methyl-Leu;
X₁₇ is selected from an amino acid residue of Ile, Lys, Gln, or alpha-methyl-Lys;
X₁₈ is selected from an amino acid residue of Ala or Aib;
X₁₉ is selected from an amino acid residue of Ala or Gln;
X₂₀ is selected from an amino acid residue of Gln, Aib, isovaline, alpha-methyl-Ser, 1-aminocyclopropane-1-carboxylic acid or 1-aminocyclobutane-1-carboxylic acid;
X₂₇ is selected from an amino acid residue of Ile or Leu;
X₂₈ is selected from an amino acid residue of Ala or Glu;
X₂₉ is selected from an amino acid residue of Gly or D-Glu;
Y₁ is a Lys residue with the side chain linked to a substituent having the structure shown below: -Z₁-Z₂, wherein Z₁ comprises 0-10 amino acid residues independently selected from Gly, Glu, γGlu, and OEG, and Z₂ is selected from C₁₂₋₃₂ fatty acids. In some embodiments, Z₁ comprises (or is) the structure shown below: -(OEG)ₐ-(γGlu)_{b}-; wherein a is any integer between 0 and 4, and b is any integer between 1 and 2. In some embodiments, Z₂ comprises (or is) the structure shown below: -CO-(CH₂)_{c}-COOH, wherein c is any integer between 10 and 30.

In some embodiments, the substituent has the structure shown below:
{[2-(2-amino-ethoxy)-ethoxy]-acetyl}ₐ-(γGlu)_{b}-CO-(CH₂)_{c}-COOH;
wherein a is any integer between 0 and 4, b is any integer between 1 and 2, and c is any integer between 10 and 30.

In some specific embodiments, a is 1, 2 or 3, b is 1 or 2, and c is any integer between 16 and 20 (e.g., 16, 17, 18, 19, 20). In some specific embodiments, a is 2 or 3, b is 1 or 2, and c is 16, 18 or 20. In some specific embodiments, a is 2, b is 1 or 2, and c is 16, 18 or 20. In some specific embodiments, a is 2, b is 1 or 2, and c is 16 or 18. In some specific embodiments, a is 2, b is 1, and c is 16 or 18.

In some embodiments, the substituent is covalently linked to the ε-amino group of the Lys residue via an amide bond. In some embodiments, Y₁ is K(-OEG-OEG-γGlu-C(O)-(CH₂)₁₆-COOH), K(-OEG-OEG-γGlu-C(O)-(CH₂)₁₈-COOH), or K(-OEG-OEG-γGlu-C(O)- (CH₂)₂₀-COOH).

In some embodiments, Y₁ has the structure shown below: 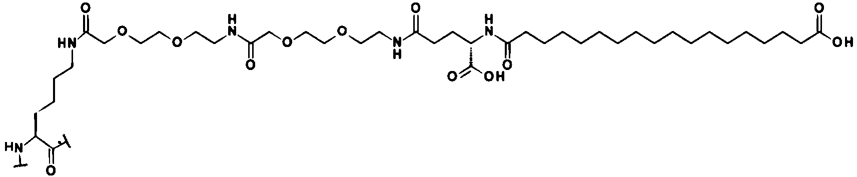 or

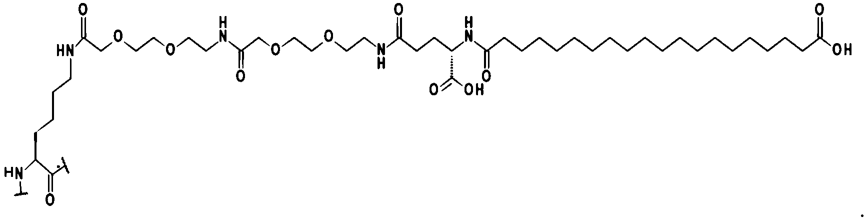

In some embodiments, the compound of the structure shown in any of the foregoing embodiments, Y₁ has the structure shown below:

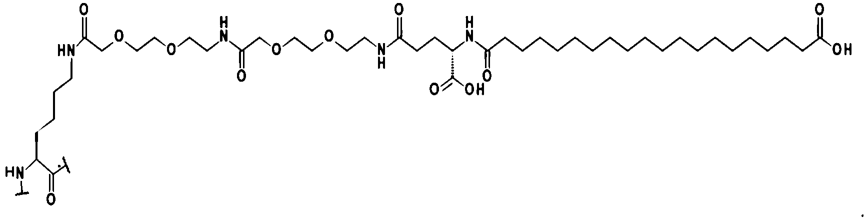

In some embodiments, the compound of the structure shown in any of the foregoing embodiments, wherein X₁, X₂ and X₃ are selected from any of the following:
X₁ is His, X₂ is Aib, and X₃ is His;
X₁ is Tyr, X₂ is Aib, and X₃ is Gln; or
X₁ is His, X₂ is Aib, and X₃ is Gln.

In some embodiments, the compound of the structure shown in any of the foregoing embodiments, X₁, X₂ and X₃ are selected from:
X₁ is His, X₂ is Aib, and X₃ is His; or
X₁ is His, X₂ is Aib, and X₃ is Gln.

In some embodiments, the compound of the structure shown in any of the foregoing embodiments, X₁, X₂ and X₃ are His, Aib and His, respectively.

In some embodiments, the compound of the structure shown in any of the foregoing embodiments, wherein X₁₇ is a Lys residue.

In some embodiments, X₁₇, X₁₈, X₁₉ and X₂₀ are selected from any of the following:
X₁₇ is Lys, X₁₈ is Ala, X₁₉ is Ala, and X₂₀ is Gln;
X₁₇ is Lys, X₁₈ is Ala, X₁₉ is Ala, and X₂₀ is alpha-methyl-Ser;
X₁₇ is Lys, X₁₈ is Ala, X₁₉ is Ala, and X₂₀ is isovaleine;
X₁₇ is Lys, X₁₈ is Ala, X₁₉ is Ala, and X₂₀ is 1-aminocyclopropane-1-carboxylic acid;
X₁₇ is Lys, X₁₈ is Ala, X₁₉ is Ala, and X₂₀ is 1-aminocyclobutane-1-carboxylic acid;
X₁₇ is Lys, X₁₈ is Ala, X₁₉ is Ala, and X₂₀ is Aib;
X₁₇ is Lys, X₁₈ is Aib, X₁₉ is Ala, and X₂₀ is Gln; and
X₁₇ is Lys, X₁₈ is Ala, X₁₉ is Gln, and X₂₀ is Gln.

In some embodiments, the compound of the structure shown in any of the foregoing embodiments, wherein X₁₇ is a Gln residue. In some embodiments, X₁₇, X₁₈, X₁₉ and X₂₀ are selected from any of the following:
X₁₇ is Gln, X₁₈ is Ala, X₁₉ is Ala, and X₂₀ is Gln;
X₁₇ is Gln, X₁₈ is Ala, X₁₉ is Ala, and X₂₀ is isovaleine;
X₁₇ is Gln, X₁₈ is Ala, X₁₉ is Ala, and X₂₀ is 1-aminocyclopropane-1-carboxylic acid; and
X₁₇ is Gln, X₁₈ is Ala, X₁₉ is Ala, and X₂₀ is 1-aminocyclobutane-1-carboxylic acid.

In some embodiments, the compound of the structure shown in any of the foregoing embodiments, wherein X₁₇ is alpha-methyl-Lys.

In some embodiments, X₁₇, X₁₈, X₁₉ and X₂₀ are alpha-methyl-Lys, Ala, Ala and Gln, respectively.

In some embodiments, the compound of the structure shown in any of the foregoing embodiments, wherein X₁₇ is Ile. In some embodiments, X₁₇, X₁₈, X₁₉ and X₂₀ are Ile, Ala, Ala and Gln, respectively.

In some embodiments, the compound of the structure shown in any of the foregoing embodiments, X₁₂ is an Ile residue.

In some embodiments, X₁₂, X₁₃ and X₁₄ are selected from any of the following:
X₁₂ is Ile, X₁₃ is Leu, and X₁₄ is Leu; or
X₁₂ is Ile, X₁₃ is alpha-methyl-Leu, and X₁₄ is Leu.

In some embodiments, the compound of the structure shown in any of the foregoing embodiments, wherein X₁₂ is a Lys residue.

In some embodiments, X₁₂, X₁₃ and X₁₄ are selected from any of the following:
X₁₂ is Lys, X₁₃ is alpha-methyl-Tyr, and X₁₄ is Leu;
X₁₂ is Lys, X₁₃ is Aib, and X₁₄ is Leu;
X₁₂ is Lys, X₁₃ is Tyr, and X₁₄ is alpha-methyl-Leu; and
X₁₂ is Lys, X₁₃ is Tyr, and X₁₄ is Leu.

In some embodiments, the compound of the structure shown in any of the foregoing embodiments, wherein X₁₂ is an alpha-methyl-Lys residue.

In some embodiments, X₁₂, X₁₃ and X₁₄ are selected from any of the following:
X₁₂ is alpha-methyl-Lys, X₁₃ is Tyr, and X₁₄ is Leu;
X₁₂ is alpha-methyl-Lys, X₁₃ is Leu, and X₁₄ is Leu; or
X₁₂ is alpha-methyl-Lys, X₁₃ is Ile, and X₁₄ is Leu.

In some embodiments, the compound of the structure shown in any of the foregoing embodiments, wherein X₁ to X₂₉ are selected from the amino acid combination shown in any of (i) to (xi) above.

In some embodiments, Z is selected from Gly-Pro-Ser-Ser-Gly-Ala-Pro-Pro-Pro-Ser (SEQ ID NO: 146), Gly-Pro-Ser-Ser-Gly-Ala-Pro-Pro-Ser (SEQ ID NO: 147), Pro-Ser-Ser-Gly-Ala-Pro-Pro-Pro-Ser (SEQ ID NO: 148), or Pro-Ser-Ser-Gly-Ala-Pro-Pro-Ser (SEQ ID NO: 149). In some embodiments, Z is Gly-Pro-Ser-Ser-Gly-Ala-Pro-Pro-Pro-Ser (SEQ ID NO: 146). In some embodiments, X₄₀ is selected from an amino acid residue of Lys or is absent. In some embodiments, X₄₀ is absent. In some embodiments, R₁ is hydrogen, alkyl, acetyl, formyl, benzoyl, trifluoroacetyl, or pGlu, and R₂ is -NH₂ or -OH. In some embodiments, R₁ is hydrogen, and R₂ is -NH₂ or -OH. In some embodiments, Y₁ is a Lys, Orn, Dap, Dab, or Cys residue with the side chain linked to a substituent having the structure shown below:
{[2-(2-amino-ethoxy)-ethoxy]-acetyl}ₐ-(γGlu)_{b}-CO-(CH₂)_{c}-COOH;
wherein a is any integer between 0 and 4, b is any integer between 1 and 2, and c is any integer between 10 and 30. In some specific embodiments, a is 1, 2 or 3, b is 1 or 2, and c is any integer between 16 and 20 (e.g., 16, 17, 18, 19, 20). In some specific embodiments, a is 2 or 3, b is 1 or 2, and c is 16, 18 or 20. In some specific embodiments, a is 2, b is 1 or 2, and c is 16, 18 or 20. In some specific embodiments, a is 2, b is 1 or 2, and c is 16 or 18. In some specific embodiments, a is 2, b is 1, and c is 16 or 18.

In some embodiments, the substituent is covalently linked to the ε-amino group of the Lys residue via an amide bond. In some embodiments, Y₁ is K(-OEG-OEG-γGlu-C(O)-(CH₂)₁₆-COOH) or K(-OEG-OEG-γGlu-C(O)-(CH₂)₁₈-COOH), the Y₁ having the structure shown below: 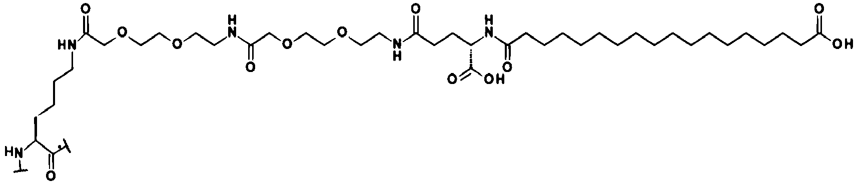 or

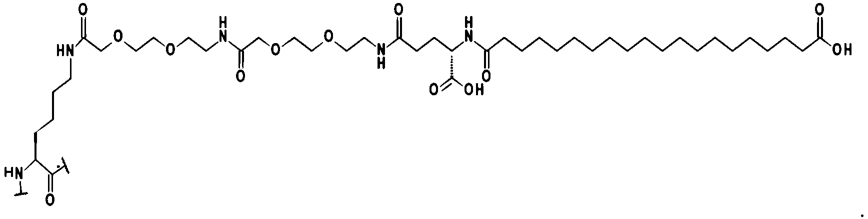

In some embodiments, the compound comprises (or is) the structure shown in any of the following:

**Table 2. Examples of sequence structures of compounds**

| Sequence | SEQ ID NO: |
|---|---|
| H-Aib-HGTFTSDYS-αK-YLEEY₁AAQEFVEWLLAGGPSSGAPPPS-NH₂ | 49 |
| H-Aib-HGTFTSDYS-αK-YLEY₁KAAQEFVEWLLAGGPSSGAPPPS-NH₂ | 50 |
| H-Aib-HGTFTSDYSK-αY-LEY₁KAAQEFVEWLLAGGPSSGAPPPS-NH₂ | 51 |
| H-Aib-HGTFTSDYSK-Aib-LEY₁KAAQEFVEWLLAGGPSSGAPPPS-NH₂ | 52 |
| H-Aib-HGTFTSDYSKY-αL-EY₁KAAQEFVEWLLAGGPSSGAPPPS-NH₂ | 53 |
| H-Aib-HGTFTSDYSKYLEY₁-αK-AAQEFVEWLLAGGPSSGAPPPS-NH₂ | 54 |
| H-Aib-HGTFTSDYSKYLEY₁K-Aib-AQEFVEWLLAGGPSSGAPPPS-NH₂ | 55 |
| H-Aib-HGTFTSDYS-αK-YLEY₁KAAQEFVEWLIAGGPSSGAPPPS-NH₂ | 56 |
| H-Aib-HGTFTSDYS-αK-LLEY₁KAAQEFVEWLLAGGPSSGAPPPS-NH₂ | 57 |
| H-Aib-HGTFTSDYS-αK-YLEY₁KAQQEFVEWLLAGGPSSGAPPPS-NH₂ | 58 |
| Y-Aib-QGTFTSDYS-αK-LLEY₁KAAQEFVEWLLAGGPSSGAPPPS-NH₂ | 59 |
| H-Aib-HGTFTSDYS-αK-YLEY₁KAAQEFVEWLIEGGPSSGAPPPS-NH₂ | 60 |
| H-Aib-HGTFTSDYS-αK-LLEY₁QAAQEFVEWLLAGGPSSGAPPPS-NH₂ | 61 |
| H-Aib-HGTFTSDYS-αK-LLEY₁IAAQEFVEWLLAGGPSSGAPPPS-NH₂ | 62 |
| H-Aib-HGTFTSDYS-αK-ILEY₁KAAQEFVEWLLAGGPSSGAPPPS-NH₂ | 63 |
| H-Aib-HGTFTSDYSILLEY₁KAAQEFVEWLLAGGPSSGAPPPS-NH₂ | 64 |
| H-Aib-HGTFTSDYSI-αL-LEY₁KAAQEFVEWLLAGGPSSGAPPPS-NH₂ | 65 |
| H-Aib-HGTFTSDYS-αK-LLEY₁KAA-αS-EFVEWLIEGGPSSGAPPPS-NH₂ | 66 |
| Y-Aib-QGTFTSDYS-αK-LLEY₁KAA-Iva-EFVEWLLAGGPSSGAPPPS-NH₂ | 67 |
| H-Aib-HGTFTSDYS-αK-LLEY₁QAA-Iva-EFVEWLLAGGPSSGAPPPS-NH₂ | 68 |
| Y-Aib-QGTFTSDYS-αK-ILEY₁KAA-Iva-EFVEWLLAGGPSSGAPPPS-NH₂ | 69 |
| H-Aib-HGTFTSDYS-αK-ILEY₁QAA-Iva-EFVEWLLAGGPSSGAPPPS-NH₂ | 70 |
| H-Aib-HGTFTSDYS-αK-LLEY₁KAA-Iva-EFVEWLIEGGPSSGAPPPS-NH₂ | 71 |
| H-Aib-HGTFTSDYS-αK-ILEY₁KAA-Iva-EFVEWLIEGGPSSGAPPPS-NH₂ | 72 |
| H-Aib-HGTFTSDLS-αK-LLEY₁KAA-Iva-EFVEWLIEGGPSSGAPPPS-NH₂ | 73 |
| H-Aib-HGTFTSDLS-αK-ILEY₁KAA-Iva-EFVEWLIEGGPSSGAPPPS-NH₂ | 74 |
| H-Aib-HGTFTSDYSILLEY₁KAA-Iva-EFVEWLLAGGPSSGAPPPS-NH₂ | 75 |
| H-Aib-HGTFTSDLSILLEY₁KAA-Iva-EFVEWLLAGGPSSGAPPPS-NH₂ | 76 |
| H-Aib-HGTFTSDYSI-αL-LEY₁KAA-Iva-EFVEWLLAGGPSSGAPPPS-NH₂ | 77 |
| H-Aib-HGTFTSDLSI-αL-LEY₁KAA-Iva-EFVEWLLAGGPSSGAPPPS-NH₂ | 78 |
| H-Aib-HGTFTSDYSI-αL-LEY₁KAAQEFVEWLLAeGPSSGAPPPS-NH₂ | 79 |
| H-Aib-HGTFTSDLSI-αL-LEY₁KAA-Ac4c-EFVEWLLAGGPSSGAPPPS-NH₂ | 80 |
| H-Aib-HGTFTSDLSI-αL-LEY₁KAAQEFVEWLLAeGPSSGAPPPS-NH₂ | 81 |
| H-Aib-HGTFTSDLSI-αL-LEY₁KAAQEFVEWLLAGGPSSGAPPPS-NH₂ | 82 |
| H-Aib-HGTFTSDLSILLEY₁KAAQEFVEWLLAeGPSSGAPPPS-NH₂ | 83 |
| H-Aib-HGTFTSDLSILLEY₁QAA-Ac4c-EFVEWLLAeGPSSGAPPPS-NH₂ | 84 |
| H-Aib-HGTFTSDLSILLEY₁KAA-Ac3c-EFVEWLLAeGPSSGAPPPS-NH₂ | 85 |
| H-Aib-HGTFTSDLSILLEY₁KAA-Ac4c-EFVEWLLAeGPSSGAPPPS-NH₂ | 86 |
| H-Aib-HGTFTSDLSILLEY₁QAA-Ac3c-EFVEWLLAeGPSSGAPPPS-NH₂ | 87 |
| H-Aib-HGTFTSDYSILLEY₁QAA-Ac3c-EFVEWLLAeGPSSGAPPPS-NH₂ | 88 |
| H-Aib-HGTFTSDYSILLEY₁QAA-Ac4c-EFVEWLLAeGPSSGAPPPS-NH₂ | 89 |
| H-Aib-HGTFTSDYSILLE₁KAAQEFVEWLLAeGPSSGAPPPS-NH₂ | 90 |
| H-Aib-HGTFTSDYSILLEY₁QAAQEFVEWLLAGGPSSGAPPPS-NH₂ | 91 |
| H-Aib-HGTFTSDYSILLEY₁QAAQEFVEWLLAeGPSSGAPPPS-NH₂ | 92 |
| H-Aib-QGTFTSDYSILLEY₁KAAQEFVEWLLAGGPSSGAPPPS-NH₂ | 93 |
| H-Aib-QGTFTSDYSI-αL-LEY₁KAAQEFVEWLLAGGPSSGAPPPS-NH₂ | 94 |
| H-Aib-QGTFTSDYSILLEY₁KAA-Aib-EFVEWLLAGGPSSGAPPPS-NH₂ | 95 |
| H-Aib-QGTFTSDYSI-αL-LEY₁KAA-Aib-EFVEWLLAGGPSSGAPPPS-NH₂; | 96 |

In Table 2, αK is alpha-methyl-Lys, αY is alpha-methyl-Tyr, αL is alpha-methyl-Leu, Iva is isovaleine, e is D-Glu, Aib is α-aminoisobutyric acid, Ac3c is 1-aminocyclopropane-1-carboxylic acid, and Ac4c is 1-aminocyclobutane-1-carboxylic acid;
Y₁ is a Lys, Orn, Dap, Dab, or Cys residue with the side chain linked to a substituent having the structure shown below: -Z₁-Z₂, wherein Z₁ comprises 0-10 amino acid residues independently selected from Gly, Glu, yGlu, and OEG, and Z₂ is selected from C₁₂₋₃₂ fatty acids. In some embodiments, Z₁ comprises (or is) the structure shown below: - (OEG)ₐ-(γGlu)_{b}-; wherein a is any integer between 0 and 4, and b is any integer between 1 and 2. In some embodiments, Z₂ comprises (or is) the structure shown below: -CO-(CH₂)_{c}-COOH, wherein c is any integer between 10 and 30.

In some embodiments, the substituent has the structure shown below:
{[2-(2-amino-ethoxy)-ethoxy]-acetyl}ₐ-(γGlu)_{b}-CO-(CH₂)_{c}-COOH;
wherein a is any integer between 0 and 4, b is any integer between 1 and 2, and c is any integer between 10 and 30. In some specific embodiments, a is 1, 2 or 3, b is 1 or 2, and c is any integer between 16 and 20 (e.g., 16, 17, 18, 19, 20). In some specific embodiments, a is 2 or 3, b is 1 or 2, and c is 16, 18 or 20. In some specific embodiments, a is 2, b is 1 or 2, and c is 16, 18 or 20. In some specific embodiments, a is 2, b is 1 or 2, and c is 16 or 18. In some specific embodiments, a is 2, b is 1, and c is 16 or 18.

In some embodiments, the substituent is covalently linked to the ε-amino group of the Lys residue via an amide bond. In some embodiments, Y₁ is K(-OEG-OEG-γGlu-C(O)-(CH₂)₁₆-COOH), K(-OEG-OEG-γGlu-C(O)-(CH₂)₁₈-COOH), or K(-OEG-OEG-γGlu-C(O)- (CH₂)₂₀-COOH).

In some embodiments, Y₁ has the structure shown below: 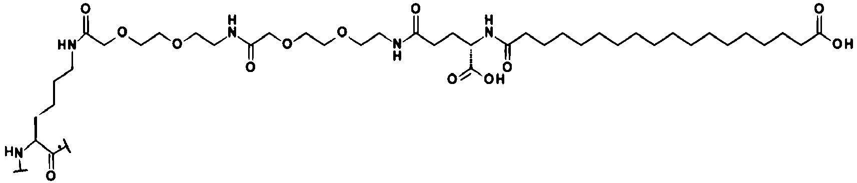 or

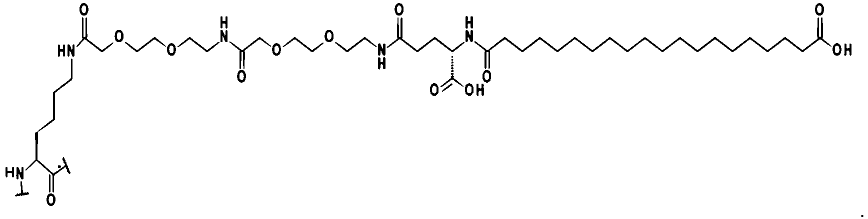

In some more specific embodiments, the structure of Y₁ is:

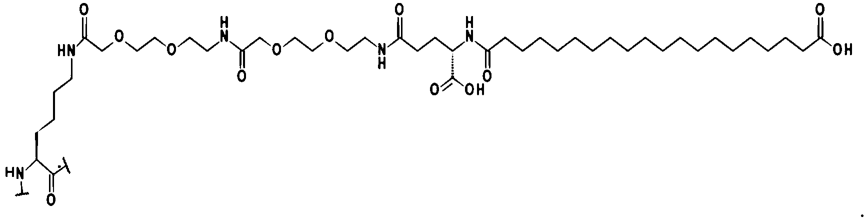

As mentioned earlier, αK, also known as alpha-methyl-Lys, refers to Lys substituted with α-methyl. αY, also known as alpha-methyl-Tyr, refers to Tyr substituted with α-methyl. αL, also known as alpha-methyl-Leu, refers to Leu substituted with α-methyl. Iva is isovaline. e is D-Glu, which is a type D Glu. Aib is α-aminoisobutyric acid. Ac3c is 1-aminocyclopropane-1-carboxylic acid, and Ac4c is 1-aminocyclobutane-1-carboxylic acid.

In some embodiments, the compound comprises (or is) any of the following structures:

**Table 3**

| No. | Sequence Structure | | SEQ ID NO: |
|---|---|---|---|
| 0100 | H-Aib-HGTFTSDYS-αK-YLEE-K(-OEG-OEG-γGlu-C(O)-(CH₂)₁₈-COOH)-AAQEFVEWLLAGGPSSGAPPPS-NH₂ | Compound 1 | 97 |
| 0104 | H-Aib-HGTFTSDYS-αK-YLE-K(-OEG-OEG-γGlu-C(O)-(CH₂)₁₈-COOH)-KAAQEFVEWLLAGGPSSGAPPPS-NH₂ | Compound 2 | 98 |
| 0106 | H-Aib-HGTFTSDYSK-αY-LE-K(-OEG-OEG-γGlu-C(O)-(CH₂)₁₅-COOH)-KAAQEFVEWLLAGGPSSGAPPPS-NH₂ | Compound 3 | 99 |
| 0107 | H-Aib-HGTFTSDYSK-Aib-LE-K(-OEG-OEG-γGlu-C(O)-(CH₂)₁₈-COOH)-KAAQEFVEWLLAGGPSSGAPPPS-NH₂ | Compound 4 | 100 |
| 0108 | H-Aib-HGTFTSDYSKY-αL-E-K(-OEG-OEG-γGlu-C(O)-(CH₂)₁₈-COOH)-KAAQEFVEWLLAGGPSSGAPPPS-NH₂ | Compound 5 | 101 |
| 0109 | H-Aib-HGTFTSDYSKYLE-K(-OEG-OEG-γGlu-C(O)-(CH₂)₁₈-COOH)-αK-AAQEFVEWLLAGGPSSGAPPPS-NH₂ | Compound 6 | 102 |
| 0110 | H-Aib-HGTFTSDYSKYLE-K(-OEG-OEG-γGlu-C(O)-(CH₂)₁₈-COOH)-K-Aib-AQEFVEWLLAGGPSSGAPPPS-NH₂ | Compound 7 | 103 |
| 0111 | H-Aib-HGTFTSDYS-αK-YLE-K(-OEG-OEG-γGlu-C(O)-(CH₂)₁₈-COOH)-KAAQEFVEWLIAGGPSSGAPPPS-NH₂ | Compound 8 | 104 |
| 0112 | H-Aib-HGTFTSDYS-αK-LLE-K(-OEG-OEG-γGlu-C(O)-(CH₂)₁₈-COOH)-KAAQEFVEWLLAGGPSSGAPPPS-NH₂ | Compound 9 | 105 |
| 0113 | H-Aib-HGTFTSDYS-αK-YLE-K(-OEG-OEG-γGlu-C(O)-(CH₂)₁₈-COOH)-KAQQEFVEWLLAGGPSSGAPPPS-NH₂ | Compound 10 | 106 |
| 0114 | Y-Aib-QGTFTSDYS-αK-LLE-K(-OEG-OEG-γGlu-C(O)-(CH₂)₁₈-COOH)-KAAQEFVEWLLAGGPSSGAPPPS-NH₂ | Compound 11 | 107 |
| 0115 | H-Aib-HGTFTSDYS-αK-YLE-K(-OEG-OEG-γGlu-C(O)-(CH₂)₁₈-COOH)-KAAQEFVEWLIEGGPSSGAPPPS-NH₂ | Compound 12 | 108 |
| 0117 | H-Aib-HGTFTSDYS-αK-LLE-K(-OEG-OEG-γGlu-C(O)-(CH₂)₁₈-COOH)-QAAQEFVEWLLAGGPSSGAPPPS-NH₂ | Compound 13 | 109 |
| 0118 | H-Aib-HGTFTSDYS-αK-LLE-K(-OEG-OEG-γGlu-C(O)-(CH₂)₁₅-COOH)-IAAQEFVEWLLAGGPSSGAPPPS-NH₂ | Compound 14 | 110 |
| 0119 | H-Aib-HGTFTSDYS-αK-ILE-K(-OEG-OEG-γGlu-C(O)-(CH₂)₁₈-COOH)-KAAQEFVEWLLAGGPSSGAPPPS-NH₂ | Compound 15 | 111 |
| 0120 | H-Aib-HGTFTSDYSILLE-K(-OEG-OEG-γGlu-C(O)-(CH₂)₁₈-COOH)-KAAQEFVEWLLAGGPSSGAPPPS-NH₂ | Compound 16 | 112 |
| 0125 | H-Aib-HGTFTSDYSI-αL-LE-K(-OEG-OEG-γGlu-C(O)-(CH₂)₁₈-COOH)-KAAQEFVEWLLAGGPSSGAPPPS-NH₂ | Compound 17 | 113 |
| 0126 | H-Aib-HGTFTSDYS-αK-LLE-K(-OEG-OEG-γGlu-C(O)-(CH₂)₁₈-COOH)-KAA-αS-EFVEWLIEGGPSSGAPPPS-NH₂ | Compound 18 | 114 |
| 0127 | Y-Aib-QGTFTSDYS-αK-LLE-K(-OEG-OEG-γGlu-C(O)-(CH₂)₁₈-COOH)-KAA-Iva-EFVEWLLAGGPSSGAPPPS-NH₂ | Compound 19 | 115 |
| 0128 | H-Aib-HGTFTSDYS-αK-LLE-K(-OEG-OEG-γGlu-C(O)-(CH₂)₁₈-COOH)-QAA-Iva-EFVEWLLAGGPSSGAPPPS-NH₂ | Compound 20 | 116 |
| 0129 | Y-Aib-QGTFTSDYS-αK-ILE-K(-OEG-OEG-γGlu-C(O)-(CH₂)₁₈-COOH)-KAA-Iva-EFVEWLLAGGPSSGAPPPS-NH₂ | Compound 21 | 117 |
| 0130 | H-Aib-HGTFTSDYS-αK-ILE-K(-OEG-OEG-γGlu-C(O)-(CH₂)₁₈-COOH)-QAA-Iva-EFVEWLLAGGPSSGAPPPS-NH₂ | Compound 22 | 118 |
| 0131 | H-Aib-HGTFTSDYS-αK-LLE-K(-OEG-OEG-γGlu-C(O)-(CH₂)₁₈-COOH)-KAA-Iva-EFVEWLIEGGPSSGAPPPS-NH₂ | Compound 23 | 119 |
| 0132 | H-Aib-HGTFTSDYS-αK-ILE-K(-OEG-OEG-γGlu-C(O)-(CH₂)₁₈-COOH)-KAA-Iva-EFVEWLIEGGPSSGAPPPS-NH₂ | Compound 24 | 120 |
| 0133 | H-Aib-HGTFTSDLS-αK-LLE-K(-OEG-OEG-γGlu-C(O)-(CH₂)₁₈-COOH)-KAA-Iva-EFVEWLIEGGPSSGAPPPS-NH₂ | Compound 25 | 121 |
| 0134 | H-Aib-HGTFTSDLS-αK-ILE-K(-OEG-OEG-γGlu-C(O)-(CH₂)₁₈-COOH)-KAA-Iva-EFVEWLIEGGPSSGAPPPS-NH₂ | Compound 26 | 122 |
| 0135 | H-Aib-HGTFTSDYSILLE-K(-OEG-OEG-γGlu-C(O)-(CH₂)₁₈-COOH)-KAA-Iva-EFVEWLLAGGPSSGAPPPS-NH₂ | Compound 27 | 123 |
| 0136 | H-Aib-HGTFTSDLSILLE-K(-OEG-OEG-γGlu-C(O)-(CH₂)₁₈-COOH)-KAA-Iva-EFVEWLLAGGPSSGAPPPS-NH₂ | Compound 28 | 124 |
| 0137 | H-Aib-HGTFTSDYSI-αL-LE-K(-OEG-OEG-γGlu-C(O)-(CH₂)₁₈-COOH)-KAA-Iva-EFVEWLLAGGPSSGAPPPS-NH₂ | Compound 29 | 125 |
| 0138 | H-Aib-HGTFTSDLSI-αL-LE-K(-OEG-OEG-γGlu-C(O)-(CH₂)₁₈-COOH)-KAA-Iva-EFVEWLLAGGPSSGAPPPS-NH₂ | Compound 30 | 126 |
| 0139 | H-Aib-HGTFTSDYSI-αL-LE-K(-OEG-OEG-γGlu-C(O)-(CH₂)₁₈-COOH)-KAAQEFVEWLLA-e-GPSSGAPPPS-NH₂ | Compound 31 | 127 |
| 0140 | H-Aib-HGTFTSDLSI-αL-LE-K(-OEG-OEG-γGlu-C(O)-(CH₂)₁₈-COOH)-KAA-Ac4c-EFVEWLLAGGPSSGAPPPS-NH₂ | Compound 32 | 128 |
| 0142 | H-Aib-HGTFTSDLSI-αL-LE-K(-OEG-OEG-γGlu-C(O)-(CH₂)₁₈-COOH)-KAAQEFVEWLLA-e-GPSSGAPPPS-NH₂ | Compound 33 | 129 |
| 0143 | H-Aib-HGTFTSDLSI-αL-LE-K(-OEG-OEG-γGlu-C(O)-(CH₂)₁₈-COOH)-KAAQEFVEWLLAGGPSSGAPPPS-NH₂ | Compound 34 | 130 |
| 0144 | H-Aib-HGTFTSDLSILLE-K(-OEG-OEG-γGlu-C(O)-(CH₂)₁₈-COOH)-KAAQEFVEWLLA-e-GPSSGAPPPS-NH₂ | Compound 35 | 131 |
| 0145 | H-Aib-HGTFTSDLSILLE-K(-OEG-OEG-γGlu-C(O)-(CH₂)₁₈-COOH)-QAA-Ac4c-EFVEWLLA-e-GPSSGAPPPS-NH₂ | Compound 36 | 132 |
| 0146 | H-Aib-HGTFTSDLSILLE-K(-OEG-OEG-γGlu-C(O)-(CH₂)₁₈-COOH)-KAA-Ac3c-EFVEWLLA-e-GPSSGAPPPS-NH₂ | Compound 37 | 133 |
| 0147 | H-Aib-HGTFTSDLSILLE-K(-OEG-OEG-_{γ}Glu-C(O)-(CH₂)₁₈-COOH)-KAA-Ac4c-EFVEWLLA-e-GPSSGAPPPS-NH₂ | Compound 38 | 134 |
| 0148 | H-Aib-HGTFTSDLSILLE-K(-OEG-OEG-γGlu-C(O)-(CH₂)₁₈-COOH)-QAA-Ac3c-EFVEWLLA-e-GPSSGAPPPS-NH₂ | Compound 39 | 135 |
| 0149 | H-Aib-HGTFTSDYSILLE-K(-OEG-OEG-γGlu-C(O)-(CH₂)₁₈-COOH)-QAA-Ac3c-EFVEWLLA-e-GPSSGAPPPS-NH₂ | Compound 40 | 136 |
| 0150 | H-Aib-HGTFTSDYSILLE-K(-OEG-OEG-yGlu-C(O)-(CH₂)₁₈-COOH)-QAA-Ac4c-EFVEWLLA-e-GPSSGAPPPS-NH₂ | Compound 41 | 137 |
| 0151 | H-Aib-HGTFTSDYSILLE-K(-OEG-OEG-γGlu-C(O)-(CH₂)₁₈-COOH)-KAAQEFVEWLLA-e-GPSSGAPPPS-NH₂ | Compound 42 | 138 |
| 0152 | H-Aib-HGTFTSDYSILLE-K(-OEG-OEG-γGlu-C(O)-(CH₂)₁₈-COOH)-QAAQEFVEWLLAGGPSSGAPPPS-NH₂ | Compound 43 | 139 |
| 0153 | H-Aib-HGTFTSDYSILLE-K(-OEG-OEG-γGlu-C(O)-(CH₂)₁₈-COOH)-QAAQEFVEWLLA-e-GPSSGAPPPS-NH₂ | Compound 44 | 140 |
| 0154 | H-Aib-QGTFTSDYSILLE-K(-OEG-OEG-γGlu-C(O)-(CH₂)₁₈-COOH)-KAAQEFVEWLLAGGPSSGAPPPS-NH₂ | Compound 45 | 141 |
| 0155 | H-Aib-QGTFTSDYSI-αL-LE-K(-OEG-OEG-γGlu-C(O)-(CH₂)₁₈-COOH)-KAAQEFVEWLLAGGPSSGAPPPS-NH₂ | Compound 46 | 142 |
| 0156 | H-Aib-QGTFTSDYSILLE-K(-OEG-OEG-γGlu-C(O)-(CH₂)₁₈-COOH)-KAA-Aib-EFVEWLLAGGPSSGAPPPS-NH₂ | Compound 47 | 143 |
| 0157 | H-Aib-QGTFTSDYSI-αL-LE-K(-OEG-OEG-γGlu-C(O)-(CH₂)₁₈-COOH)-KAA-Aib-EFVEWLLAGGPSSGAPPPS-NH_{2∘} | Compound 48 | 144 |

In Table 3, αK is alpha-methyl-Lys, αY is alpha-methyl-Tyr, αL is alpha-methyl-Leu, Iva is isovaleine, e is D-Glu, Aib is α-aminoisobutyric acid, Ac3c is 1-aminocyclopropane-1-carboxylic acid, Ac4c is 1-aminocyclobutane-1-carboxylic acid, and OEG is [2-(2-amino-ethoxy)-ethoxy]-acetyl.

Exemplary structural formulas are as follows:

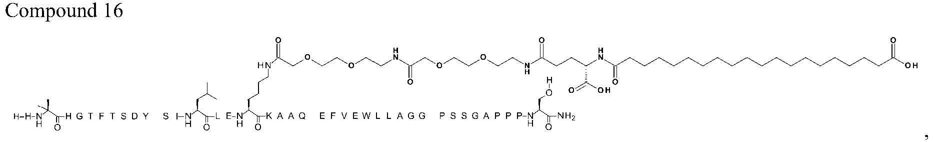

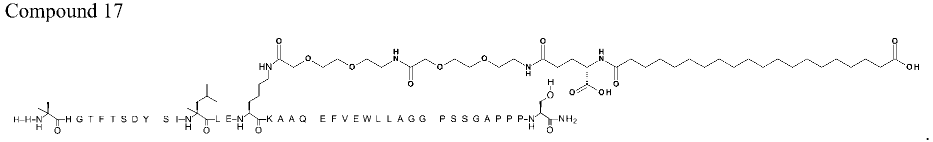

In some embodiments, the present disclosure provides pharmaceutically acceptable salts or solvates of any of the foregoing compounds. In some embodiments, the compound with any of the structures in the present disclosure is an amphoteric compound, and those skilled in the art can use an acidic or basic compound to react with it to form a salt through well-known techniques, or it can form a solvate through techniques known in the art. For example, a solvate is a stoichiometric complex formed between a compound or a pharmaceutically acceptable salt and a solvent (including but not limited to water).

In some embodiments, the present disclosure provides a compound that comprises (or is) the polypeptide sequence of any of the foregoing compounds.

In some embodiments, the compound with any of the structures in the present disclosure has at least the following agonistic activity against at least one of the following receptors: GLP-1 receptor, GIP receptor, and GCG receptor.

In some embodiments, the compound of any of the structures in the present disclosure has an agonist activity against GLP-1 receptor (GLP-1-R). In some embodiments, the compound with any of the foregoing structures has an agonist activity against GIP receptor (GIP-R). In some embodiments, the compound with any of the foregoing structures has an agonist activity against GCG receptor (GCG-R).

In some embodiments, the compound with any of the structures in the present disclosure has an agonistic activity against GLP-1 receptor, GIP receptor, and GCG receptor. In some embodiments, any of Compound 1 to Compound 48 in the present disclosure has an agonistic activity against GLP-1 receptor, GIP receptor and GCG receptor.

In some embodiments, the compound with any of the structures in the present disclosure can activate GLP-1 receptor, GIP receptor and/or GCG receptor. In some embodiments, any of Compound 1 to Compound 48 in the present disclosure can activate GLP-1 receptor, GIP receptor and/or GCG receptor.

In some embodiments, the compound with any of the structures in the present disclosure can activate GLP-1 receptor, GIP receptor and GCG receptor. In some embodiments, any of Compound 1 to Compound 48 in the present disclosure can activate GLP-1 receptor, GIP receptor and GCG receptor.

As used herein and with respect to one or more of GIP, GLP-1, or GCG receptors, "activity," "activation," "activation," and the like refer to, as measured when using assays known in the art, for example, the assay methods provided in the examples of the present disclosure.

In some embodiments, the activity in an assay in vitro can also be used as a measure of the activity of a compound. The EC₅₀ value can be used as a numerical measure of the potency of an agonist against a given receptor. The EC₅₀ value is a measured value of the concentration of a compound required to achieve half of its maximum activity in a specific assay. In some embodiments of the present disclosure, for example, when evaluated using the assay methods described in the test examples, EC₅₀ GLP-1-R and/or EC₅₀ GIP-R and/or EC₅₀ GCG-R are less than 10.0nM, less than 8.0nM, less than 5.0nM, less than 3.0nM, less than 1.0nM, less than 0.9nM, less than 0.8nM, less than 0.7nM, less than 0.6nM, less than 0.5nM, less than 0.4nM, less than 0.3nM, less than 0.2nM, less than 0.1nM, less than 0.09nM, less than 0.08nM, less than 0.07nM, less than 0.06nM, less than 0.05nM, less than 0.04nM, less than 0.03nM, less than 0.02nM, less than 0.01nM, less than 0.009nM, less than 0.008nM, less than 0.007nM, less than 0.006nM, less than 0.005nM, less than 0.004nM, less than 0.003nM, or less than 0.002nM.

In some embodiments, the compound with any of the structures in the present disclosure has an enhanced weight reduction effect. For example, any of Compound 1 to Compound 48 in the present disclosure has a better weight reduction effect in DIO mice, DIO rats and/or db/db mice. In some embodiments, the compound with any of the structures in the present disclosure has a greater weight reduction effect than the LY3437943 molecule.

In some embodiments, the compounds in the present disclosure have good safety. For example, the compounds in the present disclosure (e.g., Compound 16 or Compound 17) reduce the body weight of DIO mice to 80% or more compared to that of normal-diet (chow) mice after administered to DIO mice. In some embodiments, the compounds in the present disclosure have an increased dosing window. For example, they have an increased dosing window compared to LY3437943. In some embodiments, the compounds in the present disclosure have an increased maximum safe dose. For example, the maximum safe dose of the compounds in the present disclosure is at least 1, at least 2, at least 3, at least 4, at least 5 times that of LY3437943.

In some embodiments, the compounds in the present disclosure reduce liver weight, perirenal fat weight, inguinal fat, and/or epididymal fat. For example, the compounds in the present disclosure, represented by Compound 1 to Compound 48, reduce liver weight, perirenal fat weight, inguinal fat, and/or epididymal fat in the DIO mouse model shown in Example 4 or the DIO rat model shown in Example 5.

In some embodiments, the compounds in the present disclosure reduce alanine aminotransferase (ALT), high-density lipoprotein cholesterol (HDL-c), low-density lipoprotein cholesterol (LDL-c), serum triglycerides (TG), and/or serum total cholesterol (TC) in subjects. For example, the compounds in the present disclosure, represented by Compound 1 to Compound 48, reduce ALT, HDL, LDL, serum TG and/or serum TC in the DIO mouse model shown in Example 4 or the DIO rat model shown in Example 5.

In some embodiments, the compound with any of the structures in the present disclosure has improved solubility. In some embodiments, any of Compound 1 to Compound 48 in the present disclosure has improved solubility. In some embodiments, Compounds 17 and 18 in the present disclosure have a solubility in the vehicle of ≥20 mg/mL.

In some embodiments, the compound with any of the structures in the present disclosure has improved chemical and/or physical stability. In some embodiments, any of Compound 1 to Compound 48 in the present disclosure has improved chemical and/or physical stability. In some embodiments, after Compounds 17 and 18 in the present disclosure are dissolved in the vehicle, the solution remains clear and transparent after incubation at 25 degrees Celsius for 28 days.

In some embodiments, the compound with any of the structures in the present disclosure has improved pharmacokinetic properties. In some embodiments, any of Compound 1 to Compound 48 in the present disclosure has improved pharmacokinetic properties.

The pharmacokinetic parameters of the compounds in the present disclosure can be determined using assays known in the art. For example, area under the curve (AUC), clearance rate (CL), half-life (T_{1/2}), and the like. In some embodiments, the pharmacokinetic properties of the compounds are determined using the methods provided in the examples of the present disclosure.

In some embodiments, the compound with any of the structures in the present disclosure has extended half-life. For example, compared to native GLP-1, GIP, or GCG. In some embodiments, any of Compound 1 to Compound 48 in the present disclosure has extended half-life. By way of example, the half-lives of the compounds in the present disclosure are ≥30 min, 40 min, 45 min, 50 min, 1 h, 1.5 h, 1.8 h, 2 h, 2.2 h, 2.5 h, 2.8 h, 3.0 h, 3.2 h, 3.5 h, 3.8 h, 4.0 h, 4.2 h, 4.5 h, 4.8 h, 5.0 h, 5.2 h, 5.5 h, 5.8 h, 6.0 h, 6.2 h, 6.5 h, 6.8 h, 7.0 h, 7.2 h, 7.5 h, 7.8 h, 8.0 h, 8.2 h, 8.5 h, 8.8 h, 9.0 h, 9.2 h, 9.5 h, 9.8 h, 10.0 h, 10.2 h, 10.5 h, 10.8 h, 11.0 h, 11.2 h, 11.5 h, 11.8 h, 12.0 h, 12.2 h, 12.5 h, 12.8 h, 13.0 h, 13.2 h, 13.5 h, 13.8 h, 14.0 h, 14.2 h, 14.5 h, 14.8 h, 15.0 h, 15.2 h, 15.5 h, 15.8 h, 16.0 h, 16.5 h, 16.0 h, 17.0 h, 17.5 h, 18.0 h, 18.5 h, 19.0 h, 19.5 h, 20.0 h, 25 h, 30 h, 40 h, 50 h, 60 h, 70 h, 80 h, 90 h, 100 h, 120 h, 150 h, 200 h, etc. (including any range between any of the above point values).

In some embodiments, the compound provided in the present disclosure is a derivative of a GLP-1 analog that has agonist activity against human GIP and GCG receptors, and has a triple agonist effect against human GLP-1 receptor, human GIP receptor and human GCG receptor. In some embodiments, some of the compounds of the present disclosure have stronger efficacy in reducing body weight than other triagonist products in the art. In some embodiments, some of the compounds of the present disclosure have extremely high plasma stability.

### GLP-1/GIP/GCG receptor triagonist

In some embodiments, the present disclosure provides a GLP-1/GIP/GCG receptor triagonist comprising a compound with any of the foregoing structures or a pharmaceutically acceptable salt thereof.

In some embodiments, the GLP-1/GIP/GCG receptor triagonist has an agonistic activity against GLP-1 receptor, GIP receptor, and GCG receptor.

In some embodiments, the GLP-1/GIP/GCG receptor triagonist can activate GLP-1 receptor, GIP receptor and/or GCG receptor.

In some embodiments, the GLP-1/GIP/GCG receptor triagonist can activate GLP-1 receptor, GIP receptor and GCG receptor.

In some embodiments, the activity in an assay in vitro can also be used as a measure of the activity of a compound. The EC₅₀ value can be used as a numerical measure of the potency of an agonist against a given receptor. The EC₅₀ value is a measured value of the concentration of a compound required to achieve half of its maximum activity in a specific assay. In some embodiments of the present disclosure, for example, when evaluated using the assay methods described in the test examples, EC₅₀ GLP-1-R and/or EC₅₀ GIP-R and/or EC₅₀ GCG-R are less than 10.0nM, less than 8.0nM, less than 5.0nM, less than 3.0nM, less than 1.0nM, less than 0.9nM, less than 0.8nM, less than 0.7nM, less than 0.6nM, less than 0.5nM, less than 0.4nM, less than 0.3nM, less than 0.2nM, less than 0.1nM, less than 0.09nM, less than 0.08nM, less than 0.07nM, less than 0.06nM, less than 0.05nM, less than 0.04nM, less than 0.03nM, less than 0.02nM, less than 0.01nM, less than 0.009nM, less than 0.008nM, less than 0.007nM, less than 0.006nM, less than 0.005nM, less than 0.004nM, less than 0.003nM, or less than 0.002nM.

In some embodiments, the GLP-1/GIP/GCG receptor triagonist has improved solubility.

In some embodiments, the GLP-1/GIP/GCG receptor triagonist has improved chemical and/or physical stability.

In some embodiments, the GLP-1/GIP/GCG receptor triagonist has improved pharmacokinetic properties.

The pharmacokinetic parameters of the compounds in the present disclosure can be determined using assays known in the art. For example, area under the curve (AUC), clearance rate (CL), half-life (T_{1/2}), and the like. In some embodiments, the pharmacokinetic properties of the compounds are determined using the methods provided in the examples of the present disclosure.

In some embodiments, the GLP-1/GIP/GCG receptor triagonist has extended half-life. For example, compared to native GLP-1, GIP, or GCG. In some embodiments, any of Compound 1 to Compound 48 in the present disclosure has extended half-life. By way of example, the half-lives of the compounds in the present disclosure are ≥30 min, 40 min, 45 min, 50 min, 1 h, 1.5 h, 1.8 h, 2 h, 2.2 h, 2.5 h, 2.8 h, 3.0 h, 3.2 h, 3.5 h, 3.8 h, 4.0 h, 4.2 h, 4.5 h, 4.8 h, 5.0 h, 5.2 h, 5.5 h, 5.8 h, 6.0 h, 6.2 h, 6.5 h, 6.8 h, 7.0 h, 7.2 h, 7.5 h, 7.8 h, 8.0 h, 8.2 h, 8.5 h, 8.8 h, 9.0 h, 9.2 h, 9.5 h, 9.8 h, 10.0 h, 10.2 h, 10.5 h, 10.8 h, 11.0 h, 11.2 h, 11.5 h, 11.8 h, 12.0 h, 12.2 h, 12.5 h, 12.8 h, 13.0 h, 13.2 h, 13.5 h, 13.8 h, 14.0 h, 14.2 h, 14.5 h, 14.8 h, 15.0 h, 15.2 h, 15.5 h, 15.8 h, 16.0 h, 16.5 h, 16.0 h, 17.0 h, 17.5 h, 18.0 h, 18.5 h, 19.0 h, 19.5 h, 20.0 h, 25 h, 30 h, 40 h, 50 h, 60 h, 70 h, 80 h, 90 h, 100 h, 120 h, 150 h, 200 h, etc. (including any range between any of the above point values).

### Pharmaceutical composition

In another aspect, the present disclosure provides a pharmaceutical composition comprising a compound with any of the foregoing structures or a pharmaceutically acceptable salt or solvate thereof, or any of the foregoing GLP-1/GIP/GCG receptor triagonists.

In some embodiments, the pharmaceutical composition further comprises a pharmaceutically acceptable excipient or pharmaceutical carrier.

In some embodiments, the pharmaceutical composition comprises a therapeutically effective amount of the compound or a pharmaceutically acceptable salt or solvate thereof, or the GLP-1/GIP/GCG receptor triagonist. In some embodiments, the pharmaceutical composition may contain 0.01 to 99% by weight of the foregoing compound or a pharmaceutically acceptable salt or solvate thereof in a unit dose, or the amount of the foregoing compound or a pharmaceutically acceptable salt or solvate thereof in a unit dose of the pharmaceutical composition is 0.1-2000 mg, and in some embodiments, 1-1000 mg.

### Medical use and treatment method

In another aspect, the present disclosure provides use of the foregoing compound or pharmaceutically acceptable salt thereof, GLP-1/GIP/GCG receptor triagonist or pharmaceutical composition as a medicament.

In some embodiments, the present disclosure provides use of the foregoing compound or pharmaceutically acceptable salt thereof, GLP-1/GIP/GCG receptor triagonist, or pharmaceutical composition in the preparation of a medicament for preventing and/or treating a disease or condition related to metabolic disorders.

In some embodiments, the disease or condition related to metabolic disorders comprises diabetes, diabetes-related conditions, obesity, obesity-related conditions, body weight control, and metabolic dysfunction-associated steatotic liver disease (MASLD).

In some embodiments, the present disclosure provides a method for preventing or treating a disease, comprising administering to a subject in need the foregoing compound or pharmaceutically acceptable salt thereof, GLP-1/GIP/GCG receptor triagonist, or pharmaceutical composition.

In some embodiments, the present disclosure also provides a method for preventing and/or treating a disease or condition related to metabolic disorders, comprising administering to a subject in need the foregoing compound or pharmaceutically acceptable salt thereof.

In some embodiments, the disease or condition related to metabolic disorders comprises diabetes, diabetes-related conditions, obesity, obesity-related conditions, body weight control, and metabolic dysfunction-associated steatotic liver disease.

In some embodiments, the present disclosure provides any of the following uses of the foregoing compound or pharmaceutically acceptable salt thereof, triagonist, or pharmaceutical composition:
(i) prevention and/or treatment of eating disorders such as obesity, for example, by reducing food intake, increasing energy expenditure, reducing body weight, suppressing appetite, or inducing satiety;
(ii) body weight maintenance after successful weight loss (whether induced by medication or by diet and exercise) - that is, preventing body weight gain after successful weight loss.
(iii) prevention and/or treatment of all forms of diabetes.
(iv) delaying or prevention of the progression of diabetes.

In some embodiments, the present disclosure provides any of the following uses of the foregoing compound or pharmaceutically acceptable salt thereof, triagonist, or pharmaceutical composition:
(1) for activating GLP-1 receptor, GIP receptor and GCG receptor,
(2) in the preparation of a medicament for activating GLP-1 receptor, GIP receptor and GCG receptor,
(3) as a medicament for preventing and/or treating diseases,
(4) in the preparation of a medicament for preventing and/or treating diseases,
(5) for preventing and/or treating diseases or conditions related to metabolic disorders,
(6) in the preparation of a medicament for preventing and/or treating diseases or conditions related to metabolic disorders.

In some embodiments, the disease or condition related to metabolic disorders comprises diabetes, diabetes-related conditions, obesity, body weight control, and metabolic dysfunction-associated steatotic liver disease.

### Preparation method

In another aspect, the present disclosure provides a method for preparing the foregoing compound or pharmaceutically acceptable salt thereof, or GLP-1/GIP/GCG receptor triagonist.

In some embodiments, the method is a chemical synthesis method.

In some embodiments, the method further comprises the step of purifying or isolating the compound or the pharmaceutically acceptable salt thereof, or the GLP-1/GIP/GCG receptor triagonist.

In some embodiments, the chemical synthesis method is a solid-phase synthesis process. In some embodiments, the compounds, triagonists, or fragments thereof described herein can be produced by classical peptide synthesis, for example, a solid-phase synthesis process using t-Boc or Fmoc chemistry, or other established techniques; see, for example, Greene and Wuts, "Protective Groups in Organic Synthesis", John Wiley & Sons, 1999, Florencio Zaragoza "Organic Synthesis on solid Phase", Wiley-VCH Verlag GmbH, 2000, and "Fmoc Solid Phase Peptide Synthesis", edited by W.C.Chan and P.D.White, Oxford University Press, 2000. In some embodiments, the method for preparing the compound or GLP-1/GIP/GCG receptor triagonist comprises a solid-phase peptide synthesis step.

In some embodiments, the compound or GLP-1/GIP/GCG receptor triagonist described herein can be produced by a recombinant method, i.e. by culturing host cells containing a DNA sequence encoding the peptide sequence of the compound or GLP-1/GIP/GCG receptor triagonist and capable of expressing the peptide in a suitable nutrient medium under conditions that allow the peptide to be expressed. Non-limiting examples of host cells suitable for peptide expression are: Escherichia coli, Saccharomyces cerevisiae, and mammalian BHK or CHO cell lines.

As non-limiting examples, the compounds and derivatives thereof provided in the present disclosure are obtained using the Fmoc-tBu solid-phase synthesis method, and the carrier for synthesis is Rink-amide MBHA (Xi'an Sunresin New Materials) resin. In the synthesis process, the α-amino group of an amino acid derivative used is protected by the Fmoc group (fluorenylmethoxycarbonyl). For the side chain of an amino acid, the following protecting groups are selected according to different functional groups: for example, the sulfydryl group of the cysteine side chain, the amido group of the glutamine side chain, and the imidazole group of the histidine side chain are protected by Trt (trityl); the guanidine group of the arginine side chain is protected by Pbf (2,2,4,6,7-pentamethyldihydrobenzofuran-5-sulfonyl); the indole group of the tryptophan side chain and the amino group of the lysine side chain are protected by Boc (tert-butyloxycarbonyl); the hydroxyl group of the threonine side chain, the phenol group of the tyrosine side chain, and the hydroxyl group of the serine side chain are protected by t-Bu (tert-butyl); and the carboxyl group of glutamic acid and aspartic acid side chains is protected by OtBu (tert-butyl ester), and the like. During the synthesis process, the carboxyl group of the amino acid residue at the C-terminal of the polypeptide is first condensed to the high molecular weight insoluble Rink-amide MBHA resin in the form of an amide bond, and then the Fmoc protecting group on the α-amino group is removed using an N,N-dimethylformamide (DMF) solution containing 20% 4-methylpiperidine. The solid phase carrier is then condensed in excess with the next amino acid derivative in the sequence to form an amide bond to extend the peptide chain. The procedures of condensation → washing → deprotection → washing → next round of amino acid condensation are repeated to achieve the desired length of the polypeptide chain to be synthesized. Finally, the resin is reacted with a mixed solution of trifluoroacetic acid : water : triisopropylsilane (90:5:5, v:v:v) to cleave the polypeptide from the solid phase carrier, which is then precipitated with methyl tert-butyl ether to obtain a solid crude of the polypeptide derivative. The solid of the crude peptide is dissolved in a mixed solution of acetonitrile/water containing 0.1% trifluoroacetic acid and then purified and separated using a C-18 reversed-phase preparative chromatography column to obtain a pure product of the polypeptide and derivative thereof.

### Particular embodiments

The technical solutions of the present disclosure are further described through the following non-limiting embodiments:
1. A compound represented by formula (I) or a pharmaceutically acceptable salt thereof:

   R₁-X₁-X₂-X₃-Gly-Thr-Phe-Thr-Ser-Asp-X₁₀-Ser-X₁₂-X₁₃-X₁₄-Glu-X₁₆-X₁₇-X₁₈-X₁₉-X₂₀-X₂₁-Phe-Val-Glu-Trp-Leu-X₂₇-X₂₈-X₂₉-Z-X₄₀-R₂ (SEQ ID NO: 145) (I);

   wherein R₁ is hydrogen, alkyl, acetyl, formyl, benzoyl, trifluoroacetyl, or pGlu, and R₂ is -NH₂ or -OH;
   X₁ is selected from an amino acid residue of Tyr or His;
   X₂ is an amino acid residue of Aib;
   X₃ is selected from an amino acid residue of Gln or His;
   X₁₀ is selected from an amino acid residue of Leu or Tyr;
   X₁₂ is selected from an amino acid residue of Lys, alpha-methyl-Lys, or Ile;
   X₁₃ is selected from an amino acid residue of Aib, Ile, Leu, Tyr, alpha-methyl-Tyr or alpha-methyl-Leu;
   X₁₄ is selected from an amino acid residue of Leu or alpha-methyl-Leu;
   X₁₆ is selected from an amino acid residue of Glu or Lys, or Y₁;
   X₁₇ is selected from an amino acid residue of Glu, Arg, homoArg, Ile, Lys, Gln or alpha-methyl-Lys, or Y₁;
   X₁₈ is selected from an amino acid residue of Ala or Aib;
   X₁₉ is selected from an amino acid residue of Ala or Gln;
   X₂₀ is selected from an amino acid residue of Gln, Aib, isovaline, alpha-methyl-Ser, 1-aminocyclopropane-1-carboxylic acid or 1-aminocyclobutane-1-carboxylic acid;
   X₂₁ is selected from an amino acid residue of Glu, or Y₁;
   X₂₇ is selected from an amino acid residue of Ile or Leu;
   X₂₈ is selected from an amino acid residue of Ala or Glu;
   X₂₉ is selected from an amino acid residue of Gly or D-Glu;
   X₄₀ is selected from an amino acid residue of Lys, or Y₁, or is absent;
   Z is selected from Gly-Pro-Ser-Ser-Gly-Ala-Pro-Pro-Pro-Ser (SEQ ID NO: 146), Gly-Pro-Ser-Ser-Gly-Ala-Pro-Pro-Ser (SEQ ID NO: 147), Pro-Ser-Ser-Gly-Ala-Pro-Pro-Pro-Ser (SEQ ID NO: 148), or Pro-Ser-Ser-Gly-Ala-Pro-Pro-Ser (SEQ ID NO: 149);
   Y₁ is a Lys, Orn, Dap, Dab, or Cys residue with the side chain linked to a substituent.
2. The compound or the pharmaceutically acceptable salt thereof according to embodiment 1, which is a compound represented by formula (Ia) or a pharmaceutically acceptable salt thereof:

   R₁-X₁-X₂-X₃-Gly-Thr-Phe-Thr-Ser-Asp-X₁₀-Ser-X₁₂-X₁₃-X₁₄-Glu-Y₁-X₁₇-X₁₈-X₁₉-X₂₀-Glu-Phe-Val-Glu-Trp- Leu-X₂₇-X₂₈-X₂₉-Z-X₄₀-R₂ (Ia);

   wherein R₁ is hydrogen, alkyl, acetyl, formyl, benzoyl, trifluoroacetyl, or pGlu, and R₂ is -NH₂ or -OH;
   X₁ is selected from an amino acid residue of Tyr or His;
   X₂ is an amino acid residue of Aib;
   X₃ is selected from an amino acid residue of Gln or His;
   X₁₀ is selected from an amino acid residue of Leu or Tyr;
   X₁₂ is selected from an amino acid residue of Lys, alpha-methyl-Lys, or Ile;
   X₁₃ is selected from an amino acid residue of Aib, Ile, Leu, Tyr, alpha-methyl-Tyr or alpha-methyl-Leu;
   X₁₄ is selected from an amino acid residue of Leu or alpha-methyl-Leu;
   X₁₇ is selected from an amino acid residue of Glu, Arg, homoArg, Ile, Lys, Gln, or alpha-methyl-Lys;
   X₁₈ is selected from an amino acid residue of Ala or Aib;
   X₁₉ is selected from an amino acid residue of Ala or Gln;
   X₂₀ is selected from an amino acid residue of Gln, Aib, isovaline, alpha-methyl-Ser, 1-aminocyclopropane-1-carboxylic acid or 1-aminocyclobutane-1-carboxylic acid;
   X₂₇ is selected from an amino acid residue of Ile or Leu;
   X₂₈ is selected from an amino acid residue of Ala or Glu;
   X₂₉ is selected from an amino acid residue of Gly or D-Glu;
   X₄₀ is selected from an amino acid residue of Lys, or is absent;
   Z is selected from Gly-Pro-Ser-Ser-Gly-Ala-Pro-Pro-Pro-Ser (SEQ ID NO: 146), Gly-Pro-Ser-Ser-Gly-Ala-Pro-Pro-Ser (SEQ ID NO: 147), Pro-Ser-Ser-Gly-Ala-Pro-Pro-Pro-Ser (SEQ ID NO: 148), or Pro-Ser-Ser-Gly-Ala-Pro-Pro-Ser (SEQ ID NO: 149);
   Y₁ is a Lys, Orn, Dap, Dab, or Cys residue with the side chain linked to a substituent.
3. The compound or the pharmaceutically acceptable salt thereof according to embodiment 1 or 2, which is a compound represented by formula (Ib) or a pharmaceutically acceptable salt thereof:

   R₁-X₁-X₂-X₃-Gly-Thr-Phe-Thr-Ser-Asp-X₁₀-Ser-X₁₂-X₁₃-X₁₄-Glu-Y₁-X₁₇-X₁₈-X₁₉-X₂₀-Glu-Phe-Val-Glu-Trp-Leu-X₂₇-X₂₈-X₂₉-Gly-Pro-Ser-Ser-Gly-Ala-Pro-Pro-Pro-Ser-R₂ (SEQ ID NO: 150) (Ib);

   wherein R₁ is hydrogen, alkyl, acetyl, formyl, benzoyl, trifluoroacetyl, or pGlu, and R₂ is -NH₂ or -OH;
   X₁ is selected from an amino acid residue of Tyr or His;
   X₂ is an amino acid residue of Aib;
   X₃ is selected from an amino acid residue of Gln or His;
   X₁₀ is selected from an amino acid residue of Leu or Tyr;
   X₁₂ is selected from an amino acid residue of Lys, alpha-methyl-Lys, or Ile;
   X₁₃ is selected from an amino acid residue of Aib, Ile, Leu, Tyr, alpha-methyl-Tyr or alpha-methyl-Leu;
   X₁₄ is selected from an amino acid residue of Leu or alpha-methyl-Leu;
   X₁₇ is selected from an amino acid residue of Ile, Lys, Gln, or alpha-methyl-Lys;
   X₁₈ is selected from an amino acid residue of Ala or Aib;
   X₁₉ is selected from an amino acid residue of Ala or Gln;
   X₂₀ is selected from an amino acid residue of Gln, Aib, isovaline, alpha-methyl-Ser, 1-aminocyclopropane-1-carboxylic acid or 1-aminocyclobutane-1-carboxylic acid;
   X₂₇ is selected from an amino acid residue of Ile or Leu;
   X₂₈ is selected from an amino acid residue of Ala or Glu;
   X₂₉ is selected from an amino acid residue of Gly or D-Glu;
   Y₁ is a Lys, Orn, Dap, Dab, or Cys residue with the side chain linked to a substituent.
4. The compound or the pharmaceutically acceptable salt thereof according to any one of embodiments 1-3, wherein X₁, X₂ and X₃ are selected from any one of the following:
   X₁ is His, X₂ is Aib, and X₃ is His;
   X₁ is Tyr, X₂ is Aib, and X₃ is Gln; or
   X₁ is His, X₂ is Aib, and X₃ is Gln.
5. The compound or the pharmaceutically acceptable salt thereof according to any one of embodiments 1-3, wherein X₁ is His, X₂ is Aib, and X₃ is His; or
   X₁ is His, X₂ is Aib, and X₃ is Gln.
6. The compound or the pharmaceutically acceptable salt thereof according to any one of embodiments 1-3, wherein X₁ is His, X₂ is Aib, and X₃ is His.
7. The compound or the pharmaceutically acceptable salt thereof according to any one of embodiments 1-6, wherein X₁₇ is a Lys residue.
8. The compound or the pharmaceutically acceptable salt thereof according to any one of embodiments 1-6, wherein X₁₇ is a Gln residue.
9. The compound or the pharmaceutically acceptable salt thereof according to any one of embodiments 1-6, wherein X₁₇ is an alpha-methyl-Lys residue.
10. The compound or the pharmaceutically acceptable salt thereof according to any one of embodiments 1-6, wherein X₁₇ is an Ile residue.
11. The compound or the pharmaceutically acceptable salt thereof according to any one of embodiments 1-10, wherein X₁₈ is an Ala residue.
12. The compound or the pharmaceutically acceptable salt thereof according to any one of embodiments 1-11, wherein X₁₉ is an Ala residue.
13. The compound or the pharmaceutically acceptable salt thereof according to any one of embodiments 1-12, wherein X₂₀ is a Gln residue.
14. The compound or the pharmaceutically acceptable salt thereof according to any one of embodiments 1-12, wherein X₂₀ is an Aib residue.
15. The compound or the pharmaceutically acceptable salt thereof according to any one of embodiments 1-12, wherein X₂₀ is an isovaline residue.
16. The compound or the pharmaceutically acceptable salt thereof according to any one of embodiments 1-12, wherein X₂₀ is an alpha-methyl-Ser residue.
17. The compound or the pharmaceutically acceptable salt thereof according to any one of embodiments 1-12, wherein X₂₀ is an 1-aminocyclobutane-1-carboxylic acid residue.
18. The compound or the pharmaceutically acceptable salt thereof according to any one of embodiments 1-12, wherein X₂₀ is an 1-aminocyclopropane-1-carboxylic acid residue.
19. The compound or the pharmaceutically acceptable salt thereof according to any one of embodiments 1-6, wherein X₁₇, X₁₈, X₁₉ and X₂₀ are selected from any one of the following:
   X₁₇ is Lys, X₁₈ is Ala, X₁₉ is Ala, and X₂₀ is Gln;
   X₁₇ is Lys, X₁₈ is Ala, X₁₉ is Ala, and X₂₀ is alpha-methyl-Ser;
   X₁₇ is Lys, X₁₈ is Ala, X₁₉ is Ala, and X₂₀ is isovaleine;
   X₁₇ is Lys, X₁₈ is Ala, X₁₉ is Ala, and X₂₀ is 1-aminocyclopropane-1-carboxylic acid;
   X₁₇ is Lys, X₁₈ is Ala, X₁₉ is Ala, and X₂₀ is 1-aminocyclobutane-1-carboxylic acid;
   X₁₇ is Lys, X₁₈ is Ala, X₁₉ is Ala, and X₂₀ is Aib;
   X₁₇ is Lys, X₁₈ is Aib, X₁₉ is Ala, and X₂₀ is Gln;
   X₁₇ is Lys, X₁₈ is Ala, X₁₉ is Gln, and X₂₀ is Gln;
   X₁₇ is Gln, X₁₈ is Ala, X₁₉ is Ala, and X₂₀ is Gln;
   X₁₇ is Gln, X₁₃ is Ala, X₁₉ is Ala, and X₂₀ is isovaleine;
   X₁₇ is Gln, X₁₈ is Ala, X₁₉ is Ala, and X₂₀ is 1-aminocyclopropane-1-carboxylic acid;
   X₁₇ is Gln, X₁₈ is Ala, X₁₉ is Ala, and X₂₀ is 1-aminocyclobutane-1-carboxylic acid;
   X₁₇ is alpha-methyl-Lys, X₁₈ is Ala, X₁₉ is Ala, and X₂₀ is Gln; or
   X₁₇ is Ile, X₁₈ is Ala, X₁₉ is Ala, and X₂₀ is Gln.
20. The compound or the pharmaceutically acceptable salt thereof according to any one of embodiments 1-19, wherein X₁₂ is an alpha-methyl-Lys residue.
21. The compound or the pharmaceutically acceptable salt thereof according to any one of embodiments 1-19, wherein X₁₂ is an Ile residue.
22. The compound or the pharmaceutically acceptable salt thereof according to any one of embodiments 1-19, wherein X₁₂ is a Lys residue.
23. The compound or the pharmaceutically acceptable salt thereof according to any one of embodiments 1-22, wherein X₁₃ is a Leu residue.
24. The compound or the pharmaceutically acceptable salt thereof according to any one of embodiments 1-22, wherein X₁₃ is an alpha-methyl-Leu residue.
25. The compound or the pharmaceutically acceptable salt thereof according to any one of embodiments 1-22, wherein X₁₃ is an Ile residue.
26. The compound or the pharmaceutically acceptable salt thereof according to any one of embodiments 1-22, wherein X₁₃ is a Tyr residue.
27. The compound or the pharmaceutically acceptable salt thereof according to any one of embodiments 1-22, wherein X₁₃ is an Aib residue.
28. The compound or the pharmaceutically acceptable salt thereof according to any one of embodiments 1-22, wherein X₁₃ is an alpha-methyl-Tyr residue.
29. The compound or the pharmaceutically acceptable salt thereof according to any one of embodiments 1-22, wherein X₁₄ is a Leu residue.
30. The compound or the pharmaceutically acceptable salt thereof according to any one of embodiments 1-22, wherein X₁₄ is an alpha-methyl-Leu residue.
31. The compound or the pharmaceutically acceptable salt thereof according to any one of embodiments 1-19, wherein X₁₂, X₁₃ and X₁₄ are selected from any one of the following:
   X₁₂ is Ile, X₁₃ is Leu, and X₁₄ is Leu;
   X₁₂ is Ile, X₁₃ is alpha-methyl-Leu, and X₁₄ is Leu;
   X₁₂ is Lys, X₁₃ is alpha-methyl-Tyr, and X₁₄ is Leu;
   X₁₂ is Lys, X₁₃ is Aib, and X₁₄ is Leu;
   X₁₂ is Lys, X₁₃ is Tyr, and X₁₄ is alpha-methyl-Leu;
   X₁z is Lys, X₁₃ is Tyr, and X₁₄ is Leu;
   X₁₂ is alpha-methyl-Lys, X₁₃ is Tyr, and X₁₄ is Leu;
   X₁₂ is alpha-methyl-Lys, X₁₃ is Leu, and X₁₄ is Leu; or
   X₁₂ is alpha-methyl-Lys, X₁₃ is Ile, and X₁₄ is Leu;.
32. The compound or the pharmaceutically acceptable salt thereof according to any one of embodiments 1-31, wherein X₂₇ is an Ile residue.
33. The compound or the pharmaceutically acceptable salt thereof according to any one of embodiments 1-31, wherein X₂₇ is a Leu residue.
34. The compound or the pharmaceutically acceptable salt thereof according to any one of embodiments 1-33, wherein X₂₈ is an Ala residue.
35. The compound or the pharmaceutically acceptable salt thereof according to any one of embodiments 1-33, wherein X₂₈ is a Gln residue.
36. The compound or the pharmaceutically acceptable salt thereof according to any one of embodiments 1-35, wherein X₂₉ is a Gly residue.
37. The compound or the pharmaceutically acceptable salt thereof according to any one of embodiments 1-35, wherein X₂₉ is a D-Glu residue.
38. The compound or the pharmaceutically acceptable salt thereof according to any one of embodiments 1-37, which is a compound represented by formula (Ib) or a pharmaceutically acceptable salt thereof:

   R₁-X₁-X₂-X₃-Gly-Thr-Phe-Thr-Ser-Asp-X₁₀-Ser-X₁₂-X₁₃-X₁₄-Glu-Y₁-X₁₇-X₁₈-X₁₉-X₂₀-Glu-Phe-Val-Glu-Trp-Leu-X₂₇-X₂₈-X₂₉-Gly-Pro-Ser-Ser-Gly-Ala-Pro-Pro-Pro-Ser-R₂ (SEQ ID NO: 150) (Ib);

   wherein R₁ is hydrogen, alkyl, acetyl, formyl, benzoyl, trifluoroacetyl, or pGlu, and R₂ is -NH₂ or -OH;
   X₁, X₂ and X₃ are selected from any of the following:
      X₁ is His, X₂ is Aib, and X₃ is His;
      X₁ is Tyr, X₂ is Aib, and X₃ is Gln; or
      X₁ is His, X₂ is Aib, and X₃ is Gln;
      X₁₀ is selected from an amino acid residue of Leu or Tyr;
      X₁₂ is selected from an amino acid residue of Lys, alpha-methyl-Lys, or Ile;
      X₁₃ is selected from an amino acid residue of Aib, Ile, Leu, Tyr, alpha-methyl-Tyr or alpha-methyl-Leu;
      X₁₄ is selected from an amino acid residue of Leu or alpha-methyl-Leu;
      X₁₇ is selected from an amino acid residue of Lys;
      X₁₈ is selected from an amino acid residue of Ala or Aib;
      X₁₉ is selected from an amino acid residue of Ala or Gln;
      X₂₀ is selected from an amino acid residue of Gln, Aib, isovaline, alpha-methyl-Ser, 1-aminocyclopropane-1-carboxylic acid or 1-aminocyclobutane-1-carboxylic acid;
      X₂₇ is selected from an amino acid residue of Ile or Leu;
      X₂₈ is selected from an amino acid residue of Ala or Glu;
      X₂₉ is selected from an amino acid residue of Gly or D-Glu;
      Y₁ is a Lys, Orn, Dap, Dab, or Cys residue with the side chain linked to a substituent.
39. The compound or the pharmaceutically acceptable salt thereof according to embodiment 38, wherein:
   X₁ is His, X₂ is Aib, and X₃ is His; or
   X₁ is His, X₂ is Aib, and X₃ is Gln.
40. The compound or the pharmaceutically acceptable salt thereof according to embodiment 38 or 39, wherein:
   X₁ is His, X₂ is Aib, and X₃ is His; or
   X₁ is His, X₂ is Aib, and X₃ is Gln;
   X₁₀ is selected from an amino acid residue of Leu or Tyr,
   X₁₂ is selected from an amino acid residue of Lys, alpha-methyl-Lys, or Ile,
   X₁₃ is selected from an amino acid residue of Aib, Ile, Leu, Tyr, alpha-methyl-Tyr or alpha-methyl-Leu,
   X₁₄ is selected from an amino acid residue of Leu or alpha-methyl-Leu,
   X₁₇ is selected from an amino acid residue of Lys,
   X₁₈ is selected from an amino acid residue of Ala or Aib,
   X₁₉ is selected from an amino acid residue of Ala or Gln,
   X₂₀ is selected from an amino acid residue of Gln,
   X₂₇ is selected from an amino acid residue of Ile or Leu,
   X₂₈ is selected from an amino acid residue of Ala or Glu,
   X₂₉ is selected from an amino acid residue of Gly or D-Glu.
41. The compound or the pharmaceutically acceptable salt thereof according to any one of embodiments 38-40, wherein:
   X₁ is His,
   X₂ is Aib,
   X₃ is His,
   X₁₀ is an amino acid residue of Tyr,
   X₁₂ is selected from an amino acid residue of Lys or alpha-methyl-Lys,
   X₁₃ is selected from an amino acid residue of Aib, Ile or alpha-methyl-Tyr,
   X₁₄ is an amino acid residue of Leu,
   X₁₇ is an amino acid residue of Lys,
   X₁₈ is an amino acid residue of Ala,
   X₁₉ is an amino acid residue of Ala,
   X₂₀ is an amino acid residue of Gln,
   X₂₇ is an amino acid residue of Leu,
   X₂₈ is an amino acid residue of Ala,
   X₂₉ is an amino acid residue of Gly.
42. The compound or the pharmaceutically acceptable salt thereof according to any one of embodiments 38-40, wherein:
   X₁ is His,
   X₂ is Aib,
   X₃ is His or Gln,
   X₁₀ is selected from an amino acid residue of Leu or Tyr,
   X₁₂ is selected from an amino acid residue of alpha-methyl-Lys or Ile,
   X₁₃ is an amino acid residue of Leu,
   X₁₄ is an amino acid residue of Leu,
   X₁₇ is an amino acid residue of Lys,
   X₁₈ is an amino acid residue of Ala,
   X₁₉ is an amino acid residue of Ala,
   X₂₀ is an amino acid residue of Gln,
   X₂₇ is an amino acid residue of Leu,
   X₂₈ is an amino acid residue of Ala,
   X₂₉ is an amino acid residue of Gly or D-Glu.
43. The compound or the pharmaceutically acceptable salt thereof according to any one of embodiments 38-40, wherein:
   X₁ is His,
   X₂ is Aib,
   X₃ is His or Gln,
   X₁₀ is selected from an amino acid residue of Leu or Tyr,
   X₁₂ is an amino acid residue of Ile,
   X₁₃ is an amino acid residue of alpha-methyl-Leu,
   X₁₄ is an amino acid residue of Leu,
   X₁₇ is an amino acid residue of Lys,
   X₁₈ is an amino acid residue of Ala,
   X₁₉ is an amino acid residue of Ala,
   X₂₀ is an amino acid residue of Gln,
   X₂₇ is an amino acid residue of Leu,
   X₂₈ is an amino acid residue of Ala,
   X₂₉ is an amino acid residue of Gly or D-Glu.
44. The compound or the pharmaceutically acceptable salt thereof according to embodiment 38 or 39, wherein:
   X₁, X₂ and X₃ are selected from any of the following:
   X₁ is His, X₂ is Aib, and X₃ is His;
   X₁ is His, X₂ is Aib, and X₃ is Gln; or
   X₁ is Tyr, X₂ is Aib, and X₃ is Gln;
   X₁₀ is selected from an amino acid residue of Leu or Tyr,
   X₁₂ is selected from an amino acid residue of alpha-methyl-Lys or Ile,
   X₁₃ is selected from an amino acid residue of Ile, Leu or alpha-methyl-Leu,
   X₁₄ is selected from an amino acid residue of Leu,
   X₁₇ is selected from an amino acid residue of Lys,
   X₁₈ is selected from an amino acid residue of Ala,
   X₁₉ is selected from an amino acid residue of Ala,
   X₂₀ is selected from an amino acid residue of Aib, isovaline, alpha-methyl-Ser, 1-aminocyclopropane-1-carboxylic acid or 1-aminocyclobutane-1-carboxylic acid,
   X₂₇ is selected from an amino acid residue of Ile or Leu,
   X₂₈ is selected from an amino acid residue of Ala or Glu,
   X₂₉ is selected from an amino acid residue of Gly or D-Glu.
45. The compound or the pharmaceutically acceptable salt thereof according to embodiment 44, wherein:
   X₁ is His,
   X₂ is Aib,
   X₃ is His or Gln,
   X₁₀ is selected from an amino acid residue of Leu or Tyr,
   X₁₂ is an amino acid residue of Ile,
   X₁₃ is selected from an amino acid residue of alpha-methyl-Leu,
   X₁₄ is selected from an amino acid residue of Leu,
   X₁₇ is selected from an amino acid residue of Lys,
   X₁₈ is selected from an amino acid residue of Ala,
   X₁₉ is selected from an amino acid residue of Ala,
   X₂₀ is selected from an amino acid residue of Aib or isovaleine,
   X₂₇ is selected from an amino acid residue of Leu,
   X₂₈ is selected from an amino acid residue of Ala,
   X₂₉ is selected from an amino acid residue of Gly.
46. The compound or the pharmaceutically acceptable salt thereof according to embodiment 44, wherein:
   X₁-X₂-X₃ is selected from His-Aib-His, His-Aib-Gln, or Tyr-Aib-Gln,
   X₁₀ is selected from an amino acid residue of Leu or Tyr,
   X₁₂ is selected from an amino acid residue of alpha-methyl-Lys or Ile,
   X₁₃ is selected from an amino acid residue of Leu,
   X₁₄ is selected from an amino acid residue of Leu,
   X₁₇ is selected from an amino acid residue of Lys,
   X₁₈ is selected from an amino acid residue of Ala,
   X₁₉ is selected from an amino acid residue of Ala,
   X₂₀ is selected from an amino acid residue of Aib, isovaline, alpha-methyl-Ser, 1-aminocyclopropane-1-carboxylic acid or 1-aminocyclobutane-1-carboxylic acid,
   X₂₇ is selected from an amino acid residue of Ile or Leu,
   X₂₈ is selected from an amino acid residue of Ala or Glu,
   X₂₉ is selected from an amino acid residue of Gly or D-Glu.
47. The compound or the pharmaceutically acceptable salt thereof according to embodiment 44, wherein:
   X₁, X₂ and X₃ are selected from any of the following:
   X₁ is His, X₂ is Aib, and X₃ is His; or
   X₁ is Tyr, X₂ is Aib, and X₃ is Gln;
   X₁₀ is selected from an amino acid residue of Leu or Tyr,
   X₁₂ is selected from an amino acid residue of alpha-methyl-Lys,
   X₁₃ is selected from an amino acid residue of Ile,
   X₁₄ is selected from an amino acid residue of Leu,
   X₁₇ is selected from an amino acid residue of Lys,
   X₁₈ is selected from an amino acid residue of Ala,
   X₁₉ is selected from an amino acid residue of Ala,
   X₂₀ is selected from an amino acid residue of isovaleine,
   X₂₇ is selected from an amino acid residue of Ile or Leu,
   X₂₈ is selected from an amino acid residue of Ala or Glu,
   X₂₉ is selected from an amino acid residue of Gly.
48. The compound or the pharmaceutically acceptable salt thereof according to any one of embodiments 44-47, wherein X₁ is His; X₂ is Aib; X₃ is His or Gln.
49. The compound or the pharmaceutically acceptable salt thereof according to any one of embodiments 1-37, which comprises a compound represented by formula (Ib) or a pharmaceutically acceptable salt thereof:

   R₁-X₁-X₂-X₃-Gly-Thr-Phe-Thr-Ser-Asp-X₁₀-Ser-X₁₂-X₁₃-X₁₄-Glu-Y₁-X₁₇-X_{1S}-X₁₉-X₂₀-Glu-Phe-Val-Glu-Trp-Leu-X₂₇-X₂₈-X₂₉-Gly-Pro-Ser-Ser-Gly-Ala-Pro-Pro-Pro-Ser-R₂ (SEQ ID NO: 150) (Ib);

   wherein R₁ is hydrogen, alkyl, acetyl, formyl, benzoyl, trifluoroacetyl, or pGlu, and R₂ is -NH₂ or -OH;
   X₁ is His;
   X₂ is Aib;
   X₃ is His;
   X₁₀ is selected from an amino acid residue of Tyr;
   X₁₂ is selected from an amino acid residue of Lys or alpha-methyl-Lys;
   X₁₃ is selected from an amino acid residue of Tyr;
   X₁₄ is selected from an amino acid residue of Leu or alpha-methyl-Leu;
   X₁₇ is selected from an amino acid residue of Lys;
   X₁₈ is selected from an amino acid residue of Ala or Aib;
   X₁₉ is selected from an amino acid residue of Ala or Gln;
   X₂₀ is selected from an amino acid residue of Gln;
   X₂₇ is selected from an amino acid residue of Ile or Leu;
   X₂₈ is selected from an amino acid residue of Ala or Glu;
   X₂₉ is selected from an amino acid residue of Gly;
   Y₁ is a Lys, Orn, Dap, Dab, or Cys residue with the side chain linked to a substituent.
50. The compound or the pharmaceutically acceptable salt thereof according to embodiment 49, wherein X₁₈ is selected from an amino acid residue of Ala.
51. The compound or the pharmaceutically acceptable salt thereof according to embodiment 49, wherein X₁₉ is selected from an amino acid residue of Ala.
52. The compound or the pharmaceutically acceptable salt thereof according to any one of embodiments 1-37, which is a compound represented by formula (Ib) or a pharmaceutically acceptable salt thereof:

   R₁-X₁-X₂-X₃-Gly-Thr-Phe-Thr-Ser-Asp-X₁₀-Ser-X₁₂-X₁₃-X₁₄-Glu-Y₁-X₁₇-X₁₈-X₁₉-X₂₀-Glu-Phe-Val-Glu-Trp-Leu-X₂₇-X₂₈-X₂₉-Gly-Pro-Ser-Ser-Gly-Ala-Pro-Pro-Pro-Ser-R₂ (SEQ ID NO: 150) (Ib);

   wherein R₁ is hydrogen, alkyl, acetyl, formyl, benzoyl, trifluoroacetyl, or pGlu, and R₂ is -NH₂ or -OH;
   X₁ is His;
   X₂ is Aib;
   X₃ is His;
   X₁₀ is selected from an amino acid residue of Leu or Tyr;
   X₁₂ is selected from an amino acid residue of Ile;
   X₁₃ is selected from an amino acid residue of alpha-methyl-Leu;
   X₁₄ is selected from an amino acid residue of Leu;
   X₁₇ is selected from an amino acid residue of Lys;
   X₁₈ is selected from an amino acid residue of Ala;
   X₁₉ is selected from an amino acid residue of Ala;
   X₂₀ is selected from an amino acid residue of Gln, Aib, 1-aminocyclobutane-1-carboxylic acid, or isovaleine;
   X₂₇ is selected from an amino acid residue of Leu;
   X₂₈ is selected from an amino acid residue of Ala;
   X₂₉ is selected from an amino acid residue of Gly or D-Glu;
   Y₁ is a Lys, Orn, Dap, Dab, or Cys residue with the side chain linked to a substituent.
53. The compound or the pharmaceutically acceptable salt thereof according to any one of embodiments 1-37, which is a compound represented by formula (Ib) or a pharmaceutically acceptable salt thereof:

   R₁-X₁-X₂-X₃-Gly-Thr-Phe-Thr-Ser-Asp-X₁₀-Ser-X₁₂-X₁₃-X₁₄-Glu-Y₁-X₁₇-X₁₈-X₁₉-X₂₀-Glu-Phe-Val-Glu-Trp-Leu-X₂₇-X₂₈-X₂₉-Gly-Pro-Ser-Ser-Gly-Ala-Pro-Pro-Pro-Ser-R₂ (SEQ ID NO: 150) (Ib);

   wherein R₁ is hydrogen, alkyl, acetyl, formyl, benzoyl, trifluoroacetyl, or pGlu, and R₂ is -NH₂ or -OH;
   X₁, X₂ and X₃ are selected from any of the following:
      X₁ is His, X₂ is Aib, and X₃ is His;
      X₁ is His, X₂ is Aib, and X₃ is Gln; or
      X₁ is Tyr, X₂ is Aib, and X₃ is Gln;
      X₁₀ is selected from an amino acid residue of Leu or Tyr;
      X₁₂ is selected from an amino acid residue of alpha-methyl-Lys or Ile;
      X₁₃ is selected from an amino acid residue of Leu;
      X₁₄ is selected from an amino acid residue of Leu;
      X₁₇ is selected from an amino acid residue of Lys;
      X₁₈ is selected from an amino acid residue of Ala;
      X₁₉ is selected from an amino acid residue of Ala;
      X₂₀ is selected from an amino acid residue of Gln, Aib, isovaline, alpha-methyl-Ser, 1-aminocyclopropane-1-carboxylic acid or 1-aminocyclobutane-1-carboxylic acid;
      X₂₇ is selected from an amino acid residue of Ile or Leu;
      X₂₈ is selected from an amino acid residue of Ala or Glu;
      X₂₉ is selected from an amino acid residue of Gly or D-Glu;
      Y₁ is a Lys, Orn, Dap, Dab, or Cys residue with the side chain linked to a substituent.
54. The compound or the pharmaceutically acceptable salt thereof according to embodiment 53, wherein X₂₀ is selected from a Gln residue.
55. The compound or the pharmaceutically acceptable salt thereof according to embodiment 53, wherein X₂₀ is selected from an amino acid residue of Aib, isovaleine, alpha-methyl-Ser, 1-aminocyclopropane-1-carboxylic acid or 1-aminocyclobutane-1-carboxylic acid.
56. The compound or the pharmaceutically acceptable salt thereof according to embodiment 53, wherein X₂₀ is selected from an amino acid residue of isovaleine.
57. The compound or the pharmaceutically acceptable salt thereof according to embodiment 53, wherein X₂₀ is selected from an amino acid residue of 1-aminocyclopropane-1-carboxylic acid.
58. The compound or the pharmaceutically acceptable salt thereof according to embodiment 53, wherein X₂₀ is selected from an amino acid residue of 1-aminocyclobutane-1-carboxylic acid.
59. The compound or the pharmaceutically acceptable salt thereof according to any one of embodiments 53-58, wherein X₁ is His, X₂ is Aib, and X₃ is His or Gln.
60. The compound or the pharmaceutically acceptable salt thereof according to any one of embodiments 1-37, which comprises a compound represented by formula (Ib) or a pharmaceutically acceptable salt thereof:

   R₁-X₁-X₂-X₃-Gly-Thr-Phe-Thr-Ser-Asp-X₁₀-Ser-X₁₂-X₁₃-X₁₄-Glu-Y₁-X₁₇-X₁₈-X₁₉-X₂₀-Glu-Phe-Val-Glu-Trp-Leu-X₂₇-X₂₈-X₂₉-Gly-Pro-Ser-Ser-Gly-Ala-Pro-Pro-Pro-Ser-R₂ (SEQ ID NO: 150) (Ib);

   wherein R₁ is hydrogen, alkyl, acetyl, formyl, benzoyl, trifluoroacetyl, or pGlu, and R₂ is -NH₂ or -OH;
   X₁ is His,
   X₂ is Aib,
   X₃ is His,
   X₁₀ is selected from an amino acid residue of Tyr,
   X₁₂ is selected from an amino acid residue of Lys,
   X₁₃ is selected from an amino acid residue of alpha-methyl-Tyr or Aib;
   X₁₄ is selected from an amino acid residue of Leu,
   X₁₇ is selected from an amino acid residue of Lys,
   X₁₈ is selected from an amino acid residue of Ala,
   X₁₉ is selected from an amino acid residue of Ala,
   X₂₀ is selected from an amino acid residue of Gln,
   X₂₇ is selected from an amino acid residue of Leu,
   X₂₈ is selected from an amino acid residue of Ala,
   X₂₉ is selected from an amino acid residue of Gly;
   Y₁ is a Lys, Orn, Dap, Dab, or Cys residue with the side chain linked to a substituent.
61. The compound or the pharmaceutically acceptable salt thereof according to any one of embodiments 1-37, which comprises a compound represented by formula (Ib) or a pharmaceutically acceptable salt thereof:

   R₁-X₁-X₂-X₃-Gly-Thr-Phe-Thr-Ser-Asp-X₁₀-Ser-X₁₂-X₁₃-X₁₄-Glu-Y₁-X₁₇-X₁₈-X₁₉-X₂₀-Glu-Phe-Val-Glu-Trp-Leu-X₂₇-X₂₈-X₂₉-Gly-Pro-Ser-Ser-Gly-Ala-Pro-Pro-Pro-Ser-R₂ (SEQ ID NO: 150) (Ib);

   wherein R₁ is hydrogen, alkyl, acetyl, formyl, benzoyl, trifluoroacetyl, or pGlu, and R₂ is -NH₂ or -OH;
   X₁, X₂ and X₃ are selected from any of the following:
      X₁ is His, X₂ is Aib, and X₃ is His; or
      X₁ is Tyr, X₂ is Aib, and X₃ is Gln;
      X₁₀ is selected from an amino acid residue of Leu or Tyr,
      X₁₂ is selected from an amino acid residue of alpha-methyl-Lys,
      X₁₃ is selected from an amino acid residue of Ile;
      X₁₄ is selected from an amino acid residue of Leu,
      X₁₇ is selected from an amino acid residue of Lys,
      X₁₈ is selected from an amino acid residue of Ala,
      X₁₉ is selected from an amino acid residue of Ala,
      X₂₀ is selected from an amino acid residue of Gln or isovaleine,
      X₂₇ is selected from an amino acid residue of Leu or Ile,
      X₂₈ is selected from an amino acid residue of Ala or Glu,
      X₂₉ is selected from an amino acid residue of Gly;
      Y₁ is a Lys, Orn, Dap, Dab, or Cys residue with the side chain linked to a substituent.
62. The compound or the pharmaceutically acceptable salt thereof according to embodiment 61, wherein X₁-X₂-X₃ is His-Aib-His.
63. The compound or the pharmaceutically acceptable salt thereof according to any one of embodiments 1-37, which is a compound represented by formula (Ib) or a pharmaceutically acceptable salt thereof:

   R₁-X₁-X₂-X₃-Gly-Thr-Phe-Thr-Ser-Asp-X₁₀-Ser-X₁₂-X₁₃-X₁₄-Glu-Y₁-X₁₇-X₁₈-X₁₉-X₂₀-Glu-Phe-Val-Glu-Trp-Leu-X₂₇-X₂₈-X₂₉-Gly-Pro-Ser-Ser-Gly-Ala-Pro-Pro-Pro-Ser-R₂ (SEQ ID NO: 150) (Ib);

   wherein R₁ is hydrogen, alkyl, acetyl, formyl, benzoyl, trifluoroacetyl, or pGlu, and R₂ is -NH₂ or -OH;
   X₁ is His,
   X₂ is Aib,
   X₃ is His,
   X₁₀ is selected from an amino acid residue of Leu or Tyr,
   X₁₂ is selected from an amino acid residue of Lys, alpha-methyl-Lys, or Ile,
   X₁₃ is selected from an amino acid residue of Ile, Leu or Tyr,
   X₁₄ is selected from an amino acid residue of Leu,
   X₁₇ is selected from an amino acid residue of Ile, Gln or alpha-methyl-Lys,
   X₁₈ is selected from an amino acid residue of Ala,
   X₁₉ is selected from an amino acid residue of Ala,
   X₂₀ is selected from an amino acid residue of Gln, isovaline, 1-aminocyclopropane-1-carboxylic acid or 1-aminocyclobutane-1-carboxylic acid,
   X₂₇ is selected from an amino acid residue of Leu,
   X₂₈ is selected from an amino acid residue of Ala,
   X₂₉ is selected from an amino acid residue of Gly or D-Glu;
   Y₁ is a Lys, Orn, Dap, Dab, or Cys residue with the side chain linked to a substituent.
64. The compound or the pharmaceutically acceptable salt thereof according to embodiment 63, wherein:
   X₁ is His,
   X₂ is Aib,
   X₃ is His,
   X₁₀ is selected from an amino acid residue of Leu or Tyr,
   X₁₂ is selected from an amino acid residue of alpha-methyl-Lys or Ile,
   X₁₃ is selected from an amino acid residue of Ile or Leu,
   X₁₄ is selected from an amino acid residue of Leu,
   X₁₇ is selected from an amino acid residue of Gln,
   X₁₈ is selected from an amino acid residue of Ala,
   X₁₉ is selected from an amino acid residue of Ala,
   X₂₀ is selected from an amino acid residue of Gln, isovaline, 1-aminocyclopropane-1-carboxylic acid or 1-aminocyclobutane-1-carboxylic acid,
   X₂₇ is selected from an amino acid residue of Leu,
   X₂₈ is selected from an amino acid residue of Ala,
   X₂₉ is selected from an amino acid residue of Gly or D-Glu.
65. The compound or the pharmaceutically acceptable salt thereof according to embodiment 63, wherein:
   X₁ is His,
   X₂ is Aib,
   X₃ is His,
   X₁₀ is selected from an amino acid residue of Tyr,
   X₁₂ is selected from an amino acid residue of Lys or alpha-methyl-Lys,
   X₁₃ is selected from an amino acid residue of Leu or Tyr,
   X₁₄ is selected from an amino acid residue of Leu,
   X₁₇ is selected from an amino acid residue of Ile or alpha-methyl-Lys,
   X₁₈ is selected from an amino acid residue of Ala,
   X₁₉ is selected from an amino acid residue of Ala,
   X₂₀ is selected from an amino acid residue of Gln,
   X₂₇ is selected from an amino acid residue of Leu,
   X₂₈ is selected from an amino acid residue of Ala,
   X₂₉ is selected from an amino acid residue of Gly.
66. The compound or the pharmaceutically acceptable salt thereof according to any one of embodiments 63-65, wherein: X₂₀ is selected from an amino acid residue of Gln.
67. The compound or the pharmaceutically acceptable salt thereof according to any one of embodiments 63-64, wherein: X₂₀ is selected from an amino acid residue of isovaline, 1-aminocyclopropane-1-carboxylic acid, or 1-aminocyclobutane-1-carboxylic acid.
68. The compound or the pharmaceutically acceptable salt thereof according to any one of embodiments 1-74, wherein Y₁ is a Lys residue with the side chain linked to a substituent, the substituent being covalently linked to the ε-amino group of the Lys residue via an amide bond.
69. The compound or the pharmaceutically acceptable salt thereof according to any one of embodiments 1-68, wherein: the structure of the substituent is shown below:
   {[2-(2-amino-ethoxy)-ethoxy]-acetyl}ₐ-(γGlu)_{b}-CO-(CH₂)_{c}-COOH;
   wherein a is any integer between 0 and 4, b is any integer between 1 and 2, and c is any integer between 10 and 30.
70. The compound or the pharmaceutically acceptable salt thereof according to embodiment 69, wherein: a is 1, 2 or 3, b is 1 or 2, and c is 16-20.
71. The compound or the pharmaceutically acceptable salt thereof according to embodiment 69, wherein: a is 2 or 3, b is 1 or 2, and c is 16-20.
72. The compound or the pharmaceutically acceptable salt thereof according to embodiment 69, wherein: a is 2 or 3, b is 1 or 2, and c is 16, 18 or 20.
73. The compound or the pharmaceutically acceptable salt thereof according to embodiment 69, wherein: a is 2, b is 1 or 2, and c is 16, 18 or 20.
74. The compound or the pharmaceutically acceptable salt thereof according to embodiment 69, wherein: a is 2, b is 1 or 2, and c is 16 or 18.
75. The compound or the pharmaceutically acceptable salt thereof according to embodiment 69, wherein: a is 2, b is 1, and c is 16 or 18.
76. The compound or the pharmaceutically acceptable salt thereof according to any one of embodiments 1-75, wherein:
   Y₁ is K(-OEG-OEG-γGlu-C(O)-(CH₂)₁₆-COOH) or K(-OEG-OEG-γGlu-C(O)- (CH₂)₁₃-COOH), the group having the structure shown below: 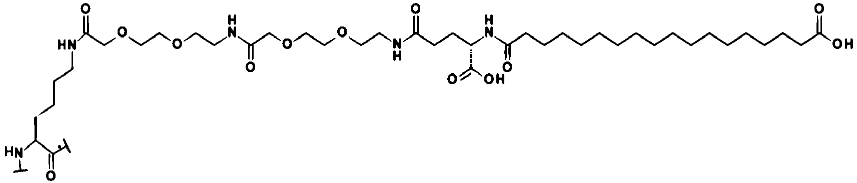 or 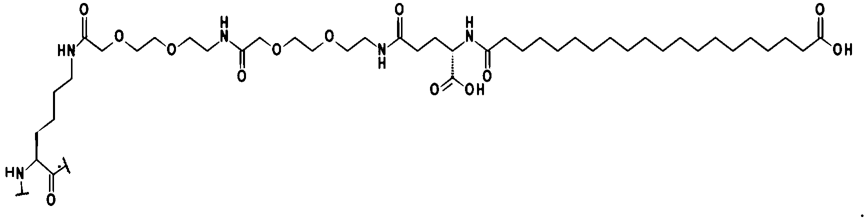
77. The compound or the pharmaceutically acceptable salt thereof according to any one of embodiments 1-76, wherein Y₁ is 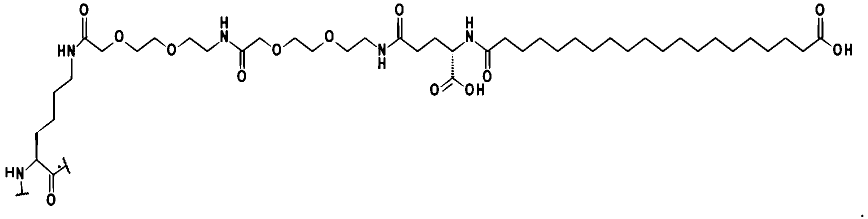
78. The compound or the pharmaceutically acceptable salt thereof according to any one of embodiments 1-77, wherein R₁ is hydrogen.
79. The compound or the pharmaceutically acceptable salt thereof according to any one of embodiments 1-78, wherein R₂ is -NH₂ or -OH.
80. The compound or the pharmaceutically acceptable salt thereof according to embodiment 79, wherein R₂ is - NH₂.
81. The compound or the pharmaceutically acceptable salt thereof according to any one of embodiments 1-80, wherein the compound is selected from any of Compound 1 to Compound 48.
82. The compound or the pharmaceutically acceptable salt thereof according to any one of embodiments 1-81, wherein the compound has a triple agonistic activity against GLP-1 receptor, GIP receptor and GCG receptor.
83. The compound or the pharmaceutically acceptable salt thereof according to any one of embodiments 1-81, wherein the compound has an extended half-life.
84. The compound or the pharmaceutically acceptable salt thereof according to any one of embodiments 1-81, wherein the compound has improved solubility, physical stability and/or chemical stability.
85. The compound according to any one of embodiments 1-84, or a pharmaceutically acceptable salt or solvate thereof.
86. A GLP-1/GIP/GCG receptor triagonist, comprising the compound or the pharmaceutically acceptable salt thereof of any one of embodiments 1-84.

### BRIEF DESCRIPTION OF THE DRAWINGS

FIG. 1 shows the body weight change (%) at different time points (days) in diet-induced obesity (DIO) mice after subcutaneous injection of the compounds.
FIG. 2 shows the changes in body weight (g) over time in diet-induced obese (DIO) mice after subcutaneous injection of the compounds according to the present disclosure.
FIG. 3 shows the changes in body weight (g) over time in diet-induced obese (DIO) rats after subcutaneous injection of the compounds.
FIG. 4 shows the body weight change (%) at different time points (days) in diet-induced obesity (DIO) rats after subcutaneous injection of the compounds.
FIG. 5 shows a graph illustrating the change in daily food intake over time in diet-induced obese (DIO) rats after subcutaneous injection of the compounds.
FIG. 6 shows a graph illustrating the change in cumulative food intake over time in diet-induced obese (DIO) rats after subcutaneous injection of the compounds.

### DETAILED DESCRIPTION OF THE INVENTION

Unless otherwise stated, the terms used in the specification and claims have the following meanings.

In the context of the present disclosure, Greek letters may be represented by their symbols or corresponding written names, for example: α = alpha; β = beta; ε = epsilon; γ = gamma; ω = omega; etc.

This article uses the term "compound" to refer to molecular entities. Therefore, a "compound" may have different structural elements in addition to having a minimum element defined for each compound or group of compounds. Therefore, the compound can be a peptide or its derivative, as long as the compound contains the defined structural and/or functional elements.

The term "polypeptide" refers to a sequence of two or more amino acids. "Polypeptide" may also include amino acid elongation at the N-terminal and/or C-terminal positions and/or truncation at the N-terminal and/or C-terminal positions. Typically, amino acid residues can be represented by their full name, their single-letter code, and/or their three-letter code. These three methods are completely equivalent.

In the context of the present disclosure, the terms "polypeptide," "compound," and "polypeptide compound" are used interchangeably.

Amino acids are molecules that contain amino and carboxylic acid groups and optionally one or more additional groups, often referred to as side chains.

The term "amino acid" includes proteinogenic (or natural) amino acids (of which 20 are standard amino acids) as well as non-proteinogenic (or unnatural) amino acids. Proteinogenic amino acids are amino acids that are naturally incorporated into proteins. Standard amino acids are amino acids encoded by the genetic code. Non-proteinogenic amino acids either do not exist in proteins or are not produced through standard cellular mechanisms (e.g., they may have undergone post-translational modifications). Non-limiting examples of non-proteinogenic amino acids are Aib (α-aminoisobutyric acid or 2-aminoisobutyric acid), ortholeucine (Nle), orthovaline, and D-isomers of proteinogenic amino acids. D-Ala is D-alanine, -αL- is alpha-methylated leucine, αS is alpha-methylated serine, and αK is alpha-methylated lysine, etc.

"Natural amino acids" refer to 20 common amino acids, namely, alanine (A), cysteine (C), aspartic acid (D), glutamic acid (E), phenylalanine (F), glycine (G), histidine (H), isoleucine (I), lysine (K), leucine (L), methionine (M), asparagine (N), proline (P), glutamine (Q), arginine (R), serine (S), threonine (T), valine (V), tryptophan (W) and tyrosine (Y).

"Unnatural amino acid" refers to an amino acid that is not naturally encoded or found in the genetic code of any organism. They may be, for example, purely synthetic compounds. Examples of unnatural amino acids include, but are not limited to, hydroxyproline, γ-carboxyglutamic acid, O-phosphoserine, azetidine carboxylic acid, 2-aminoadipic acid, 3-aminoadipic acid, β-alanine, aminopropionic acid, 2-aminobutyric acid, 4-aminobutyric acid, 6-aminohexanoic acid, 2-aminoheptanoic acid, 2-aminoisobutyric acid, 3-aminoisobutyric acid, 2-aminopimelic acid, tert-butylglycine, 2,4-diaminoisobutyric acid (Dap), desmosine, 2,2'-diaminopimelic acid, 2,3-diaminopropionic acid (Dab), N-ethylglycine, N-methylglycine, N-ethylasparagine, homoproline, hydroxylysine, allo-hydroxylysine, 3-hydroxyproline, 4-hydroxyproline, isodesmosine, alloisoleucine, N-methylalanine, N-methylglycine, N-methylisoleucine, N-methylpentylglycine, N-methylvaline, naphthalanine, norvaline, norleucine, ornithine (Orn), D-ornithine, D-arginine, p-aminophenylalanine, pentylglycine, pipecolic acid and thioproline. In addition, it also includes natural amino acids or unnatural amino acids with the C-terminal carboxyl group, N-terminal amino group and/or side chain functional groups being chemically modified.

The term "agonist" is defined as a substance that activates the receptor type in question. For example, the term "GLP-1/GIP/GCG triagonist" as used in the context of the present disclosure refers to a substance or ligand that can activate GLP-1 receptor, GIP receptor and GCG receptor.

The term "native GLP-1" refers to a peptide comprising the human GLP-1 (7-36 or 7-37) sequence, the term "native GIP" refers to a peptide comprising the human GIP (1-42) sequence, and the term "native GCG" refers to a peptide comprising the human GCG (1-29) sequence. Unless otherwise specified, the terms "GLP-1", "GIP", or "GCG" refer to native GLP-1, native GIP, or native GCG, respectively.

The term "alkyl" refers to a saturated aliphatic hydrocarbon group, which is a straight or branched group containing 1 to 20 carbon atoms, preferably, an alkyl group containing 1 to 8 carbon atoms, more preferably, an alkyl group containing 1 to 6 carbon atoms, most preferably, an alkyl group containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, and the like. More preferred is a lower alkyl group containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl group may be substituted or unsubstituted. When it is substituted, the substitution with a substituent may be performed at any available attachment point. The substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkyloxy, heterocycloalkyloxy, cycloalkylthio, heterocycloalkylthio, oxo, carboxy or a carboxylate group. Methyl, ethyl, isopropyl, tert-butyl, haloalkyl, deuterated alkyl, alkoxy-substituted alkyl and hydroxy-substituted alkyl are preferred in the present invention.

"Optional" or "optionally" means that the subsequently described event or circumstance may, but not necessarily, occur, and that the description includes instances where the event or circumstance occurs and does not occur. For example, "a heterocyclic group optionally substituted with an alkyl group" means that the alkyl group may be, but not necessarily, present, and the description includes both the case where the heterocyclic group is substituted with the alkyl group and the case where the heterocyclic group is not substituted with the alkyl group.

"Substituted" means that one or more hydrogen atoms, preferably up to 5, more preferably 1 to 3 hydrogen atoms in a group are independently of each other replaced by a corresponding number of substituents. It is understood that the substituents are only located in their possible chemical positions and those skilled in the art can determine (either experimentally or theoretically) which substitutions are possible or impossible without undue effort. For example, it may be unstable when an amino or hydroxyl group having a free hydrogen is bound to a carbon atom having an unsaturated (e.g., olefinic) bond.

A "pharmaceutical composition" means a mixture containing one or more compounds described herein or their physiologically/pharmaceutically acceptable salts or prodrugs together with other chemical components, as well as other components such as physiologically/pharmaceutically acceptable carriers and excipients. The purpose of the pharmaceutical composition is to promote administration to an organism, which facilitates the absorption of the active ingredients, thereby exerting biological activities.

"Pharmaceutically acceptable salts" refer to salts of the compound of the present disclosure, which are safe and effective for use in mammals and have the desired biological activity.

When applied to animals, humans, experimental subjects, cells, tissues, organs, or biological fluids, "giving," "administering," and "treating" refer to the contact between an exogenous drug, therapeutic agent, diagnostic agent, or composition and the animal, human, subject, cell, tissue, organ, or biological fluid, for example in treatment, pharmacokinetics, diagnosis, research, and experimental methods. Cell processing includes contact between a reagent and a cell, and contact between a reagent and a fluid, wherein the fluid is in contact with the cell. "Giving," "administering," and "treating" also refer to in vitro and ex vivo processing of cells, such as by means of reagents, diagnostics, binding compositions, or another cell type. When applied to human, veterinary, or research subjects, they refer to therapeutic treatment or prevention, or preventative measures, and research and diagnostic applications.

"Treatment" means administering a therapeutic agent for an internal or external use, for example, a therapeutic agent comprising any of the binding proteins of the present disclosure or a pharmaceutical composition thereof, to a subject who has, is suspected of having, or is predisposed to having one or more metabolic-related diseases or symptoms thereof, and the therapeutic agent is known to have a therapeutic effect on these symptoms. Typically, a therapeutic agent is administered to a subject or population being treated in an amount that is effective in alleviating the symptoms of one or more diseases, whether by inducing the regression of such symptoms or inhibiting the development of such symptoms to any clinically measurable degree. The amount of a therapeutic agent that is effective in alleviating the symptoms of any specific disease (also referred to as the "therapeutically effective amount") can vary depending on a variety of factors, such as the disease state, age and body weight of a subject, and the ability of a drug to produce the desired therapeutic effect in the subject. Whether the symptoms of a disease have been relieved can be assessed using any clinical test that a doctor or other healthcare professional typically uses to assess the severity or progression of the symptoms. Although the embodiments of the present disclosure (e.g., treatment methods or products) may be ineffective in alleviating the symptoms of the target disease in a particular subject, they should reduce the symptoms of the target disease in a statistically significant number of subjects, as determined by any statistical test known in the art, such as the Student t-test, chi-square test, U-test according to Mann and Whitney, Kruskal-Wallis test (H-test), Jonckheere-Terpstra test, and Wilcoxon test.

An "effective amount" refers to an amount sufficient to improve or prevent the symptoms or condition of a medical condition. An effective amount also refers to an amount sufficient to allow or facilitate a diagnosis. An effective amount for a subject can vary depending on factors such as the condition to be treated, the overall health of the subject, the route and dosage of administration, and the severity of side effects. An effective amount can be the maximum dose or dosing regimen that avoids significant side effects or toxicity. Subjects in the present disclosure may be animal or human subjects.

"Pharmaceutically acceptable carriers, diluents, or excipients" include any material that, when combined with an active ingredient, allows the ingredient to retain its biological activity and does not serve the intended therapeutic purpose. Examples include, but are not limited to, any standard pharmaceutical carriers, such as phosphate-buffered saline solutions, water, emulsions such as oil/water emulsions, and various types of wetting agents. In some embodiments, a diluent used for aerosol or parenteral administration is phosphate-buffered saline (PBS) or physiological (0.9%) saline. Compositions comprising such carriers are formulated using well-known conventional methods (see, for example, Remington's Pharmaceutical Sciences, version 18, edited by A. Gennaro, Mack Publishing Co., Easton, PA, 1990; and R Remington, The Science and Practice of Pharmacy, version 20, Mack Publishing, 2000).

"Optional" or "optionally" means that the subsequently described event or circumstance may, but not necessarily, occur, and that the description includes instances where the event or circumstance occurs and does not occur. "And/or" should be construed as specifically disclosing each of the two specified features or components with or without the other. Therefore, the term "and/or" as used in phrases such as "A and/or B" in the present disclosure includes "A and B", "A or B", "A" (alone) and "B" (alone). Unless the context clearly requires otherwise, throughout the specification and claims, the words "comprising," "having," "including," etc., should be understood as having an inclusive meaning, rather than an exclusive or exhaustive meaning; that is, the meaning of "including but not limited to."

The "subject" or "patient" in the present disclosure can be a mammal, including humans or non-human mammals such as non-human primates (e.g., apes, Old World monkeys, or New World monkeys), livestock (e.g., cattle or pigs), companion animals (e.g., dogs or cats), or laboratory animals such as rodents (e.g., mice or rats).

### DETAILED DESCRIPTION OF THE EMBODIMENTS

In order to illustrate the present invention in more detail, the following specific embodiments are provided in the specification, but the embodiments of the present invention are not limited thereto.

### Example 1. Preparation of target compound

### 1. Experimental reagents

**Table 4**

| Serial No. | Reagents | Source |
|---|---|---|
| 1 | Rink-amide MBHA resin | Xi'an Sunresin New Materials |
| 2 | HATU | Suzhou Highfine Biotech |
| 3 | Fmoc-Aib-OH | GL Biochem |
| 4 | Fmoc-L-Lys(Mtt)-OH | GL Biochem |
| 5 | N,N-Dimethylformamide | Sinopharm Chemical Reagent |
| 6 | Dichloromethane | Sinopharm Chemical Reagent |
| 7 | Trifluoroacetic acid | TCI Chemicals |
| 8 | Triisopropylsilane | TCI Chemicals |
| 9 | Hexafluoroisopropanol | TCI Chemicals |
| 10 | Acetonitrile | Merck-Millipore |
| 11 | Diisopropylethylamine | TCI Chemicals |
| 12 | 4-Methylpiperidine | TCI Chemicals |
| 13 | Methyl tert-butyl ether | TCI Chemicals |
| 14 | Fmoc-L-alpha-Methyl-Lys(Boc)-OH | GL Biochem |
| 15 | Fmoc-L-alpha-Methyl-Leu-OH | GL Biochem |
| 16 | Boc-L-Tyr(tBu)-OH | GL Biochem |
| 17 | 4-Methylmorpholine | TCI Chemicals |
| 18 | Fmoc-L-alpha-methyl-Ser(tBu)-OH | GL Biochem |
| 19 | Fmoc-L-Glu-OtBu | GL Biochem |
| 20 | Fmoc-AEEA-OH | GL Biochem |
| 21 | Boc-L-His(Trt)-OH | GL Biochem |
| 22 | Eicosanedioic acid monotert-butyl ester | QYAOBIO |
| 23 | Fmoc-L-alpha-methyl-Tyr(tBu)-OH | GL Biochem |
| 24 | Fmoc-Ac3c-OH | GL Biochem |
| 25 | Fmoc-Ac4c-OH | GL Biochem |

### 2. Experimental instruments

**Table 5**

| Serial No. | Instrument | Source |
|---|---|---|
| 1 | H-CLASS Analytical Ultra Performance Liquid Chromatography | WATERS |
| 2 | Angilent Q-TOF 6530 LC-MS | Angilent |
| 3 | Labconco Multifunctional Freeze Dryer | Thermo-Fisher Scientific |
| 4 | Prep150 Preparative High Performance Liquid Chromatography | WATERS |
| 5 | Multi-channel High-speed Centrifuge | Sigma |
| 6 | Prelude-X Automatic Polypeptide Synthesizer | Gyros Protein Technologies |

### 3.1 Chemical synthesis of Compound No. 0100

The chemical synthesis of Compound 0100 was accomplished on the Prelude-X automatic polypeptide synthesizer using the fluorenylmethoxycarbonyl (Fmoc)/tert-butyl (t-Bu) synthesis method. The resin used was Rink-amide MBHA resin with a degree of substitution of 0.54 mmole/g. Fmoc-L-Lys(Mtt)-OH was used as the amino acid residue for the fatty acid modification site and Boc-L-His(Trt)-OH was used as the N-terminal amino acid. Before the amino acid condensation step, the Fmoc group was removed using a DMF solution containing 20% 4-methylpiperidine (reaction twice, 8 min each time). All standard amino acid condensations were performed using equimolar Fmoc amino acids, HATU, and 2 equivalents of 4-methylmorpholine, in a 10-fold excess relative to the theoretical peptide loading, at room temperature for 25 min. The exception is coupling to the Cα-methylated amino acid, which requires two or three condensations, each lasting 60 min, to ensure complete condensation. After the extension of the above peptide-resin was completed, the resin peptide was washed with dichloromethane, and then a mixed solution of hexafluoroisopropanol/dichloromethane (30% hexafluoroisopropanol, 10 mL) was added, and the mixture was shaken for reaction at room temperature for 45 min, and then the mixed solution was removed. Then, a mixed solution of hexafluoroisopropanol/dichloromethane (30%, 10 mL) was added, and the mixture was shaken for reaction at room temperature for 45 min, and then the mixed solution was removed. After the reaction was completed, the resin was washed with DMF for three times. Additional coupling/deprotection cycles to extend the lysine side chain on the same polypeptide synthesizer using the Fmoc/tBu solid-phase synthesis strategy involved Fmoc-AEEA-OH (twice), Fmoc-L-Glu-OtBu (once), and eicosanedioic acid monotert-butyl ester (once). Condensations were performed using equimolar Fmoc amino acids, HATU, and 2 equivalents of 4-methylmorpholine, in a 10-fold excess relative to the theoretical peptide loading, at room temperature for 25 min. The exception is the coupling of eicosanedioic acid monotert-butyl ester, which requires condensation at room temperature for at least three hours to ensure complete condensation.

After synthesis, the resin peptide obtained was washed three times with DMF and DCM in sequence and then dried in vacuo. 10 mL of a freshly prepared cleavage solution (trifluoroacetic acid: triisopropylsilane: water = 90:5:5, v:v:v) was added and the mixture was shaken for reaction at room temperature for three to four hours. After the reaction was completed, the mixture was filtered, and the resin was washed twice with trifluoroacetic acid. After the filtrates were combined, a large amount of methyl tert-butyl ether was added to precipitate the crude peptide as a solid. After the supernatant was removed by centrifugation, a crude peptide of Compound No. 0100 was obtained. The crude peptide was dissolved in a mixed solvent containing 20% acetic acid/water, and the solution was filtered through a 0.22 um membrane. Then, separation was performed using a WATERS Prep150 LC reversed-phase high-performance liquid chromatography system with mobile phases A (0.1% trifluoroacetic acid, 10% acetonitrile, in water) and B (0.1% trifluoroacetic acid, 90% acetonitrile, in water). The chromatographic column was an X-SELECT OBD C-18 (WATERS) reversed-phase chromatographic column, and during the purification process, the detection wavelength of the chromatograph was set to 220 nm and the flow rate was 15-20 mL/min. The related fractions of the product were collected and lyophilized to obtain a pure polypeptide of Compound No. 0100 with a yield of about 16%. The purity of the pure polypeptide, i.e., the identity of the compound were determined by analytical ultra-high performance liquid chromatography and liquid chromatography/mass spectrometry. The purity was 94.83% and the measured molecular weight of the compound was 4934.5.

### 3.2 Chemical synthesis of the remaining compounds in the present disclosure

The remaining compounds were synthesized using the experimental scheme of Compound 0100, where N-terminal amino acid can be either Boc-L-Tyr(tBu)-OH or Boc-L-His(Trt)-OH, depending on the sequence, and the purity and molecular weight of the compounds were determined by analytical ultra-high performance liquid chromatography and liquid chromatography/mass spectrometry, as shown in Table 6 below:

**Table 6. Purity and molecular weight of compounds**

| Compound No. | Measured Molecular Weight | Purity |
|---|---|---|
| 0104 | 4933.5 | 97.18% |
| 0106 | 4933.5 | 96.91% |
| 0107 | 4840.5 | 96.06% |
| 0108 | 4933.5 | 96.82% |
| 0109 | 4933.6 | 97.75% |
| 0110 | 4933.5 | 96.69% |
| 0111 | 4933.6 | 97.66% |
| 0112 | 4883.6 | 93.14% |
| 0113 | 4990.6 | 94.25% |
| 0114 | 4900.6 | 99.50% |
| 0115 | 4991.6 | 95.98% |
| 0117 | 4883.5 | 97.07% |
| 0118 | 4868.5 | 93.00% |
| 0119 | 4883.5 | 96.89% |
| 0120 | 4854.2 | 98.03% |
| 0125 | 4868.7 | 95.58% |
| 0126 | 4914.7 | 99.27% |
| 0127 | 4870.6 | 93.21% |
| 0128 | 4853.6 | 99.50% |
| 0129 | 4870.6 | 86.65% |
| 0130 | 4853.6 | 93.33% |
| 0131 | 4912.6 | 87.55% |
| 0132 | 4912.6 | 92.10% |
| 0133 | 4862.6 | 97.95% |
| 0134 | 4861.6 | 97.21% |
| 0135 | 4824.6 | 92.44% |
| 0136 | 4774.6 | 98.08% |
| 0137 | 4838.6 | 99.02% |
| 0138 | 4788.6 | 99.09% |
| 0139 | 4940.6 | 86.75% |
| 0140 | 4786.6 | 95.39% |
| 0142 | 4889.6 | 95.29% |
| 0143 | 4817.6 | 94.97% |
| 0144 | 4875.6 | 91.68% |
| 0145 | 4844.6 | 94.81% |
| 0146 | 4830.6 | 90.58% |
| 0147 | 4844.6 | 88.49% |
| 0148 | 4830.5 | 93.74% |
| 0149 | 4880.6 | 92.76% |
| 0150 | 4894.5 | 91.27% |
| 0151 | 4926.6 | 95.15% |
| 0152 | 4853.5 | 93.06% |
| 0153 | 4925.5 | 88.34% |
| 0154 | 4844.5 | 96.53% |
| 0155 | 4858.6 | 92.72% |
| 0156 | 4801.5 | 96.82% |
| 0157 | 4815.6 | 93.26% |

### Example 2. Determination of agonist activity of compounds on human glucagon-like peptide-1 receptor (GLP-1R), human glucose-dependent insulinotropic polypeptide receptor (GIPR), and human glucagon receptor (GCGR)

This example aims to determine the agonist activity of the test compounds on human glucagon-like peptide-1 receptor, human glucose-dependent insulinotropic polypeptide receptor, and human glucagon receptor.

### 2.1. Experimental methods:

Functional activity was determined in CHO-K1 clonal cell lines expressing GIPR, GLP-1R, and GCGR. In a 10 µL assay volume, the polypeptide was diluted (8-10 points, 10-fold dilution) in DMEM/F12 1:1 (HyClone, SH30023.01) supplemented with 5% FBS (fetal bovine serum, Gibco, 10099141), 0.1% Casein (Sigma, C4765), 0.25 mM IBMX (Selleck, S5836). Each recipient cell (5000 cells/well) was added to an SV 96-well plate (Cisbio, 66PL96025) and incubated with the prepared polypeptide at 37°Cfor 30 min. The resulting increase in intracellular cAMP was quantitatively measured using the cAMP-GS dynamic HTRF assay kit (Cisbio, 62AM4PEJ). Briefly, the intracellular cAMP levels were determined by adding cAMP-d2 conjugate in cell lysis buffer, followed by the antibody anti-cAMP-Eu cryptate (also in cell lysis buffer). The resulting competitive assay was incubated at room temperature for at least 60 min and then determination was performed using Tecan Infinite F Plex instrument with excitation at 320 nm and emission at 665 nm and 620 nm. The measured data (emission at 665 nm/620 nm * 10000) were inversely proportional to the amount of cAMP present and converted to the cAMP content per well using the cAMP standard curve.

The amount of cAMP generated in each well was converted to the percentage of the maximum response observed with human GLP-1(7-36)-NH₂, human GCG (1-29), or human GIP(1-42)-NH₂ (purchased from Sigma). Using the maximum response percentage vs. the added polypeptide concentration, the relative EC₅₀ values were derived through nonlinear regression analysis and fitted to a four-parameter logarithmic equation. For specific data, refer to Tables 7-1 to 7-8 below. For the LY3437943 molecule, see Example 12 of WO2019/125938, which is incorporated herein by reference in its entirety.

**Table 7-1**

| Compound | Activity against human GLP-1R (EC₅₀ nM) | Activity against human GIPR (EC₅₀ nM) | Activity against human GCGR (EC₅₀ nM) |
|---|---|---|---|
| Native GLP-1 | 0.003 | N/A | N/A |
| Native GIP | N/A | 0.001 | N/A |
| Native GCG | N/A | N/A | 0.010 |
| 0104 | 0.010 | 0.016 | 0.037 |
| 0106 | 0.030 | 0.027 | 0.095 |
| 0107 | 0.016 | 0.014 | 0.274 |
| 0108 | 0.014 | 0.078 | 0.315 |
| 0109 | 0.011 | 0.027 | 0.031 |
| 0110 | 0.016 | 0.043 | 0.044 |

**Table 7-2**

| | | | |
|---|---|---|---|
| Native GLP-1 | 0.005 | N/A | N/A |
| Native GIP | N/A | 0.0003 | N/A |
| Native GCG | N/A | N/A | 0.087 |
| LY3437943 | 0.080 | 0.002 | 0.412 |
| 0111 | 0.015 | 0.024 | 0.552 |
| 0112 | 0.031 | 0.005 | 0.087 |
| 0113 | 0.032 | 0.015 | 5.911 |
| 0114 | 0.178 | 0.010 | 0.330 |
| 0115 | 0.023 | 0.051 | 0.425 |
| 0117 | 0.059 | 0.003 | 0.125 |
| 0118 | 0.073 | 0.004 | 0.132 |
| 0119 | 0.023 | 0.005 | 0.087 |
| 0120 | 0.026 | 0.002 | 0.121 |

**Table 7-3**

| | | | |
|---|---|---|---|
| Native GLP-1 | 0.002 | N/A | N/A |
| Native GIP | N/A | 0.0003 | N/A |
| Native GCG | N/A | N/A | 0.009 |
| 0125 | 0.032 | 0.002 | 0.071 |
| 0126 | 0.008 | 0.002 | 0.008 |

**Table 7-4**

| | | | |
|---|---|---|---|
| Native GLP-1 | 0.006 | N/A | N/A |
| Native GIP | N/A | 0.0001 | N/A |
| Native GCG | N/A | N/A | 0.014 |
| 0127 | 0.132 | 0.0007 | 0.037 |
| 0128 | 0.040 | 0.0005 | 0.031 |
| 0129 | 0.159 | 0.002 | 0.067 |
| 0130 | 0.048 | 0.0005 | 0.030 |
| 0131 | 0.020 | 0.0007 | 0.026 |
| 0132 | 0.024 | 0.001 | 0.030 |

**Table 7-5**

| | | | |
|---|---|---|---|
| Native GLP-1 | 0.002 | N/A | N/A |
| Native GIP | N/A | 0.0006 | N/A |
| Native GCG | N/A | N/A | 0.040 |
| 0133 | 0.016 | 0.002 | 0.030 |
| 0134 | 0.017 | 0.002 | 0.025 |
| 0135 | 0.013 | 0.001 | 0.019 |
| 0136 | 0.014 | 0.001 | 0.039 |
| 0137 | 0.013 | 0.001 | 0.047 |
| 0138 | 0.019 | 0.0006 | 0.055 |

**Table 7-6**

| | | | |
|---|---|---|---|
| Native GLP-1 | 0.009 | N/A | N/A |
| Native GIP | N/A | 0.002 | N/A |
| Native GCG | N/A | N/A | 0.032 |
| 0139 | 0.043 | 0.007 | 0.430 |
| 0142 | 0.058 | 0.007 | 0.490 |
| 0143 | 0.090 | 0.004 | 0.423 |
| 0144 | 0.035 | 0.012 | 0.311 |

**Table 7-7**

| | | | |
|---|---|---|---|
| Native GLP-1 | 0.006 | N/A | N/A |
| Native GIP | N/A | 0.001 | N/A |
| Native GCG | N/A | N/A | 0.014 |
| 0145 | 0.057 | 0.003 | 0.143 |
| 0146 | 0.033 | 0.009 | 0.146 |
| 0147 | 0.035 | 0.005 | 0.041 |
| 0148 | 0.060 | 0.006 | 0.549 |
| 0149 | 0.071 | 0.004 | 0.301 |
| 0150 | 0.076 | 0.002 | 0.076 |
| 0151 | 0.037 | 0.004 | 0.055 |

**Table 7-8**

| | | | |
|---|---|---|---|
| Native GLP-1 | 0.005 | N/A | N/A |
| Native GIP | N/A | 0.005 | N/A |
| Native GCG | N/A | N/A | 0.019 |
| 0152 | 0.054 | 0.009 | 0.290 |
| 0153 | 0.051 | 0.010 | 0.334 |
| 0154 | 0.033 | 0.031 | 1.621 |
| 0155 | 0.036 | 0.014 | 3.655 |
| 0156 | 0.015 | 0.019 | 0.566 |
| 0157 | 0.017 | 0.008 | 1.333 |

The results above show that the polypeptide molecules provided in the present disclosure have good agonistic activity against GLP-1R, GIPR and GCGR.

### Example 3. Determination of pharmacokinetic characteristics of compounds in mice in vivo

Male C57 mice were used as test animals. The target compound was administered via a single intravenous injection, and the pharmacokinetic characteristics of the compound in C57 mice in vivo (plasma) were determined.

### 3.1 Experimental methods:

Male C57 mice, weighing approximately 25g, aged 4-6 weeks. After preparing the polypeptide compound solution using 1×PBS dissolving solution, the compound was administered intravenously at a dose of 200 nmol/kg, where each compound was administered to 3 animals. Blood samples of 60 µL were collected at 5 min, 15 min, 0.5 h, 1 h, 2 h, 4 h, 6 h, 24 h, 48 h and 72 h after the infusion was completed and transferred to centrifuge tubes containing EDTA-K2 anticoagulant. Whole blood samples were centrifuged at 4000 g for 5 min at 4°C. Plasma was separated and plasma samples were frozen and stored at -80 °C.

Sample pretreatment method: 30 µL of plasma samples was taken, and 120 µL of 0.1% formic acid in methanol containing 1 ng/mL internal standard verapamil was added. The mixture was vortexed for 5 min, and centrifuged at 10000 rpm for 10 min at low temperature. 70 µL of supernatant was taken, and 70 µL of water was added and mixed well. Analysis was performed by LC-MS instrument.

LC-MS analysis method: (1) chromatographic conditions: mobile phases A and B: 0.1% formic acid in water and 0.1% formic acid in acetonitrile, respectively; gradient elution mode; flow rate: 0.5 mL/min; injection volume: 10 µL; chromatographic column: nanomicro Unisil C18aq (4.6 mm × 150 mm, 5 µm); column temperature: 40°C. (2) Mass spectrometry conditions: For mass spectrometry, an electrospray ionization source (ESI), positive ion analysis mode, and multiple reaction monitoring (MRM) scanning mode were used.

For data processing, SCIEX OS (Version 2.1.6) software was used to quantify blood drug concentration data and PKSolver (Version 2.0) software was used to calculate pharmacokinetic parameters.

### 3.2. Experimental results:

**Table 8. Comparison of pharmacokinetic parameters for compounds (200 nmol/kg) administered to mice**

| **Compound No.** | **AUC(inf) (h·nmol/L)** | **C0 (nmol/L)** | **CLp (mL/min/kg)** | **T1/2 (h)** |
|---|---|---|---|---|
| 0119 | 55886 | 6166 | 0.058 | 13.0 |
| 0120 | 94062 | 9928 | 0.034 | 14.7 |
| 0121 | 20343 | 3415 | 0.163 | 6.8 |
| 0104 | 30935 | 4217 | 0.108 | 11.5 |
| 0123 | 67881 | 5142 | 0.049 | 7.9 |
| 0125 | 96054 | 5788 | 0.035 | 14.6 |
| 0126 | 102555 | 7167 | 0.033 | 10.1 |
| 0131 | 22484 | 5978 | 0.148 | 10.2 |
| 0137 | 20469 | 5023 | 0.163 | 8.8 |
| 0138 | 18465 | 3277 | 0.181 | 8.2 |
| 0140 | 28880 | 5041 | 0.115 | 9.3 |
| 0141 | 22598 | 4889 | 0.148 | 7.5 |
| 0145 | 25767 | 8800 | 0.129 | 7.7 |
| 0139 | 41636 | 4732 | 0.081 | 11.9 |
| 0142 | 36284 | 5595 | 0.092 | 10.5 |
| 0143 | 70359 | 7554 | 0.047 | 13.7 |
| 0144 | 29304 | 4013 | 0.114 | 9.7 |

As can be seen from the above results, the polypeptide molecules in the present disclosure have a relatively long half-life.

### Example 4. Evaluation of in vivo efficacy of compounds in diet-induced obesity (DIO) mouse model

In this example, a DIO mouse model established by high-fat diet induction was used to evaluate the effects on and improvement of obesity and diabetes-related indicators in DIO mice after subcutaneous injection of some of the compounds in the present disclosure.

4.1 Experimental methods: Obese male C57BL/6J mice (23-24 weeks old, obtained from Beijing Vital River Laboratory Animal Technology Co., Ltd.) induced by a high-fat diet were purchased. Six-week-old male C57BL/6J mice were fed a 60% high-fat diet (RD D12492) until their body weight reached 45-50g to obtain DIO model mice. After arriving at the facility and undergoing a week of acclimatization feeding, the animals were randomly divided into groups according to their body weight and administered vehicle (model group), LY3437943, Compound 15, Compound 16, Compound 17 and Compound 18 via subcutaneous injection in the neck. The compound was administered at a dose of 10 nmol/kg, and the frequency of administration was once daily via subcutaneous injection for 24 days. Each group contained 5 DIO mice. According to the experimental design, the body weight of each mouse was weighed and recorded daily at 3 pm or before administration, the average body weight of each group of mice was calculated, and a curve of body weight change over time was plotted. After the experiment, the fat and other organs of the mice were removed from various parts of the body, weighed, and recorded.

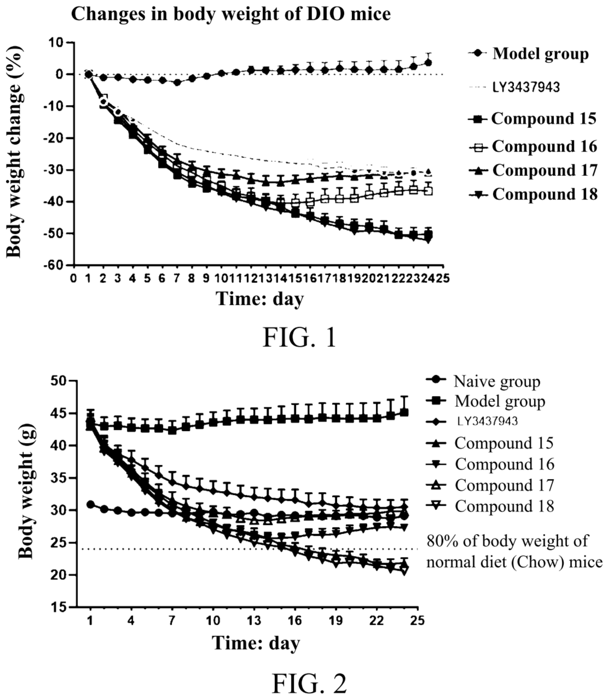

4.2 Experimental results: the body weight change (%) at different time points (days) in DIO mice after subcutaneous injection of the compounds is shown in FIG. 1. Change (%) refers to the percentage decrease in body weight of mice after injection. For example, the body weight change on day 15 (%) refers to the percentage decrease (%) in body weight between day 1 and day 15 after injection. Compared with the test group given the vehicle, administration of LY3437943 and Compounds 15, 16, 17 and 18 of the present disclosure resulted in a significant reduction in body weight of DIO mice. At the experimental endpoint, the body weight reduction was 30.4% in the LY3437943 group, 50.2% in the Compound 15 group, 36.6% in the Compound 16 group, 30.7% in the Compound 17 group, and 52.1% in the Compound 18 group. Among them, Compound 17 produced a significantly greater reduction in body weight than LY3437943 in DIO mice during the period from Day 5 to Day 15.

The changes in body weight (g) over time in DIO mice after subcutaneous injection of the compounds are shown in FIG. 2. Among them, LY3437943 and Compounds 15, 16, 17 and 18 can significantly reduce the body weight of DIO mice. Mice injected with Compounds 16 and 17 can maintain a final body weight which is 80% or more of the body weight of mice on a normal diet (Chow), indicating good safety.

### Example 5. Evaluation of in vivo efficacy of compounds in diet-induced obesity (DIO) rat model

### 5.1. Experimental purpose

In this experiment, a DIO rat model established on a 45% high-fat diet (HFD) was used to evaluate the effects of compounds on obesity-related indicators in DIO rats.

### 5.2. Experimental methods

After one week of acclimatization, all rats were randomly divided into two groups: a normal group and a model group. The rats in the model group were then fed 45% HFD (Medison: MD12032), while the rats in the normal group were fed normal food. The body weight of the rats was measured weekly to monitor the modeling process.

DIO rats fed a high-fat diet for 37 weeks were weighed, and 75 DIO rats (approximately 1100g) and 8 rats (approximately 800g) on a normal diet (Chow diet) were selected and separated into individual cages. Each cage was fed approximately 100g of food at a time, and the food was changed every 48 hours. A preliminary sham dose experiment was then conducted. Subcutaneous acupuncture was performed on the neck once daily at 4 pm for 9 consecutive days. Body weight was measured before each acupuncture session and the effect of sham dose on body weight was observed. A curve of body weight changes over time was plotted. The formal dosing experiment was initiated after the body weight had stabilized.

Six Chow diet rats (weighing approximately 800g) and 30 HFD rats (weighing approximately 1100g) were selected through a preliminary sham dose experiment for subsequent dosing experiments. Six Chow diet rats were used as the naive group, and the remaining HFD rats were randomly divided into six groups according to their body weight: model group, LY3437943 group, 0120 low-dose group, 0120 high-dose group, 0125 low-dose group, and 0125 high-dose group, with five rats in each group. During the experiment, all rats were housed in single cages. The compound was dissolved in PBS solution at pH 7.4. On the day of grouping (Day 1), rats in each group were given the drug by subcutaneous injection in the neck according to their body weight, once daily (QD), as detailed in Table 9. Starting on day 15 (D15), the administration dose of the LY3437943 group was adjusted from 1 nmol/kg to 10 nmol/kg, and the administration dose of the 0120 low-dose group and the 0125 low-dose group was adjusted from 0.3 nmol/kg to 3 nmol/kg. The body weight was measured once daily at 15:00 or before administration, and a curve of body weight changes over time was plotted. Food intake: 1-7 days, measured once daily at 15:00 or before administration; 8-40 days, measured once every three days at 15:00 or before administration. A curve of changes in daily/cumulative food intake over time was plotted.

**Table 9. Dosing information for experimental groups**

| Group | Drug | Number | Administration mode | Administration frequency | Administration dose (nmol/kg) | Administration concentration (nmol/mL) |
|---|---|---|---|---|---|---|
| Naive group | Vehicle (PBS) | 6 | S.C. | QD | / | / |
| Model group | Vehicle (PBS) | 5 | S.C. | QD | / | / |
| LY3437943 group | LY3437943 | 5 | S.C. | QD | 1 | 0.2 |
| Low-dose group of Compound 16 | 00-0120 | 5 | S.C. | QD | 0.3 | 0.06 |
| High-dose group of Compound 16 | 00-0120 | 5 | S.C. | QD | 1 | 0.2 |
| Low-dose group of Compound 17 | 00-0125 | 5 | S.C. | QD | 0.3 | 0.06 |
| High-dose group of Compound 17 | 00-0125 | 5 | S.C. | QD | 1 | 0.2 |

### 5.3. Experimental results:

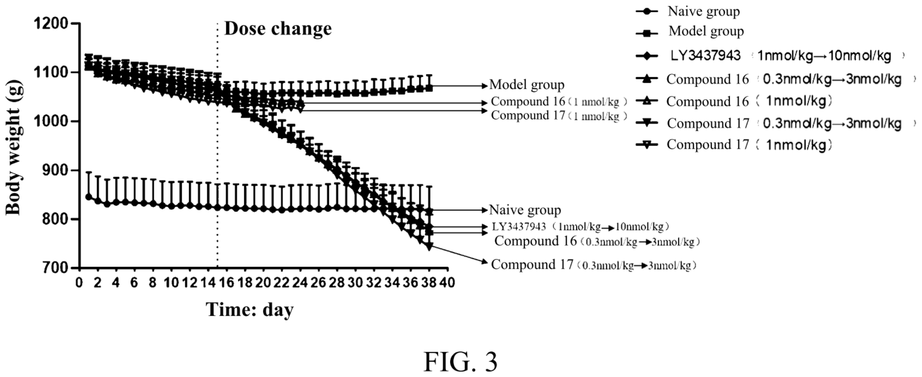

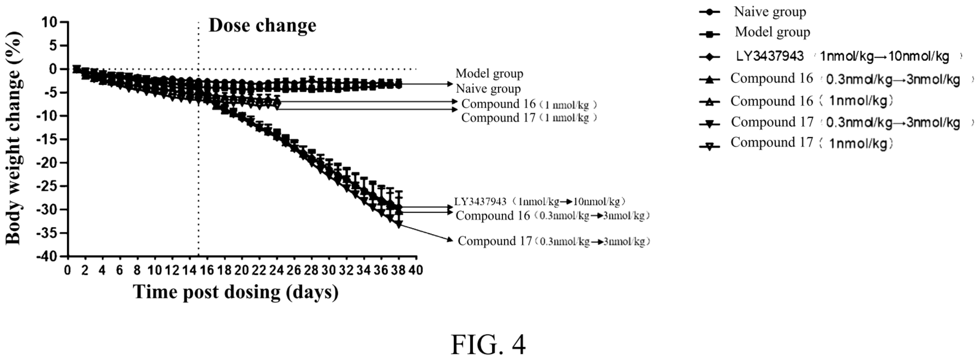

The rat body weight results are shown in FIGS. 3 and 4. Before adjusting the administration dose, the body weight of rats in the model group was significantly increased compared with the naive group; compared with the model group, the body weight of rats in the Compound 16 (1 nmol/kg) group and the Compound 17 (1 nmol/kg) group showed a decreasing trend. After the dose of LY3437943 was adjusted from 1 nmol/kg to 10 nmol/kg, the dose of Compound 16 was adjusted from 0.3 nmol/kg to 3 nmol/kg, and the dose of Compound 17 was adjusted from 0.3 nmol/kg to 3 nmol/kg, the body weight of rats in each group began to decrease rapidly. Among them, the body weight loss of rats in the LY3437943 (10 nmol/kg) group was 29.4% at the end of the experiment; the body weight loss of rats in the Compound 16 (3 nmol/kg) group was 30.2% at the end of the experiment; and the body weight loss of rats in the Compound 17 (3 nmol/kg) group was 33.2% at the end of the experiment.

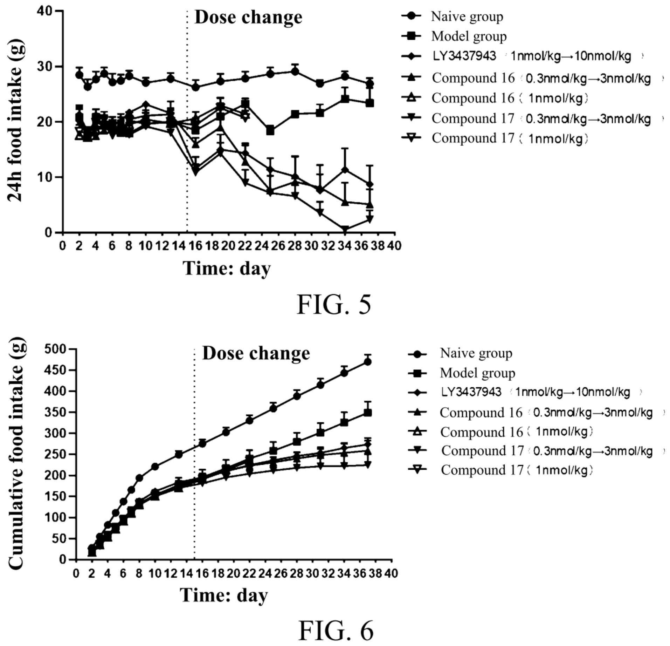

As shown in FIGS. 5 and 6, before dose adjustment, the 24h food intake and cumulative food intake of each administration group were basically equivalent to those of the model group. After dose adjustment, compared with the model group, the 24h food intake and cumulative food intake of rats in the LY3437943 (10 nmol/kg), Compound 16 (3 nmol/kg), and Compound 17 (3 nmol/kg) groups were significantly reduced. Furthermore, the 24h food intake gradually decreased with the extension of the administration time. Throughout the entire administration period (before and after dose adjustment), the cumulative food intake of each administration group was as follows: LY3437943 (10 nmol/kg) group: 273.8 ± 15.0 g, with a 29.4% body weight loss; Compound 16 (3 nmol/kg) group: 258.8 + 23.5 g, with a 30.2% body weight loss; Compound 17 (3 nmol/kg) group: 224.5 ± 25.8 g, with a 33.2% body weight loss.

## Claims

1. A compound represented by formula (I) or a pharmaceutically acceptable salt thereof:
R₁-X₁-X₂-X₃-Gly-Thr-Phe-Thr-Ser-Asp-X₁₀-Ser-X₁₂-X₁₃-X₁₄-Glu-X₁₆-X₁₇-X₁₈-X₁₉-X₂₀-X₂₁-Phe-Val-Glu-Trp-Leu-X₂₇-X₂₈-X₂₉-Z-X₄₀-R₂ (SEQ ID NO: 145) (I);
wherein,
R₁ is hydrogen, alkyl, acetyl, formyl, benzoyl, trifluoroacetyl, or pGlu,
R₂ is -NH₂ or -OH;
X₁, X₂, X₃, X₁₀, X₁₂, X₁₃, X₁₄, X₁₆, X₁₇, X₁₈, X₁₉, X₂₀, X₂₁, X₂₇, X₂₈, X₂₉, Z, and X₄₀ are each independently selected from any natural amino acids, unnatural amino acids, or peptide segments composed of natural amino acids and/or unnatural amino acids.

2. The compound or the pharmaceutically acceptable salt thereof according to claim 1, wherein:
R₁ is hydrogen, alkyl, acetyl, formyl, benzoyl, trifluoroacetyl, or pGlu,
R₂ is -NH₂ or -OH;
X₁ is selected from an amino acid residue of Tyr or His;
X₂ is an amino acid residue of Aib;
X₃ is selected from an amino acid residue of Gln or His;
X₁₀ is selected from an amino acid residue of Leu or Tyr;
X₁₂ is selected from an amino acid residue of Lys, alpha-methyl-Lys, or Ile;
X₁₃ is selected from an amino acid residue of Aib, Ile, Leu, Tyr, alpha-methyl-Tyr or alpha-methyl-Leu;
X₁₄ is selected from an amino acid residue of Leu or alpha-methyl-Leu;
X₁₆ is selected from an amino acid residue of Glu or Lys, or Y₁;
X₁₇ is selected from an amino acid residue of Glu, Arg, homoArg, Ile, Lys, Gln or alpha-methyl-Lys, or Y₁;
X₁₈ is selected from an amino acid residue of Ala or Aib;
X₁₉ is selected from an amino acid residue of Ala or Gln;
X₂₀ is selected from an amino acid residue of Gln, Aib, isovaline, alpha-methyl-Ser, 1-aminocyclopropane-1-carboxylic acid (Ac3c) or 1-aminocyclobutane-1-carboxylic acid (Ac4c);
X₂₁ is selected from an amino acid residue of Glu, or Y₁;
X₂₇ is selected from an amino acid residue of Ile or Leu;
X₂₈ is selected from an amino acid residue of Ala or Glu;
X₂₉ is selected from an amino acid residue of Gly or D-Glu;
X₄₀ is selected from an amino acid residue of Lys, or Y₁, or is absent;
Z is selected from Gly-Pro-Ser-Ser-Gly-Ala-Pro-Pro-Pro-Ser (SEQ ID NO: 146), Gly-Pro-Ser-Ser-Gly-Ala-Pro-Pro-Ser (SEQ ID NO: 147), Pro-Ser-Ser-Gly-Ala-Pro-Pro-Pro-Ser (SEQ ID NO: 148), or Pro-Ser-Ser-Gly-Ala-Pro-Pro-Ser (SEQ ID NO: 149);
Y₁ is a Lys, Orn, Dap, Dab, or Cys residue with the side chain linked to a substituent.

3. The compound or the pharmaceutically acceptable salt thereof according to claim 1 or 2, which is a compound represented by formula (Ib) or a pharmaceutically acceptable salt thereof:
R₁-X₁-X₂-X₃-Gly-Thr-Phe-Thr-Ser-Asp-X₁₀-Ser-X₁₂-X₁₃-X₁₄Glu-Y₁-X₁₇X₁₈-X₁₉-X₂₀-Glu-Phe-Val-Glu-Trp-Leu-X₂₇-X₂₈-X₂₉-Gly-Pro-Ser-Ser-Gly-Ala-Pro-Pro-Pro-Ser-R₂ (SEQ ID NO: 150) (Ib);
wherein,
R₁ is hydrogen,
R₂ is -NH₂ or -OH;
X₁ is selected from an amino acid residue of Tyr or His;
X₂ is an amino acid residue of Aib;
X₃ is selected from an amino acid residue of Gln or His;
X₁₀ is selected from an amino acid residue of Leu or Tyr;
X₁₂ is selected from an amino acid residue of Lys, alpha-methyl-Lys, or Ile;
X₁₃ is selected from an amino acid residue of Aib, Ile, Leu, Tyr, alpha-methyl-Tyr or alpha-methyl-Leu;
X₁₄ is selected from an amino acid residue of Leu or alpha-methyl-Leu;
X₁₇ is selected from an amino acid residue of Ile, Lys, Gln, or alpha-methyl-Lys;
X₁₈ is selected from an amino acid residue of Ala or Aib;
X₁₉ is selected from an amino acid residue of Ala or Gln;
X₂₀ is selected from an amino acid residue of Gln, Aib, isovaline, alpha-methyl-Ser, 1-aminocyclopropane-1-carboxylic acid or 1-aminocyclobutane-1-carboxylic acid;
X₂₇ is selected from an amino acid residue of Ile or Leu;
X₂₈ is selected from an amino acid residue of Ala or Glu;
X₂₉ is selected from an amino acid residue of Gly or D-Glu;
Y₁is a Lys residue with the side chain linked to a substituent.

4. The compound or the pharmaceutically acceptable salt thereof according to any one of claims 1-3, wherein,
X₁, X₂ and X₃ are selected from any of the following:
X₁ is His, X₂ is Aib, and X₃ is His;
X₁ is Tyr, X₂ is Aib, and X₃ is Gln; or
X₁ is His, X₂ is Aib, and X₃ is Gln;
preferably,
X₁ is His, X₂ is Aib, and X₃ is His; or
X₁ is His, X₂ is Aib, and X₃ is Gln;
more preferably,
X₁ is His, X₂ is Aib, and X₃ is His.

5. The compound or the pharmaceutically acceptable salt thereof according to any one of claims 1-4, wherein
X₁₇ is an amino acid residue of Ile, Lys, Gln, or alpha-methyl-Lys;
preferably, X₁₇, X₁₈, X₁₉ and X₂₀ are selected from any of the following:
X₁₇ is Lys, X₁₈ is Ala, X₁₉ is Ala, and X₂₀ is Gln;
X₁₇ is Lys, X₁₈ is Ala, X₁₉ is Ala, and X₂₀ is alpha-methyl-Ser;
X₁₇ is Lys, X₁₈ is Ala, X₁₉ is Ala, and X₂₀ is isovaleine;
X₁₇ is Lys, X₁₈ is Ala, X₁₉ is Ala, and X₂₀ is 1-aminocyclopropane-1-carboxylic acid;
X₁₇ is Lys, X₁₈ is Ala, X₁₉ is Ala, and X₂₀ is 1-aminocyclobutane-1-carboxylic acid;
X₁₇ is Lys, X₁₈ is Ala, X₁₉ is Ala, and X₂₀ is Aib;
X₁₇ is Lys, X₁₈ is Aib, X₁₉ is Ala, and X₂₀ is Gln;
X₁₇ is Lys, X₁₈ is Ala, X₁₉ is Gln, and X₂₀ is Gln;
X₁₇ is Gln, X₁₈ is Ala, X₁₉ is Ala, and X₂₀ is Gln;
X₁₇ is Gln, X₁₈ is Ala, X₁₉ is Ala, and X₂₀ is isovaleine;
X₁₇ is Gln, X₁₈ is Ala, X₁₉ is Ala, and X₂₀ is 1-aminocyclopropane-1-carboxylic acid;
X₁₇ is Gln, X₁₈ is Ala, X₁₉ is Ala, and X₂₀ is 1-aminocyclobutane-1-carboxylic acid;
X₁₇ is alpha-methyl-Lys, X₁₈ is Ala, X₁₉ is Ala, and X₂₀ is Gln; or
X₁₇ is Ile, X₁₈ is Ala, X₁₉ is Ala, and X₂₀ is Gln.

6. The compound or the pharmaceutically acceptable salt thereof according to any one of claims 1-5, wherein X₁₂, X₁₃ and X₁₄ are selected from any of the following:
X₁₂ is Ile, X₁₃ is Leu, and X₁₄ is Leu;
X₁₂ is Ile, X₁₃ is alpha-methyl-Leu, and X₁₄ is Leu;
X₁₂ is Lys, X₁₃ is alpha-methyl-Tyr, and X₁₄ is Leu;
X₁₂ is Lys, X₁₃ is Aib, and X₁₄ is Leu;
X₁₂ is Lys, X₁₃ is Tyr, and X₁₄ is alpha-methyl-Leu;
X₁₂ is Lys, X₁₃ is Tyr, and X₁₄ is Leu;
X₁₂ is alpha-methyl-Lys, X₁₃ is Tyr, and X₁₄ is Leu;
X₁₂ is alpha-methyl-Lys, X₁₃ is Leu, and X₁₄ is Leu; or
X₁₂ is alpha-methyl-Lys, X₁₃ is Ile, and X₁₄ is Leu.

7. The compound or the pharmaceutically acceptable salt thereof according to any one of claims 1-6, which is a compound represented by formula (Ib) or a pharmaceutically acceptable salt thereof:
R₁-X₁-X₂-X₃-Gly-Thr-Phe-Thr-Ser-Asp-X₁₀-Ser-X₁₂-X₁₃-X₁₄-Glu-Y₁-X₁₇-X₁₈-X₁₉-X₂₀-Glu-Phe-Val-Glu-Trp-Leu-X₂₇-X₂₈-X₂₉-Gly-Pro-Ser-Ser-Gly-Ala-Pro-Pro-Pro-Ser-R₂ (SEQ ID NO: 150) (Ib);
wherein,
R₁ is hydrogen,
R₂ is -NH₂ or -OH;
X₁ is His;
X₂ is Aib;
X₃ is His or Gln;
X₁₀ is selected from an amino acid residue of Leu or Tyr;
X₁₂ is selected from an amino acid residue of Lys, alpha-methyl-Lys, or Ile;
X₁₃ is selected from an amino acid residue of Aib, Ile, Leu, Tyr, alpha-methyl-Tyr or alpha-methyl-Leu;
X₁₄ is selected from an amino acid residue of Leu or alpha-methyl-Leu;
X₁₇ is selected from an amino acid residue of Ile, Lys, Gln, or alpha-methyl-Lys;
X₁₈ is selected from an amino acid residue of Ala or Aib;
X₁₉ is selected from an amino acid residue of Ala or Gln;
X₂₀ is selected from an amino acid residue of Gln, Aib, isovaline, alpha-methyl-Ser, 1-aminocyclopropane-1-carboxylic acid or 1-aminocyclobutane-1-carboxylic acid;
X₂₇ is selected from an amino acid residue of Ile or Leu;
X₂₈ is selected from an amino acid residue of Ala or Glu;
X₂₉ is selected from an amino acid residue of Gly or D-Glu;
Y₁ is a Lys residue with the side chain linked to a substituent;
preferably,
X₁₇, X₁₈, X₁₉ and X₂₀ are selected from any of the following:
X₁₇ is Lys, X₁₈ is Ala, X₁₉ is Ala, and X₂₀ is Gln;
X₁₇ is Lys, X₁₈ is Ala, X₁₉ is Ala, and X₂₀ is alpha-methyl-Ser;
X₁₇ is Lys, X₁₈ is Ala, X₁₉ is Ala, and X₂₀ is isovaleine;
X₁₇ is Lys, X₁₈ is Ala, X₁₉ is Ala, and X₂₀ is 1-aminocyclopropane-1-carboxylic acid;
X₁₇ is Lys, X₁₈ is Ala, X₁₉ is Ala, and X₂₀ is 1-aminocyclobutane-1-carboxylic acid;
X₁₇ is Lys, X₁₈ is Ala, X₁₉ is Ala, and X₂₀ is Aib;
X₁₇ is Lys, X₁₈ is Aib, X₁₉ is Ala, and X₂₀ is Gln;
X₁₇ is Lys, X₁₈ is Ala, X₁₉ is Gln, and X₂₀ is Gln;
X₁₇ is Gln, X₁₈ is Ala, X₁₉ is Ala, and X₂₀ is Gln;
X₁₇ is Gln, X₁₈ is Ala, X₁₉ is Ala, and X₂₀ is isovaleine;
X₁₇ is Gln, X₁₈ is Ala, X₁₉ is Ala, and X₂₀ is 1-aminocyclopropane-1-carboxylic acid;
X₁₇ is Gln, X₁₈ is Ala, X₁₉ is Ala, and X₂₀ is 1-aminocyclobutane-1-carboxylic acid;
X₁₇ is alpha-methyl-Lys, X₁₈ is Ala, X₁₉ is Ala, and X₂₀ is Gln; or
X₁₇ is Ile, X₁₈ is Ala, X₁₉ is Ala, and X₂₀ is Gln:
preferably,
X₁₂, X₁₃ and X₁₄ are selected from any of the following:
X₁₂ is Ile, X₁₃ is Leu, and X₁₄ is Leu;
X₁₂ is Ile, X₁₃ is alpha-methyl-Leu, and X₁₄ is Leu;
X₁₂ is Lys, X₁₃ is alpha-methyl-Tyr, and X₁₄ is Leu;
X₁₂ is Lys, X₁₃ is Aib, and X₁₄ is Leu;
X₁₂ is Lys, X₁₃ is Tyr, and X₁₄ is alpha-methyl-Leu;
X₁₂ is Lys, X₁₃ is Tyr, and X₁₄ is Leu;
X₁₂ is alpha-methyl-Lys, X₁₃ is Tyr, and X₁₄ is Leu;
X₁₂ is alpha-methyl-Lys, X₁₃ is Leu, and X₁₄ is Leu; or
X₁₂ is alpha-methyl-Lys, X₁₃ is Ile, and X₁₄ is Leu.

8. The compound or the pharmaceutically acceptable salt thereof according to claim 1 or 2, wherein X₁ to X₂₉ are selected from any of the following groups:
(i) X₁, X₂ and X₃ are selected from any of the following:
X₁ is His, X₂ is Aib, and X₃ is His;
X₁ is His, X₂ is Aib, and X₃ is Gln; or
X₁ is Tyr, X₂ is Aib, and X₃ is Gln;
X₁₀ is selected from an amino acid residue of Leu or Tyr;
X₁₂ is selected from an amino acid residue of Lys, alpha-methyl-Lys, or Ile;
X₁₃ is selected from an amino acid residue of Aib, Ile, Leu, Tyr, alpha-methyl-Tyr or alpha-methyl-Leu;
X₁₄ is selected from an amino acid residue of Leu or alpha-methyl-Leu;
X₁₆ is Y₁;
X₁₇ is selected from an amino acid residue of Lys;
X₁₈ is selected from an amino acid residue of Ala or Aib;
X₁₉ is selected from an amino acid residue of Ala or Gln;
X₂₀ is selected from an amino acid residue of Gln, Aib, isovaline, alpha-methyl-Ser, 1-aminocyclopropane-1-carboxylic acid or 1-aminocyclobutane-1-carboxylic acid;
X₂₇ is selected from an amino acid residue of Ile or Leu;
X₂₈ is selected from an amino acid residue of Ala or Glu; and
X₂₉ is selected from an amino acid residue of Gly or D-Glu;
(ii) X₁, X₂ and X₃ are selected from any of the following:
X₁ is His, X₂ is Aib, and X₃ is His;
X₁ is His, X₂ is Aib, and X₃ is Gln; or
X₁ is Tyr, X₂ is Aib, and X₃ is Gln;
X₁₀ is selected from an amino acid residue of Leu or Tyr;
X₁₂ is selected from an amino acid residue of Lys, alpha-methyl-Lys, or Ile;
X₁₃ is selected from an amino acid residue of Aib, Ile, Leu, Tyr, alpha-methyl-Tyr or alpha-methyl-Leu;
X₁₄ is selected from an amino acid residue of Leu or alpha-methyl-Leu;
X₁₆ is Y₁;
X₁₇ is selected from an amino acid residue of Lys;
X₁₈ is selected from an amino acid residue of Ala or Aib;
X₁₉ is selected from an amino acid residue of Ala or Gln;
X₂₀ is selected from an amino acid residue of Gln;
X₂₇ is selected from an amino acid residue of Ile or Leu;
X₂₈ is selected from an amino acid residue of Ala or Glu; and
X₂₉ is selected from an amino acid residue of Gly or D-Glu;
(iii) X₁, X₂ and X₃ are selected from any of the following:
X₁ is His, X₂ is Aib, and X₃ is His;
X₁ is His, X₂ is Aib, and X₃ is Gln; or
X₁ is Tyr, X₂ is Aib, and X₃ is Gln;
X₁₀ is selected from an amino acid residue of Leu or Tyr;
X₁₂ is selected from an amino acid residue of alpha-methyl-Lys or Ile;
X₁₃ is selected from an amino acid residue of Ile, Leu or alpha-methyl-Leu;
X₁₄ is selected from an amino acid residue of Leu or alpha-methyl-Leu;
X₁₆ is Y₁,
X₁₇ is selected from an amino acid residue of Lys;
X₁₈ is selected from an amino acid residue of Ala;
X₁₉ is selected from an amino acid residue of Ala;
X₂₀ is selected from an amino acid residue of Aib, isovaline, alpha-methyl-Ser, 1-aminocyclopropane-1-carboxylic acid or 1-aminocyclobutane-1-carboxylic acid;
X₂₇ is selected from an amino acid residue of Ile or Leu;
X₂₈ is selected from an amino acid residue of Ala or Glu; and
X₂₉ is selected from an amino acid residue of Gly or D-Glu;
(iv) X₁ is His;
X₂ is Aib;
X₃ is His;
X₁₀ is selected from an amino acid residue of Tyr;
X₁₂ is selected from an amino acid residue of Lys or alpha-methyl-Lys;
X₁₃ is selected from an amino acid residue of Tyr;
X₁₄ is selected from an amino acid residue of Leu or alpha-methyl-Leu;
X₁₆ is Y₁;
X₁₇ is selected from an amino acid residue of Lys;
X₁₈ is selected from an amino acid residue of Ala or Aib;
X₁₉ is selected from an amino acid residue of Ala or Gln;
X₂₀ is selected from an amino acid residue of Gln;
X₂₇ is selected from an amino acid residue of Ile or Leu;
X₂₈ is selected from an amino acid residue of Ala or Glu; and
X₂₉ is selected from an amino acid residue of Gly;
(v) X₁ is His;
X₂ is Aib;
X₃ is His or Gln;
X₁₀ is selected from an amino acid residue of Leu or Tyr;
X₁₂ is selected from an amino acid residue of Ile;
X₁₃ is selected from an amino acid residue of alpha-methyl-Leu;
X₁₄ is selected from an amino acid residue of Leu;
X₁₆ is Y₁;
X₁₇ is selected from an amino acid residue of Lys;
X₁₈ is selected from an amino acid residue of Ala;
X₁₉ is selected from an amino acid residue of Ala;
X₂₀ is selected from an amino acid residue of Gln, Aib, 1-aminocyclobutane-1-carboxylic acid, or isovaleine;
X₂₇ is selected from an amino acid residue of Leu;
X₂₈ is selected from an amino acid residue of Ala; and
X₂₉ is selected from an amino acid residue of Gly or D-Glu;
(vi) X₁, X₂ and X₃ are selected from any of the following:
X₁ is His, X₂ is Aib, and X₃ is His;
X₁ is His, X₂ is Aib, and X₃ is Gln; or
X₁ is Tyr, X₂ is Aib, and X₃ is Gln;
X₁₀ is selected from an amino acid residue of Leu or Tyr;
X₁₂ is selected from an amino acid residue of alpha-methyl-Lys or Ile;
X₁₃ is selected from an amino acid residue of Leu;
X₁₄ is selected from an amino acid residue of Leu;
X₁₆ is Y₁;
X₁₇ is selected from an amino acid residue of Ile, Lys or Gln;
X₁₈ is selected from an amino acid residue of Ala;
X₁₉ is selected from an amino acid residue of Ala;
X₂₀ is selected from an amino acid residue of Gln, Aib, isovaline, alpha-methyl-Ser, 1-aminocyclopropane-1-carboxylic acid or 1-aminocyclobutane-1-carboxylic acid;
X₂₇ is selected from an amino acid residue of Ile or Leu;
X₂₈ is selected from an amino acid residue of Ala or Glu; and
X₂₉ is selected from an amino acid residue of Gly or D-Glu;
(vii) X₁ is His;
X₂ is Aib;
X₃ is His;
X₁₀ is selected from an amino acid residue of Tyr;
X₁₂ is selected from an amino acid residue of Lys;
X₁₃ is selected from an amino acid residue of alpha-methyl-Tyr or Aib;
X₁₄ is selected from an amino acid residue of Leu;
X₁₆ is Y₁;
X₁₇ is selected from an amino acid residue of Lys;
X₁₈ is selected from an amino acid residue of Ala;
X₁₉ is selected from an amino acid residue of Ala;
X₂₀ is selected from an amino acid residue of Gln;
X₂₇ is selected from an amino acid residue of Leu;
X₂₈ is selected from an amino acid residue of Ala; and
X₂₉ is selected from an amino acid residue of Gly;
(viii) X₁, X₂ and X₃ are selected from any of the following:
X₁ is His, X₂ is Aib, and X₃ is His; or
X₁ is Tyr, X₂ is Aib, and X₃ is Gln;
X₁₀ is selected from an amino acid residue of Leu or Tyr;
X₁₂ is selected from an amino acid residue of alpha-methyl-Lys;
X₁₃ is selected from an amino acid residue of Ile;
X₁₄ is selected from an amino acid residue of Leu;
X₁₆ is Y₁;
X₁₇ is selected from an amino acid residue of Lys;
X₁₈ is selected from an amino acid residue of Ala;
X₁₉ is selected from an amino acid residue of Ala;
X₂₀ is selected from an amino acid residue of Gln or isovaleine;
X₂₇ is selected from an amino acid residue of Leu or Ile;
X₂₈ is selected from an amino acid residue of Ala or Glu; and
X₂₉ is selected from an amino acid residue of Gly;
(ix) X₁ is His;
X₂ is Aib;
X₃ is His;
X₁₀ is selected from an amino acid residue of Leu or Tyr;
X₁₂ is selected from an amino acid residue of Lys, alpha-methyl-Lys, or Ile;
X₁₃ is selected from an amino acid residue of Ile, Leu or Tyr;
X₁₄ is selected from an amino acid residue of Leu;
X₁₆ is Y₁;
X₁₇ is selected from an amino acid residue of Ile, Gln or alpha-methyl-Lys;
X₁₈ is selected from an amino acid residue of Ala;
X₁₉ is selected from an amino acid residue of Ala;
X₂₀ is selected from an amino acid residue of Gln, isovaline, 1-aminocyclopropane-1-carboxylic acid or 1-aminocyclobutane-1-carboxylic acid;
X₂₇ is selected from an amino acid residue of Leu;
X₂₈ is selected from an amino acid residue of Ala; and
X₂₉ is selected from an amino acid residue of Gly or D-Glu;
(x) X₁ is His;
X₂ is Aib;
X₃ is His;
X₁₀ is selected from an amino acid residue of Leu or Tyr;
X₁₂ is selected from an amino acid residue of alpha-methyl-Lys or Ile;
X₁₃ is selected from an amino acid residue of Ile or Leu;
X₁₄ is selected from an amino acid residue of Leu;
X₁₆ is Y₁;
X₁₇ is selected from an amino acid residue of Gln;
X₁₈ is selected from an amino acid residue of Ala;
X₁₉ is selected from an amino acid residue of Ala;
X₂₀ is selected from an amino acid residue of Gln, isovaline, 1-aminocyclopropane-1-carboxylic acid or 1-aminocyclobutane-1-carboxylic acid;
X₂₇ is selected from an amino acid residue of Leu;
X₂₈ is selected from an amino acid residue of Ala; and
X₂₉ is selected from an amino acid residue of Gly or D-Glu;
(xi) X₁ is His;
X₂ is Aib;
X₃ is His;
X₁₀ is selected from an amino acid residue of Tyr;
X₁₂ is selected from an amino acid residue of Lys or alpha-methyl-Lys;
X₁₃ is selected from an amino acid residue of Leu or Tyr;
X₁₄ is selected from an amino acid residue of Leu;
X₁₆ is Y₁,
X₁₇ is selected from an amino acid residue of Ile or alpha-methyl-Lys;
X₁₈ is selected from an amino acid residue of Ala;
X₁₉ is selected from an amino acid residue of Ala;
X₂₀ is selected from an amino acid residue of Gln;
X₂₇ is selected from an amino acid residue of Leu;
X₂₈ is selected from an amino acid residue of Ala; and
X₂₉ is selected from an amino acid residue of Gly.

9. The compound or the pharmaceutically acceptable salt thereof according to claim 1 or 2, which comprises or is a polypeptide sequence as shown in any of the following:
H-Aib-HGTFTSDYS-αK-YLEEKAAQEFVEWLLAGGPSSGAPPPS-NH₂ (SEQ ID NO: 1);
H-Aib-HGTFTSDYS-αK-YLEKKAAQEFVEWLLAGGPSSGAPPPS-NH₂ (SEQ ID NO: 2);
H-Aib-HGTFTSDYSK-αY-LEKKAAQEFVEWLLAGGPSSGAPPPS-NH₂ (SEQ ID NO: 3);
H-Aib-HGTFTSDYSK-Aib-LEKKAAQEFVEWLLAGGPSSGAPPPS-NH₂ (SEQ ID NO: 4);
H-Aib-HGTFTSDYSKY-αL-EKKAAQEFVEWLLAGGPSSGAPPPS-NH₂ (SEQ ID NO: 5);
H-Aib-HGTFTSDYSKYLEK-αK-AAQEFVEWLLAGGPSSGAPPPS-NH₂ (SEQ ID NO: 6);
H-Aib-HGTFTSDYSKYLEKK-Aib-AQEFVEWLLAGGPSSGAPPPS-NH₂ (SEQ ID NO: 7);
H-Aib-HGTFTSDYS-αK-YLEKKAAQEFVEWLIAGGPSSGAPPPS-NH₂ (SEQ ID NO: 8);
H-Aib-HGTFTSDYS-αK-LLEKKAAQEFVEWLLAGGPSSGAPPPS-NH₂ (SEQ ID NO: 9);
H-Aib-HGTFTSDYS-αK-YLEKKAQQEFVEWLLAGGPSSGAPPPS-NH₂ (SEQ ID NO: 10);
Y-Aib-QGTFTSDYS-αK-LLEKKAAQEFVEWLLAGGPSSGAPPPS-NH₂ (SEQ ID NO: 11);
H-Aib-HGTFTSDYS-αK-YLEKKAAQEFVEWLIEGGPSSGAPPPS-NH₂ (SEQ ID NO: 12);
H-Aib-HGTFTSDYS-αK-LLEKQAAQEFVEWLLAGGPSSGAPPPS-NH₂ (SEQ ID NO: 13);
H-Aib-HGTFTSDYS-αK-LLEKIAAQEFVEWLLAGGPSSGAPPPS-NH₂ (SEQ ID NO: 14);
H-Aib-HGTFTSDYS-αK-ILEKKAAQEFVEWLLAGGPSSGAPPPS-NH₂ (SEQ ID NO: 15);
H-Aib-HGTFTSDYSILLEKKAAQEFVEWLLAGGPSSGAPPPS-NH₂ (SEQ ID NO: 16);
H-Aib-HGTFTSDYSI-αL-LEKKAAQEFVEWLLAGGPSSGAPPPS-NH₂ (SEQ ID NO: 17);
H-Aib-HGTFTSDYS-αK-LLEKKAA-αS-EFVEWLIEGGPSSGAPPPS-NH₂ (SEQ ID NO: 18);
Y-Aib-QGTFTSDYS-αK-LLEKKAA-Iva-EFVEWLLAGGPSSGAPPPS-NH₂ (SEQ ID NO: 19);
H-Aib-HGTFTSDYS-αK-LLEKQAA-Iva-EFVEWLLAGGPSSGAPPPS-NH₂ (SEQ ID NO: 20);
Y-Aib-QGTFTSDYS-αK-ILEKKAA-Iva-EFVEWLLAGGPSSGAPPPS-NH₂ (SEQ ID NO: 21);
H-Aib-HGTFTSDYS-αK-ILEKQAA-Iva-EFVEWLLAGGPSSGAPPPS-NH₂ (SEQ ID NO: 22);
H-Aib-HGTFTSDYS-αK-LLEKKAA-Iva-EFVEWLIEGGPSSGAPPPS-NH₂ (SEQ ID NO: 23);
H-Aib-HGTFTSDYS-αK-ILEKKAA-Iva-EFVEWLIEGGPSSGAPPPS-NH₂ (SEQ ID NO: 24);
H-Aib-HGTFTSDLS-αK-LLEKKAA-Iva-EFVEWLIEGGPSSGAPPPS-NH₂ (SEQ ID NO: 25);
H-Aib-HGTFTSDLS-αK-ILEKKAA-Iva-EFVEWLIEGGPSSGAPPPS-NH₂ (SEQ ID NO: 26);
H-Aib-HGTFTSDYSILLEKKAA-Iva-EFVEWLLAGGPSSGAPPPS-NH₂ (SEQ ID NO: 27);
H-Aib-HGTFTSDLSILLEKKAA-Iva-EFVEWLLAGGPSSGAPPPS-NH₂ (SEQ ID NO: 28);
H-Aib-HGTFTSDYSI-αL-LEKKAA-Iva-EFVEWLLAGGPSSGAPPPS-NH₂ (SEQ ID NO: 29);
H-Aib-HGTFTSDLSI-αL-LEKKAA-Iva-EFVEWLLAGGPSSGAPPPS-NH₂ (SEQ ID NO: 30);
H-Aib-HGTFTSDYSI-αL-LEKKAAQEFVEWLLAeGPSSGAPPPS-NH₂ (SEQ ID NO: 31);
H-Aib-HGTFTSDLSI-αL-LEKKAA-Ac4c-EFVEWLLAGGPSSGAPPPS-NH₂ (SEQ ID NO: 32);
H-Aib-HGTFTSDLSI-αL-LEKKAAQEFVEWLLAeGPSSGAPPPS-NH₂ (SEQ ID NO: 33);
H-Aib-HGTFTSDLSI-αL-LEKKAAQEFVEWLLAGGPSSGAPPPS-NH₂ (SEQ ID NO: 34);
H-Aib-HGTFTSDLSILLEKKAAQEFVEWLLAeGPSSGAPPPS-NH₂ (SEQ ID NO: 35);
H-Aib-HGTFTSDLSILLEKQAA-Ac4c-EFVEWLLAeGPSSGAPPPS-NH₂ (SEQ ID NO: 36);
H-Aib-HGTFTSDLSILLEKKAA-Ac3c-EFVEWLLAeGPSSGAPPPS-NH₂ (SEQ ID NO: 37);
H-Aib-HGTFTSDLSILLEKKAA-Ac4c-EFVEWLLAeGPSSGAPPPS-NH₂ (SEQ ID NO: 38);
H-Aib-HGTFTSDLSILLEKQAA-Ac3c-EFVEWLLAeGPSSGAPPPS-NH₂ (SEQ ID NO: 39);
H-Aib-HGTFTSDYSILLEKQAA-Ac3c-EFVEWLLAeGPSSGAPPPS-NH₂ (SEQ ID NO: 40);
H-Aib-HGTFTSDYSILLEKQAA-Ac4c-EFVEWLLAeGPSSGAPPPS-NH₂ (SEQ ID NO: 41);
H-Aib-HGTFTSDYSILLEKKAAQEFVEWLLAeGPSSGAPPPS-NH₂ (SEQ ID NO: 42);
H-Aib-HGTFTSDYSILLEKQAAQEFVEWLLAGGPSSGAPPPS-NH₂ (SEQ ID NO: 43);
H-Aib-HGTFTSDYSILLEKQAAQEFVEWLLAeGPSSGAPPPS-NH₂ (SEQ ID NO: 44);
H-Aib-QGTFTSDYSILLEKKAAQEFVEWLLAGGPSSGAPPPS-NH₂ (SEQ ID NO: 45);
H-Aib-QGTFTSDYSI-αL-LEKKAAQEFVEWLLAGGPSSGAPPPS-NH₂ (SEQ ID NO: 46);
H-Aib-QGTFTSDYSILLEKKAA-Aib-EFVEWLLAGGPSSGAPPPS-NH₂ (SEQ ID NO: 47); and
H-Aib-QGTFTSDYST-αL-LEKKAA-Aib-EFVEWLLAGGPSSGAPPPS-NH₂; (SEQ ID NO: 48);
αK is alpha-methyl-Lys, αY is alpha-methyl-Tyr, αL is alpha-methyl-Leu, Iva is isovaleine, e is D-Glu, Ac3c is 1-aminocyclopropane-1-carboxylic acid, and Ac4c is 1-aminocyclobutane-1-carboxylic acid.

10. The compound or the pharmaceutically acceptable salt thereof according to claim 1 or 2, which comprises or is the structure as shown in any of the following:
H-Aib-HGTFTSDYS-αK-YLEEY₁AAQEFVEWLLAGGPSSGAPPPS-NH₂ (SEQ ID NO: 49);
H-Aib-HGTFTSDYS-αK-YLEY₁KAAQEFVEWLLAGGPSSGAPPPS-NH₂ (SEQ ID NO: 50);
H-Aib-HGTFTSDYSK-αY-LEYiKAAQEFVEWLLAGGPSSGAPPPS-NH₂ (SEQ ID NO: 51);
H-Aib-HGTFTSDYSK-Aib-LEY₁KAAQEFVEWLLAGGPSSGAPPPS-NHz (SEQ ID NO: 52);
H-Aib-HGTFTSDYSKY-αL-EY₁KAAQEFVEWLLAGGPSSGAPPPS-NH₂ (SEQ ID NO: 53);
H-Aib-HGTFTSDYSKYLEY₁-αK-AAQEFVEWLLAGGPSSGAPPPS-NH₂ (SEQ ID NO: 54);
H-Aib-HGTFTSDYSKYLEY₁K-Aib-AQEFVEWLLAGGPSSGAPPPS-NH_{Z} (SEQ ID NO: 55);
H-Aib-HGTFTSDYS-αK-YLEY₁KAAQEFVEWLIAGGPSSGAPPPS-NH₂ (SEQ ID NO: 56);
H-Aib-HGTFTSDYS-αK-LLEY₁KAAQEFVEWLLAGGPSSGAPPPS-NH₂ (SEQ ID NO: 57);
H-Aib-HGTFTSDYS-αK-YLEY₁KAQQEFVEWLLAGGPSSGAPPPS-NH₂ (SEQ ID NO: 58);
Y-Aib-QGTFTSDYS-αK-LLEY₁KAAQEFVEWLLAGGPSSGAPPPS-NH₂ (SEQ ID NO: 59);
H-Aib-HGTFTSDYS-αK-YLEY₁KAAQEFVEWLIEGGPSSGAPPPS-NH₂ (SEQ ID NO: 60);
H-Aib-HGTFTSDYS-αK-LLEY₁QAAQEFVEWLLAGGPSSGAPPPS-NH₂ (SEQ ID NO: 61);
H-Aib-HGTFTSDYS-αK-LLEY₁IAAQEFVEWLLAGGPSSGAPPPS-NH₂ (SEQ ID NO: 62);
H-Aib-HGTFTSDYS-αK-ILEY₁KAAQEFVEWLLAGGPSSGAPPPS-NH₂ (SEQ ID NO: 63);
H-Aib-HGTFTSDYSILLEY₁KAAQEFVEWLLAGGPSSGAPPPS-NH₂ (SEQ ID NO: 64);
H-Aib-HGTFTSDYSI-αL-LEY₁KAAQEFVEWLLAGGPSSGAPPPS-NH₂ (SEQ ID NO: 65);
H-Aib-HGTFTSDYS-αK-LLEY₁KAA-αS-EFVEWLIEGGPSSGAPPPS-NH₂ (SEQ ID NO: 66);
Y-Aib-QGTFTSDYS-αK-LLEY₁KAA-Iva-EFVEWLLAGGPSSGAPPPS-NH₂ (SEQ ID NO: 67);
H-Aib-HGTFTSDYS-αK-LLEY₁QAA-Iva-EFVEWLLAGGPSSGAPPPS-NH₂ (SEQ ID NO: 68);
Y-Aib-QGTFTSDYS-αK-ILEY₁KAA-Iva-EFVEWLLAGGPSSGAPPPS-NH₂ (SEQ ID NO: 69);
H-Aib-HGTFTSDYS-αK-ILEY₁QAA-Iva-EFVEWLLAGGPSSGAPPPS-NH₂ (SEQ ID NO: 70);
H-Aib-HGTFTSDYS-αK-LLEY₁KAA-Iva-EFVEWLIEGGPSSGAPPPS-NH₂ (SEQ ID NO: 71);
H-Aib-HGTFTSDYS-αK-ILEY₁KAA-Iva-EFVEWLIEGGPSSGAPPPS-NH₂ (SEQ ID NO: 72);
H-Aib-HGTFTSDLS-αK-LLEY₁KAA-Iva-EFVEWLIEGGPSSGAPPPS-NH₂ (SEQ ID NO: 73);
H-Aib-HGTFTSDLS-αK-ILEY₁KAA-Iva-EFVEWLIEGGPSSGAPPPS-NH₂ (SEQ ID NO: 74);
H-Aib-HGTFTSDYSILLEY₁KAA-Iva-EFVEWLLAGGPSSGAPPPS-NH₂ (SEQ ID NO: 75);
H-Aib-HGTFTSDLSILLEY₁KAA-Iva-EFVEWLLAGGPSSGAPPPS-NH₂ (SEQ ID NO: 76);
H-Aib-HGTFTSDYST-αL-LEY₁KAA-Iva-EFVEWLLAGGPSSGAPPPS-NH₂ (SEQ ID NO: 77);
H-Aib-HGTFTSDLSI-αL-LEY₁KAA-Iva-EFVEWLLAGGPSSGAPPPS-NH₂ (SEQ ID NO: 78);
H-Aib-HGTFTSDYSI-αL-LEY₁KAAQEFVEWLLAeGPSSGAPPPS-NH₂ (SEQ ID NO: 79);
H-Aib-HGTFTSDLSI-αL-LEY₁KAA-Ac4c-EFVEWLLAGGPSSGAPPPS-NH₂ (SEQ ID NO: 80);
H-Aib-HGTFTSDLSI-αL-LEY₁KAAQEFVEWLLAeGPSSGAPPPS-NH₂ (SEQ ID NO: 81);
H-Aib-HGTFTSDLSI-αL-LEY₁KAAQEFVEWLLAGGPSSGAPPPS-NH₂ (SEQ ID NO: 82);
H-Aib-HGTFTSDLSILLEY₁KAAQEFVEWLLAeGPSSGAPPPS-NH₂ (SEQ ID NO: 83);
H-Aib-HGTFTSDLSILLEY₁QAA-Ac4c-EFVEWLLAeGPSSGAPPPS-NH₂ (SEQ ID NO: 84);
H-Aib-HGTFTSDLSILLEY₁KAA-Ac3c-EFVEWLLAeGPSSGAPPPS-NH₂ (SEQ ID NO: 85);
H-Aib-HGTFTSDLSILLEY₁KAA-Ac4c-EFVEWLLAeGPSSGAPPPS-NH₂ (SEQ ID NO: 86);
H-Aib-HGTFTSDLSILLEY₁QAA-Ac3c-EFVEWLLAeGPSSGAPPPS-NH₂ (SEQ ID NO: 87);
H-Aib-HGTFTSDYSILLEY₁QAA-Ac3c-EFVEWLLAeGPSSGAPPPS-NH₂ (SEQ ID NO: 88);
H-Aib-HGTFTSDYSILLEY₁QAA-Ac4c-EFVEWLLAeGPSSGAPPPS-NH₂ (SEQ ID NO: 89);
H-Aib-HGTFTSDYSILLEY₁KAAQEFVEWLLAeGPSSGAPPPS-NH₂ (SEQ ID NO: 90);
H-Aib-HGTFTSDYSILLEY₁QAAQEFVEWLLAGGPSSGAPPPS-NH₂ (SEQ ID NO: 91);
H-Aib-HGTFTSDYSILLEY₁QAAQEFVEWLLAeGPSSGAPPPS-NH₂ (SEQ ID NO: 92);
H-Aib-QGTFTSDYSILLEY₁KAAQEFVEWLLAGGPSSGAPPPS-NH₂ (SEQ ID NO: 93);
H-Aib-QGTFTSDYSI-αL-LEY₁KAAQEFVEWLLAGGPSSGAPPPS-NH₂ (SEQ ID NO: 94);
H-Aib-QGTFTSDYSILLEY₁KAA-Aib-EFVEWLLAGGPSSGAPPPS-NH₂ (SEQ ID NO: 95); and
H-Aib-QGTFTSDYSI-αL-LEY₁KAA-Alb-EFVEWLLAGGPSSGAPPPS-NH₂ (SEQ ID NO: 96);
wherein Y₁is a Lys, Orn, Dap, Dab or Cys residue with the side chain linked to a substituent, preferably a Lys residue with the side chain linked to a substituent;
αK is alpha-methyl-Lys, αY is alpha-methyl-Tyr, αL is alpha-methyl-Leu, Iva is isovaleine, e is D-Glu, Aib is α-aminoisobutyric acid, Ac3c is 1-aminocyclopropane-1-carboxylic acid, and Ac4c is 1-aminocyclobutane-1-carboxylic acid.

11. The compound or the pharmaceutically acceptable salt thereof according to any one of claims 2-10, wherein:
Y₁ is a Lys, Orn, Dap, Dab, or Cys residue with the side chain linked to a substituent having the structure shown below:
-Z₁-Z₂,
wherein Z₁ comprises 0-10 amino acid residues independently selected from Gly, Glu, yGlu, and OEG, and Z₂ is selected from C₁₂₋₃₂ fatty acids; wherein OEG is [2-(2-amino-ethoxy)-ethoxy]-acetyl;
preferably, Z₁ comprises the structure shown below: -(OEG)ₐ-(γGlu)_{b}-; wherein a is any integer between 0 and 4, and b is any integer between 1 and 2;
Z₂ comprises the structure shown below: -C(O)-(CH₂)_{c}-COOH, wherein c is any integer between 10 and 30;
preferably, the substituent comprises the structure shown below:
{[2-(2-amino-ethoxy)-ethoxy]-acetyl}ₐ-(γGlu)_{b}-C(O)-(CH₂)_{c}-COOH;
wherein a is any integer between 0 and 4, b is any integer between 1 and 2, and c is any integer between 10 and 30;
preferably, a is 1, 2 or 3, b is 1 or 2, and c is 16-20;
more preferably, a is 2, b is 1 or 2, and c is 16, 18 or 20;
more preferably, a is 2, b is 1 or 2, and c is 16 or 18.

12. The compound or the pharmaceutically acceptable salt thereof according to claims 2-11, wherein X₁₆ selected from Y₁;
Y₁ is a Lys residue with the side chain linked to a substituent, wherein the substituent is covalently linked to the ε-amino group of the Lys residue via an amide bond; the structure of the substituent is shown below:
-Z₁-Z₂,
wherein Z₁ comprises 0-10 amino acid residues independently selected from Gly, Glu, yGlu, and OEG, and Z₂ is selected from C₁₂₋₃₂ fatty acids; wherein OEG is [2-(2-amino-ethoxy)-ethoxy]-acetyl;
preferably, Z₁ comprises the structure shown below:-(OEG)ₐ-(γGlu)_{b}; wherein a is any integer between 0 and 4, and b is any integer between 1 and 2;
Z₂ comprises the structure shown below: -CO-(CH₂)_{c}-COOH, wherein c is any integer between 10 and 30;
preferably, the substituent comprises the structure shown below:
{[2-(2-amino-ethoxy)-ethoxy]-acetyl}ₐ₋(γGlu)_{b}-CO-(CH₂)_{c}-COOH;
wherein a is any integer between 0 and 4, b is any integer between 1 and 2, and c is any integer between 10 and 30;
preferably, a is 1, 2 or 3, b is 1 or 2, and c is any integer between 16 and 20;
more preferably, a is 2, b is 1 or 2, and c is 16, 18 or 20;
more preferably, a is 2, b is 1 or 2, and c is 16 or 18.

13. The compound or the pharmaceutically acceptable salt thereof according to any one of claims 2-12, wherein:
Y₁ is K(-OEG-OEG-γGlu-C(O)-(CH₂)₁₆-COOH), K(-OEG-OEG-₇Glu-C(O)-(CH₂)₁₈-COOH), or K(-OEG-OEG-γGlu-C(O)-(CH₂)₂₀-COOH);
preferably,
Y₁ is K(-OEG-OEG-γGlu-C(O)-(CH₂)₁₆-COOH), having the structural formula: 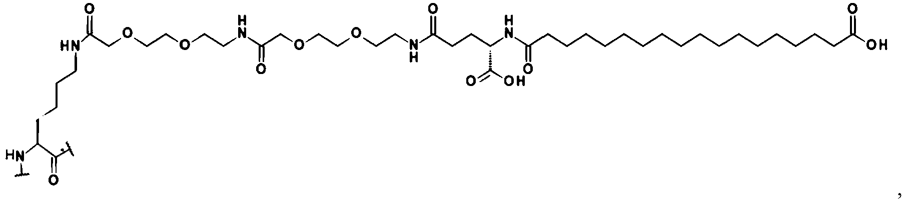 or
Y₁ is K(-OEG-OEG-γGlu-C(O)-(CH₂)₁₈-COOH), having the structural formula: 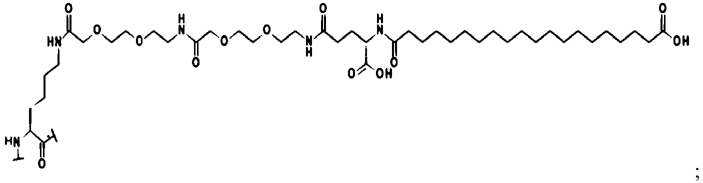
more preferably, Y₁ has the following structure:

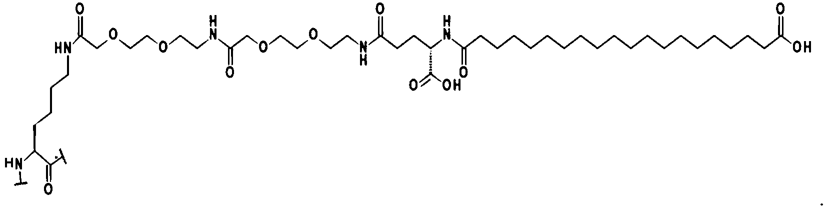

14. The compound or the pharmaceutically acceptable salt thereof according to any one of claims 1-13, selected from any of the following structures: 
H-Aib-HGTFTSDYS-αK-YLEE-K(-OEG-OEG-γGlu-C(O)-(CH2)18-COOH)-
AAQEFVEWLLAGGPSSGAPPPS-NH2 (SEQ ID NO: 97);
 
H-Aib-HGTFTSDYS-αK-YLE-K(-OEG-OEG-γGlu-C(O)-(CH2)18-COOH)-
KAAQEFVEWLLAGGPSSGAPPPS-NH2 (SEQ ID NO: 98);
 
H-Aib-HGTFTSDYSK-αY-LE-K(-OEG-OEG-γGlu-C(O)-(CH2)18-COOH)-
KAAQEFVEWLLAGGPSSGAPPPS-NH2 (SEQ ID NO: 99);
 
H-Aib-HGTFTSDYSK-Aib-LE-K(-OEG-OEG-γGlu-C(O)-(CH2)18-COOH)-
KAAQEFVEWLLAGGPSSGAPPPS-NH2 (SEQ ID NO: 100);
 
H-Aib-HGTFTSDYSKY-αL-E-K(-OEG-OEG-γGlu-C(O)-(CH2)18-COOH)-
KAAQEFVEWLLAGGPSSGAPPPS-NH2 (SEQ ID NO: 101);
 
H-Aib-HGTFTSDYSKYLE-K(-OEG-OEG-γGlu-C(O)-(CH2)18-COOH)-αK-
AAQEFVEWLLAGGPSSGAPPPS-NH2 (SEQ ID NO: 102);
 
H-Aib-HGTFTSDYSKYLE-K(-OEG-OEG-γGlu-C(O)-(CH2)18-COOH)-K-Aib-
AQEFVEWLLAGGPSSGAPPPS-NH2 (SEQ ID NO: 103);
 
H-Aib-HGTFTSDYS-αK-YLE-K(-OEG-OEG-γGlu-C(O)-(CH2)18-COOH)-
KAAQEFVEWLIAGGPSSGAPPPS-NH2 (SEQ ID NO: 104);
 
H-Aib-HGTFTSDYS-αK-LLE-K(-OEG-OEG-γGlu-C(O)-(CH2)18-COOH)-
KAAQEFVEWLLAGGPSSGAPPPS-NH2 (SEQ ID NO: 105);
 
H-Aib-HGTFTSDYS-αK-YLE-K(-OEG-OEG-γGlu-C(O)-(CH2)18-COOH)-
KAQQEFVEWLLAGGPSSGAPPPS-NH2 (SEQ ID NO: 106);
 
Y-Aib-QGTFTSDYS-αK-LLE-K(-OEG-OEG-γGlu-C(O)-(CH2)18-COOH)-
KAAQEFVEWLLAGGPSSGAPPPS-NH2 (SEQ ID NO: 107);
 
H-Aib-HGTFTSDYS-αK-YLE-K(-OEG-OEG-γGlu-C(O)-(CH2)18-COOH)-
KAAQEFVEWLIEGGPSSGAPPPS-NH2 (SEQ ID NO: 108);
 
H-Aib-HGTFTSDYS-αK-LLE-K(-OEG-OEG-γGlu-C(O)-(CH2)18-COOH)-
QAAQEFVEWLLAGGPSSGAPPPS-NH2 (SEQ ID NO: 109);
 
H-Aib-HGTFTSDYS-αK-LLE-K(-OEG-OEG-γGlu-C(O)-(CH2)18-COOH)-
IAAQEFVEWLLAGGPSSGAPPPS-NH2 (SEQ ID NO: 110);
 
H-Aib-HGTFTSDYS-αK-ILE-K(-OEG-OEG-γGlu-C(O)-(CH2)18-COOH)-
KAAQEFVEWLLAGGPSSGAPPPS-NH2 (SEQ ID NO: 111);
 
H-Aib-HGTFTSDYSILLE-K(-OEG-OEG-γGlu-C(O)-(CH2)18-COOH)-KAAQEFVEWLLAGGPSSGAPPPS-
NH2 (SEQ ID NO: 112);
 
H-Aib-HGTFTSDYSI-αL-LE-K(-OEG-OEG-γGlu-C(O)-(CH₂)₁₈-COOH)-
KAAQEFVEWLLAGGPSSGAPPPS-NH₂ (SEQ ID NO: 113);
 
H-Aib-HGTFTSDYS-αK-LLE-K(-OEG-OEG-γGlu-C(O)-(CH2)18-COOH)-KAA-αS-
EFVEWLIEGGPSSGAPPPS-NH2 (SEQ ID NO: 114);
 
Y-Aib-QGTFTSDYS-αK-LLE-K(-OEG-OEG-γGlu-C(O)-(CH2)18-COOH)-KAA-Iva-
EFVEWLLAGGPSSGAPPPS-NH2 (SEQ ID NO: 115);
 
H-Aib-HGTFTSDYS-αK-LLE-K(-OEG-OEG-γGlu-C(O)-(CH2)18-COOH)-QAA-Iva-
EFVEWLLAGGPSSGAPPPS-NH2 (SEQ ID NO: 116);
 
Y-Aib-QGTFTSDYS-αK-ILE-K(-OEG-OEG-γGlu-C(O)-(CH2)18-COOH)-KAA-Iva-
EFVEWLLAGGPSSGAPPPS-NH2 (SEQ ID NO: 117);
 
H-Aib-HGTFTSDYS-αK-ILE-K(-OEG-OEG-γGlu-C(O)-(CH2)18-COOH)-QAA-Iva-
EFVEWLLAGGPSSGAPPPS-NH2 (SEQ ID NO: 118);
 
H-Aib-HGTFTSDYS-αK-LLE-K(-OEG-OEG-γGlu-C(O)-(CH2)18-COOH)-KAA-Iva-
EFVEWLIEGGPSSGAPPPS-NH2 (SEQ ID NO: 119);
 
H-Aib-HGTFTSDYS-αK-ILE-K(-OEG-OEG-γGlu-C(O)-(CH2)18-COOH)-KAA-Iva-
EFVEWLIEGGPSSGAPPPS-NH2 (SEQ ID NO: 120);
 
H-Aib-HGTFTSDLS-αK-LLE-K(-OEG-OEG-γGlu-C(O)-(CH2)18-COOH)-KAA-Iva-
EFVEWLIEGGPSSGAPPPS-NH2 (SEQ ID NO: 121);
 
H-Aib-HGTFTSDLS-αK-ILE-K(-OEG-OEG-γGlu-C(O)-(CH2)18-COOH)-KAA-Iva-
EFVEWLIEGGPSSGAPPPS-NH2 (SEQ ID NO: 122);
 
H-Aib-HGTFTSDYSILLE-K(-OEG-OEG-γGlu-C(O)-(CH2)18-COOH)-KAA-Iva-
EFVEWLLAGGPSSGAPPPS-NH2 (SEQ ID NO: 123);
 
H-Aib-HGTFTSDLSILLE-K(-OEG-OEG-γGlu-C(O)-(CH₂)₁₈-COOH)-KAA-Iva-
EFVEWLLAGGPSSGAPPPS-NH₂ (SEQ ID NO: 124);
 
H-Aib-HGTFTSDYSI-αL-LE-K(-OEG-OEG-γGlu-C(O)-(CH2)18-COOH)-KAA-Iva-
EFVEWLLAGGPSSGAPPPS-NH2 (SEQ ID NO: 125);
 
H-Aib-HGTFTSDLSI-αL-LE-K(-OEG-OEG-γGlu-C(O)-(CH2)18-COOH)-KAA-Iva-
EFVEWLLAGGPSSGAPPPS-NH2 (SEQ ID NO: 126);
 
H-Aib-HGTFTSDYSI-αL-LE-K(-OEG-OEG-γGlu-C(O)-(CH2)18-COOH)-KAAQEFVEWLLA-e-
GPSSGAPPPS-NH2 (SEQ ID NO: 127);
 
H-Aib-HGTFTSDLSI-αL-LE-K(-OEG-OEG-γGlu-C(O)-(CH2)18-COOH)-KAA-Ac4c-
EFVEWLLAGGPSSGAPPPS-NH2 (SEQ ID NO: 128);
 
H-Aib-HGTFTSDLSI-αL-LE-K(-OEG-OEG-γGlu-C(O)-(CH2)18-COOH)-KAAQEFVEWLLA-e-
GPSSGAPPPS-NH2 (SEQ ID NO: 129);
 
H-Aib-HGTFTSDLSI-αL-LE-K(-OEG-OEG-γGlu-C(O)-(CH2)18-COOH)-
KAAQEFVEWLLAGGPSSGAPPPS-NH2 (SEQ ID NO: 130);
 
H-Aib-HGTFTSDLSILLE-K(-OEG-OEG-γGlu-C(O)-(CH2)18-COOH)-KAAQEFVEWLLA-e-GPSSGAPPPS-
NH2 (SEQ ID NO: 131);
 
H-Aib-HGTFTSDLSILLE-K(-OEG-OEG-γGlu-C(O)-(CH2)18-COOH)-QAA-Ac4c-EFVEWLLA-e-
GPSSGAPPPS-NH2 (SEQ ID NO: 132);
 
H-Aib-HGTFTSDLSILLE-K(-OEG-OEG-γGlu-C(O)-(CH2)18-COOH)-KAA-Ac3c-EFVEWLLA-e-
GPSSGAPPPS-NH2 (SEQ ID NO: 133);
 
H-Aib-HGTFTSDLSILLE-K(-OEG-OEG-γGlu-C(O)-(CH2)18-COOH)-KAA-Ac4c-EFVEWLLA-e-
GPSSGAPPPS-NH2 (SEQ ID NO: 134);
 
H-Aib-HGTFTSDLSILLE-K(-OEG-OEG-γGlu-C(O)-(CH2)18-COOH)-QAA-Ac3c-EFVEWLLA-e-
GPSSGAPPPS-NH2 (SEQ ID NO: 135);
 
H-Aib-HGTFTSDYSILLE-K(-OEG-OEG-γGlu-C(O)-(CH2)18-COOH)-QAA-Ac3c-EFVEWLLA-e-
GPSSGAPPPS-NH2 (SEQ ID NO: 136);
 
H-Aib-HGTFTSDYSILLE-K(-OEG-OEG-γGlu-C(O)-(CH2)18-COOH)-QAA-Ac4c-EFVEWLLA-e-
GPSSGAPPPS-NH2 (SEQ ID NO: 137);
 
H-Aib-HGTFTSDYSILLE-K(-OEG-OEG-γGlu-C(O)-(CH2)18-COOH)-KAAQEFVEWLLA-e-GPSSGAPPPS-
NH2 (SEQ ID NO: 138);
 
H-Aib-HGTFTSDYSILLE-K(-OEG-OEG-γGlu-C(O)-(CH2)18-COOH)-QAAQEFVEWLLAGGPSSGAPPPS-
NH2 (SEQ ID NO: 139);
 
H-Aib-HGTFTSDYSILLE-K(-OEG-OEG-γGlu-C(O)-(CH2)18-COOH)-QAAQEFVEWLLA-e-GPSSGAPPPS-
NH2 (SEQ ID NO: 140);
 
H-Aib-QGTFTSDYSILLE-K(-OEG-OEG-γGlu-C(O)-(CH2)18-COOH)-KAAQEFVEWLLAGGPSSGAPPPS-
NH2 (SEQ ID NO: 141);
 
H-Aib-QGTFTSDYSI-αL-LE-K(-OEG-OEG-γGlu-C(O)-(CH2)18-COOH)-
KAAQEFVEWLLAGGPSSGAPPPS-NH2 (SEQ ID NO: 142);
 
H-Aib-QGTFTSDYSILLE-K(-OEG-OEG-γGlu-C(O)-(CH2)18-COOH)-KAA-Aib-
EFVEWLLAGGPSSGAPPPS-NH2 (SEQ ID NO: 143); and
 
H-Aib-QGTFTSDYSI-αL-LE-K(-OEG-OEG-γGlu-C(O)-(CH2)18-COOH)-KAA-Aib-
EFVEWLLAGGPSSGAPPPS-NH2 (SEQ ID NO: 144);
 wherein αK is alpha-methyl-Lys, αY is alpha-methyl-Tyr, αL is alpha-methyl-Leu, Iva is isovaleine, e is D-Glu, Aib is α-aminoisobutyric acid, Ac3c is 1-aminocyclopropane-1-carboxylic acid, Ac4c is 1-aminocyclobutane-1-carboxylic acid, and OEG is [2-(2-amino-ethoxy)-ethoxy]-acetyl.

15. The compound or the pharmaceutically acceptable salt thereof according to any one of claims 1-14, having an agonistic activity against at least one of the following receptors: GLP-1 receptor, GIP receptor and GCG receptor; preferably, having a triple agonistic activity against GLP-1 receptor, GIP receptor and GCG receptor.

16. A GLP-1/GIP/GCG receptor triagonist, comprising the compound or the pharmaceutically acceptable salt thereof of any one of claims 1-14.

17. A pharmaceutical composition, comprising the compound of any one of claims 1-15 or a pharmaceutically acceptable salt or solvate thereof, or the GLP-1/GIP/GCG receptor triagonist of claim 16;
and a pharmaceutically acceptable excipient or pharmaceutical carrier.

18. Use of the compound or the pharmaceutically acceptable salt thereof of any one of claims 1-15, the GLP-1/GIP/GCG receptor triagonist of claim 16, or the pharmaceutical composition of claim 17 in the preparation of a medicament for preventing or treating a disease or condition related to metabolic disorders;
preferably, the disease or condition related to metabolic disorders is selected from diabetes or diabetes-related conditions, obesity or obesity-related conditions, and metabolic dysfunction-associated steatotic liver disease.

19. A method for preventing or treating a disease or condition related to metabolic disorders, comprising administering to a subject in need the compound or the pharmaceutically acceptable salt thereof of any one of claims 1 to 15, the GLP-1/GIP/GCG receptor triagonist of claim 16, or the pharmaceutical composition of claim 17;
preferably, the disease or condition related to metabolic disorders is selected from diabetes or diabetes-related conditions, obesity or obesity-related conditions, and metabolic dysfunction-associated steatotic liver disease.

20. A method for preparing the compound or the pharmaceutically acceptable salt thereof of any one of claims 1-15, comprising the step of chemically synthesizing the compound.
